# EUROPÄISCHE PATENTANMELDUNG

(11) **EP 0 600 831 A1**
(43) Veröffentlichungstag der Anmeldung: **08.06.1994**
(21) Anmeldenummer: 93810801.6
(22) Anmeldetag: 18.11.1993
(51) Int. Cl.: C07D 237/32, A61K 31/50

(54) **Phthalazinonderivate**

(30) Priorität: 27.11.1992 CH 3650/92
(71) Anmelder: CIBA-GEIGY AG, CH-4002 Basel (CH)
(72) Erfinder: Kump, Wilhelm, Dr., CH-4105 Biel-Benken (CH)

(57) **Zusammenfassung**

Erfindungsgemäss sind Verbindungen der Formel I, 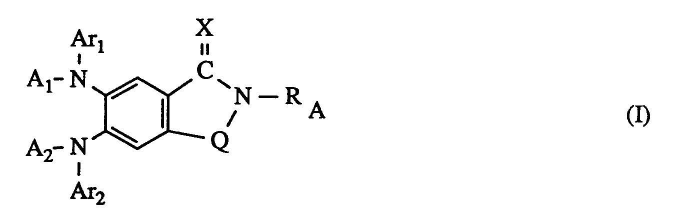
worin A₁ und A₂ Wasserstoff, gegebenenfalls substituiertes Niederalkyl, Niederalkenyl oder Niederalkinyl; Heterocyclylniederalkyl, Acyl, Niederalkylsulfonyl oder Arylsulfonyl; oder zusammen gegebenanfalls substituiertes Niederalkylen bedeuten; Ar₁ und Ar₂ Aryl, Heteroaryl oder gegebenenfalls substituiertes Cycloalkyl bedeuten; X für Sauerstoff oder Schwefel steht; und worin Q einen bivalenten Rest der Formel 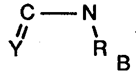
bedeutet, welcher über sein Y tragendes Kohlenstoffatom an das Q bindende Kohlenstoffatom und über sein R_{B} tragendes Stickstoffatom an das R_{A} tragende Stickstoffatom in Formel I gebunden ist, worin Y für Sauerstoff oder Schwefel steht und R_{A} und R_{B} Wasserstoff, gegebenenfalls substituiertes Niederalkyl oder Acyl bedeuten; oder worin Q einen bivalenten Rest der Formel 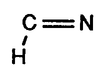
bedeutet, welcher über sein Kohlenstoffatom an das Q bindende Kohlenstoffatom und über sein Stickstoffatom an das R_{A} tragende Stickstoffatom in Formel I gebunden ist, wobei R_{A} die genannten Bedeutungen ausser Acyl hat; Salze davon und Tautomere davon. Diese hemmen Proteinkinasen, wie Protein-Tyrosinkinasen.

## Beschreibung

Die Erfindung betrifft Verbindungen der Formel I, 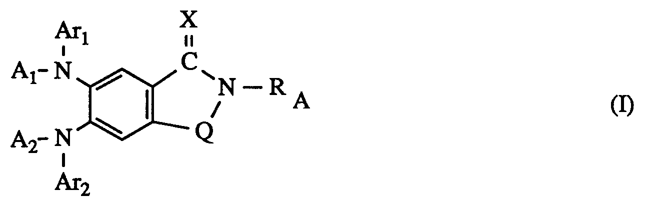
worin A₁ und A₂ unabhängig voneinander Wasserstoff, unsubstituiertes oder substituiertes Niederalkyl, unsubstituiertes oder substituiertes Niederalkenyl, unsubstituiertes oder substituiertes Niederalkinyl, Heterocyclylniederalkyl, Acyl, Niederalkylsulfonyl oder Arylsulfonyl bedeuten, oder worin A₁ und A₂ zusammen unsubstituiertes oder substituiertes Niederalkylen bedeuten; Ar₁ und Ar₂ unabhängig voneinander Aryl, Heteroaryl oder unsubstituiertes oder substituiertes Cycloalkyl bedeuten; X für Sauerstoff oder Schwefel steht; und worin Q einen bivalenten Rest der Formel 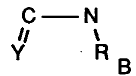
bedeutet, welcher über sein Y tragendes Kohlenstoffatom an das Q bindende Kohlenstoffatom des Benzolringes und über sein R_{B} tragendes Stickstoffatom an das R_{A} tragende Stickstoffatom in Formel I gebunden ist, worin Y unabhängig von X für Sauerstoff oder Schwefel steht und R_{A} und R_{B} unabhängig voneinander Wasserstoff, unsubstituiertes oder substituiertes Niederalkyl oder Acyl bedeuten; oder worin Q einen bivalenten Rest der Formel 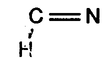
bedeutet, welcher über sein Kohlenstoffatom an das Q bindende Kohlenstoffatom des Benzolringes und über sein Stickstoffatom an das R_{A} tragende Stickstoffatom in Formel I gebunden ist, wobei R_{A} eine der genannten Bedeutungen ausser Acyl hat; Salze davon, sofern salzbildende Gruppen vorliegen, und Tautomere davon, sofern tautomerisierbare Gruppen vorliegen; Verfahren zur Herstellung dieser Verbindungen, pharmazeutische Präparate, die diese Verbindungen enthalten, und die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung vorzugsweise die folgenden Bedeutungen:

Das Präfix "Nieder" bezeichnet einen Rest bis und mit 7, insbesondere bis und mit 4, und vor allen Dingen mit 1 bis 3 Kohlenstoffatomen, sofern nichts anderes angegeben ist.

Niederalkyl ist vorzugsweise n-Propyl, Isopropyl, n-Butyl, Isobutyl, sek.-Butyl, tert.-Butyl, n-Pentyl, Neopentyl, n-Hexyl oder n-Heptyl, vorzugsweise Methyl, Ethyl oder n-Propyl.

Niederalkenyl hat 2 bis 7, vorzugsweise 3 bis 7, insbesondere 3 oder 4 Kohlenstoffatome, und ist z. B. Allyl oder Crotyl.

Niederalkinyl hat 2 bis 7, vorzugsweise 3 bis 7, insbesondere 3 oder 4 Kohlenstoffatome, und ist z. B. Propin-1- oder Propin-2-yl oder 2-Butin-1-yl.

Substituiertes Niederalkyl ist vorzugsweise wie oben definiertes Niederalkyl, welches durch bis zu 4, vorzugsweise bis zu 2 Reste ausgewählt aus Amino, wie in Aminomethyl, -ethyl, -propyl oder -butyl, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, wie Aminomethyl, Aminoethyl, Aminopropyl oder Aminobutyl, Cycloalkylamino, Phenylniederalkylamino oder Phenylamino, Acylamino, z.B. Niederalkanoylamino, Phenylniederalkanoylamino oder Phenylcarbonylamino (≙ Benzoylamino), Hydroxy, wie in Hydroxymethyl, 2-Hydroxyethyl oder 3-Hydroxypropyl, Niederalkoxy, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkoxy, Acyloxy, insbesondere Niederalkanoyloxy, Mercapto, Niederalkylthio, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Phenylniederalkylthio, Acylthio, insbesondere Niederalkanoylthio, Carboxy, wie in Carboxymethyl, -ethyl oder -propyl, verestertes Carboxy, z.B. Niederalkoxycarbonyl, wie Methoxycarbonyl, Ethoxycarbonyl oder tert-Butoxycarbonyl, vor allem in Methoxy- oder Ethoxycarbonyl-methyl, -ethyl oder -proypl, oder Phenylniederalkoxycarbonyl, wie Benzyloxycarbonyl, Cyano, Carbamoyl, wie in Carbamoylmethyl, Carbamoylethyl oder Carbamoylpropyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl, Aryl oder Arylniederalkyl substituiertes Ureido, insbesondere 1- oder 3-Mono-, 1,3- oder 3,3-Di- oder 1,3,3-Triniederalkylureido, 1- oder 3-Phenylureido, 1- oder 3-Mono-, 1,3- oder 3,3-Di- oder 1,3,3-Triphenylniederalkylureido, z. B. 3-Niederalkylureido, wie 3-Methyl- oder 3-Ethylureido, vor allem in 3-Methyl oder 3-Ethyl-ureido-methyl, -ethyl oder -propyl, Thioureido, an einem oder beiden Stickstoffatomen durch Niederalkyl, Aryl oder Arylniederalkyl substituiertes Thioureido, insbesondere 1- oder 3-Mono-, 1,3- oder 3,3-Di- oder 1,3,3-Triniederalkylthioureido, 1- oder 3-Phenylthioureido, 1- oder 3-Mono-, 1,3- oder 3,3-Di- oder 1,3,3-Triphenylniederalkylthioureido, z.B. 3-Niederalkylthioureido, wie 3-Methyl- oder 3-Ethylthioureido, vor allem in 3-Methyl- oder 3-Ethyl-thioureido-methyl, -ethyl oder -propyl, Hydrazino, an einem oder beiden Stickstoffatomen durch Niederalkyl, Aryl oder Arylniederalkyl substituiertes Hydrazino, insbesondere 1- oder 2-Mono-, 1,2- oder 2,2-Di- oder 1,2,2-Triniederalkylhydrazino, 1- oder 2-Phenylhydrazino, 1- oder 2-Mono-, 1,2- oder 2,2-Di- oder 1,2,2-Triphenylniederalkylhydrazino, z. B. 2,2-Diniederalkylhydrazino, wie 2,2-Dimethyl- oder 2,2-Diethylhydrazino, Amidino, wie in Amidinomethyl, -ethyl oder -propyl, an einem oder beiden Stickstoffatomen durch Niederalkyl, Aryl oder Arylniederalkyl substituiertes Amidino, insbesondere N¹- oder N²-Mono-, N¹,N²- oder N¹,N¹-Di- oder N¹,N¹,N²-Triniederalkylamidino, N¹- oder N²-Phenylamidino, N¹- oder N²-Mono-, N¹,N²-, N¹,N¹-Di- oder N¹,N¹,N²-Triphenylniederalkylamidino, z. B. N¹,N¹-Diniederalkylamidino, wie N¹,N¹-Dimethyl- oder N¹,N¹-Diethylamidino, Guanidino, wie in Guanidinomethyl, -ethyl oder -propyl, an einem, zwei oder allen drei Stickstoffatomen durch Niederalkyl, Aryl oder Arylniederalkyl substituiertes Guanidino, insbesondere 1-, 2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3,3-Tetra- oder 1,1,2,3,3,-Pentaniederalkyl- oder -Phenylniederalkylguanidino, insbesondere 3,3-Diniederalkylguanidino, wie 3,3-Dimethylguanidino oder 3,3-Diethylguanidino, Oxo, welches nicht am Kohlenstoff gebunden ist, welcher an den A₁ oder A₂ tragenden Stickstoff gebunden ist, wie in 2-Oxopropyl oder 3-Oxo-n-butyl, Thioxo, Imino, Niederalkylimino, Acylimino, insbesondere Niederalkanoylimino, wie Acetylimino, Hydroxyimino (HO-N=), wie in Hydroxyiminomethyl (HO-N=CH-), Hydroxyimino-ethyl oder -propyl, Niederalkoxyimino, wie Methoxyimino, Hydrazono, wie in Hydrazonomethyl, -ethyl oder -propyl, N-Mono- oder N,N-Diniederalkylhydrazono, N-Acylhydrazono, insbesondere N-Niederalkanoylhydrazono, wie Acetylhydrazono oder Niederalkoxycarbonylhydrazono, wie tert-Butoxycarbonylhydrazono, Niederalkylthioimino, wie Methyl- oder Ethylthioimino, vor allem in Methylthioimino- oder Ethylthioimino-methyl, -ethyl oder -propyl, und Aryl, vor allem unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl, oder Pentafluorphenyl, z.B. in Benzyl. Vorzugsweise ist Niederalkyl linear und endständig substituiert mit einem der genannten Substituenten.

Substituiertes Niederalkenyl ist vorzugsweise wie oben definiertes Niederalkenyl, insbesondere mit 3 bis 7, vor allem 3 oder 4 Kohlenstoffatomen, welches durch bis zu 4 Reste, vorzugsweise einen Rest ausgewählt aus den bei der Definition von substituiertem Niederalkyl genannten Substituenten substituiert ist. Bei bestimmten Substituenten sind durch Wechselwirkung mit der Doppelbindung Tautomere möglich. So können Hydroxy, Mercapto oder N-gebundene Substituenten, die noch einen freien Wasserstoff am bindenden Stickstoff haben, bei Bindung an ein Kohlenstoffatom einer Dreifachbindung tautomerisieren zu Oxo- bzw. Thioxo- bzw. Iminoverbindungen; mit einer Doppelbindung über Stickstoff gebundene Substituenten wie Hydroxyimino oder Hydrazono können bei Vorliegen in Konjugation zu einer Doppelbindung im Niederalkenylrest ebenfalls tautomerisieren; derartige Verbindungen können auch in Tautomeriegleichgewichten vorliegen. Bevorzugt als substituiertes Niederalkenyl sind solche Reste, bei denen keine Tautomerie auftritt, d. h. wo z.B. Hydroxy, Mercapto oder N-gebundene Substituenten, die noch einen freien Wasserstoff am bindenden Stickstoff haben, nicht an ein Kohlenstoffatom im Niederalkenylrest gebunden sind, von dem eine Doppelbindung ausgeht, und/oder wo mit einer Doppelbindung über Stickstoff gebundene Substituenten wie Hydroxyimino oder Hydrazono nicht in Konjugation mit einer Doppelbindung im Niederalkenylrest stehen. Bevorzugt gegenüber substituiertem Niederalkenyl ist jedoch unsubstituiertes Niederalkenyl A₁ und/oder A₂, insbesondere gebunden über ein gesättigtes Kohlenstoffatom.

Substituiertes Niederalkinyl ist vorzugsweise wie oben definiertes Niederalkinyl, insbesondere mit 3 bis 7, vor allem 3 oder 4 Kohlenstoffatomen, welches durch bis zu 4 Reste, vorzugsweise einen Rest ausgewählt aus den bei der Definition von substituiertem Niederalkyl genannten Substituenten substituiert ist. Analog der Definition von Tautomeren und Tautomeriegleichgewichten für substituiertes Niederalkenyl können auch für substituiertes Niederalkinyl entsprechenden Tautomere und Tautomeriegleichgewichte vorliegen. Bevorzugt gegenüber substituiertem Niederalkinyl ist unsubstituiertes Niederalkinyl A₁ und/oder A₂, insbesondere gebunden über ein gesättigtes Kohlenstoffatom.

Heterocyclylniederalkyl ist einer der oben genannten Niederalkylreste, vorzugsweise Methyl, Ethyl oder n-Propyl, welcher, vorzugsweise am endständigen Kohlenstoffatom, substituiert ist durch Heterocyclyl, welches in erster Linie einen gesättigten, teilweise gesättigten oder ungesättigten einfachen Ring bedeutet, der über ein Ringstickstoffatom gebunden ist und 3 bis 7, in erster Linie 5 - 7 Ringatome enthält, wobei ausser dem bindenden Stickstoffatom noch bis zu zwei weitere Heteroatome ausgewählt aus Stickstoff, Schwefel und/oder Sauerstoff vorliegen können; als einfacher Ring vorliegt oder bis zu zweifach, vorzugsweise einfach benzanneliert, cyclopenta-, cyclohexa- oder cycloheptaanneliert sein kann; und unsubstituiert oder insbesondere durch Niederalkyl, Niederalkanoyl, Hydroxy, Niederalkoxy, Halogen, Cyano und/oder Trifluormethyl substituiert sein kann, z. B. Pyrrolyl, 2,5-Dihydropyrrolyl, Pyrrolinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3,4-Tetrahydrochinolyl, oder 1,2-Dihydro- oder 1,2,3,4-Tetrahydroisochinolyl, wobei die genannten Reste unsubstituiert oder wie oben substituiert sind, insbesondere durch Niederalkyl, z. B. in 4-Niederalkyl-piperazin-1-yl, wie 4-Methyl- oder 4-Ethyl-piperazin-1-yl, oder durch Niederalkanoyl, z. B. in 4-Niederalkanoyl-piperazin-1-yl, wie 4-Acetyl-piperazin-1-yl.

Acyl bedeutet ein über eine Carbonyl- oder Thiocarbonylgruppe gebundenes Radikal einer unsubstituierten oder substituierten Niederalkancarbonsäure (Niederalkyl-(C=O)-) oder Niederalkanthiocarbonsäure (Niederalkyl-(C=S)-, worin die Substituenten vorzugsweise unabhängig voneinander aus einem oder mehreren Resten, insbesondere bis zu drei Resten, aus der Gruppe bestehend aus Hydroxy, Niederalkoxy, Phenylniederalkoxy, Niederalkanoyloxy, Halogen, wie Fluor, Chlor oder Brom, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl, Phenyl, Naphthyl, wie 1- oder 2-Naphthyl, Fluorenyl, wie 9-Fluorenyl, durch Halogen, wie Fluor oder Chlor, Trifluormethyl, Hydroxy, Niederalkoxy und/oder Cyano substituiertem Phenyl oder Naphthyl und Cyano ausgewählt sind; ein über eine Carbonyl- oder Thiocarbonylgruppe gebundenes Radikal einer unsubstituierten oder substituierten Arylcarbonsäure, vorzugsweise ausgewählt aus unsubstituiertem oder durch durch Halogen, wie Fluor oder Chlor, Trifluormethyl, Hydroxy, Niederalkoxy und/oder Cyano substituiertem Benzoyl oder oder Naphthylcarbonyl; Carbamoyl oder Thiocarbamoyl; der über seine Aminocarbonylgruppe oder Aminothiocarbonylgruppe gebundene Rest einer N-substituierten Carbaminsäure oder Thiocarbaminsäure, worin das N-Atom vorzugsweise durch einen oder zwei aus Niederalkyl und substituiertem Niederalkyl ausgewählte Reste substituiert ist, wobei substituiertes Niederalkyl in erster Linie durch die oben für substituierte Niederalkancarbonsäuren genannten Substituenten substituiert ist, vorzugsweise durch bis zu drei, in erster Linie durch einen dieser Substituenten; oder der über seine Carbonylgruppe gebundene Rest eines Halbesters der Kohlensäure, welcher vorzugsweise aus Niederalkoxycarbonyl und substituiertem Niederalkoxycarbonyl ausgewählt ist, worin vorzugsweise bis zu drei, in erster Linie einer der Substituenten vorliegt, wie sie oben für substituierte Niederalkancarbonsäuren definiert sind, und ist insbesondere Niederalkanoyl, Halogenniederalkanoyl, wie Trifluor- oder Trichloracetyl, Phenylniederalkanoyl, wie Phenylacetyl, Benzoyl, Carbamoyl, N-Mono- oder N,N-Diniederalkylcarbamoyl, N-Mono- oder N,N-Bis(hydroxyniederalkyl)carbamoyl, Thiocarbamoyl, N-Mono- oder N,N-Diniederalkylthiocarbamoyl, N-Mono- oder N,N-Bis(hydroxyniederalkyl)thiocarbamoyl, Niederalkoxycarbonyl, wie tert-Butoxycarbonyl, oder Phenylniederalkoxycarbonyl, wie Benzyloxycarbonyl. Acyl ist in erster Linie Niederalkanoyl, Aminoniederalkanoyl, wie Aminoacetyl, Carbamoyl oder Thiocarbamoyl. In bevorzugten Verbindungen der Formel I kann bei der Definition von R_{A} und R_{B} zugunsten der anderen Definitionen Acyl wegfallen.

Niederalkanoyl ist vorzugsweise Formyl, Acetyl, Propionyl, n-Butyryl, Pivaloyl oder Valeroyl, insbesondere Formyl, Acetyl oder Propionyl.

Halogen steht insbesondere für Fluor, Chlor, Brom oder Iod, vor allem Fluor oder Iod.

Niederalkylsulfonyl (= Niederalkyl-SO₂-) ist vorzugsweise Methan- oder Ethansulfonyl.

Arylsulfonyl (= Aryl-SO₂-) enthält vorzugsweise einen wie unten definierten Arylrest und ist insbesondere Benzolsulfonyl oder im Benzolrest durch Niederalkyl, Niederalkoxy, Hydroxy, Halogen und/oder Trifluormethyl, vorzugsweise einen der genannten Reste, substituiertes Benzolsulfonyl, z.B. Toluolsulfonyl, wie 4-Toluolsulfonyl.

Niederalkylen, welches aus A₁ und A₂ zusammen gebildet wird, ist unverzweigt, hat insbesondere 1 bis 4, bevorzugt 2 bis 3, Kohlenstoffatome und ist unsubstituiert oder substituiert durch einen oder mehrere, vorzugsweise bis zu 3, insbesondere einen Substituenten ausgewählt aus Niederalkyl, wie Methyl oder Ethyl, Amino, Aminoniederalkyl, wie Aminomethyl, -ethyl oder -propyl, Mono- oder Diniederalkylamino, Mono- oder Diniederalkylaminoniederalkyl, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, Cycloalkylamino, Cycloalkylaminoniederalkyl, Phenylniederalkylamino, Phenylniederalkylaminoniederalkyl, Phenylamino, Phenylaminoniederalkyl, Acylamino, z.B. Niederalkanoylamino, Phenylniederalkanoylamino oder Phenylcarbonylamino (≙ Benzoylamino), Acylaminoniederalkyl, z.B. Niederalkanoylaminoniederalkyl, Phenylniederalkanoylaminoniederalkyl oder Phenylcarbonylaminoniederalkyl (≙ Benzoylaminoniederalkyl), Hydroxy, Hydroxyniederalkyl, z. B. Hydroxymethyl, Hydroxyethyl oder Hydroxypropyl, Niederalkoxy oder Niederalkoxyniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, z. B. 2-Methoxy- oder 2-Ethoxy-ethoxyniederalkyl, Phenylniederalkoxy, wie Benzyloxy, Phenylniederalkoxyniederalkyl, wie 2-Benzyloxyethyl, Acyloxy, insbesondere Niederalkanoyloxy, Acyloxyniederalkyl, insbesondere Niederalkanoyloxyniederalkyl, wie 2-Acetoxy-ethyl, Mercapto, Mercaptoniederalkyl, z. B. Mercaptomethyl oder Mercaptoethyl, Niederalkylthio oder Niederalkylthioniederalkyl, worin der endständige Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, z.B. 2-Methylthio- oder 2-Ethylthio-ethylthioniederalkyl, Phenylniederalkylthio, wie Benzylthio, Phenylniederalkylthioniederalkyl, wie 2-Benzylthio-ethyl, Acylthio, insbesondere Niederalkanoylthio, Acylthioniederalkyl, insbesondere Niederalkanoylthioniederalkyl, wie 2-Acetylthio-ethyl, Carboxy, Carboxyniederalkyl, wie Carboxymethyl, verestertes Carboxy, z.B. Niederalkoxycarbonyl, wie Methoxycarbonyl, Ethoxycarbonyl oder tert-Butoxycarbonyl, verestertes Carboxyniederalkyl, z.B. Niederalkoxycarbonylniederalkyl, wie Methoxycarbonylmethyl, Ethoxycarbonylmethyl oder tert-Butoxycarbonylmethyl, oder Phenylniederalkoxycarbonylniederalkyl, wie Benzyloxycarbonylmethyl, Cyano, Cyanoniederalkyl, Carbamoyl, Carbamoylniederalkyl, wie Carbamoylmethyl, -ethyl oder -propyl, N-Niederalkylcarbamoyl, wie N-Methyl- oder N-Ethyl-carbamoyl-methyl, -ethyl oder -propyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylniederalkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl, Aryl oder Arylniederalkyl substituiertes Ureido oder Ureidoniederalkyl, insbesondere 1- oder 3-Mono-, 1,3- oder 3,3-Di- oder 1,3,3-Triniederalkylureido, 1- oder 3-Phenylureido, 1- oder 3-Mono-, 1,3- oder 3,3-Di- oder 1,3,3-Triphenylniederalkylureido, z. B. 3-Niederalkylureido, wie 3-Methyl- oder 3-Ethylureido, 1- oder 3-Mono-, 1,3-Di- oder 3,3-Di- oder 1,3,3-Triniederalkylureidoniederalkyl, 1- oder 3-Phenylureidoniederalkyl, 1- oder 3-Mono-, 1,3- oder 3,3-Di- oder 1,3,3-Triphenylniederalkylureidoniederalkyl, z. B. 3-Niederalkylureidoniederalkyl, wie 3-Methyl- oder 3-Ethylureidoniederalkyl, vor allem 3-Methyl- oder 3-Ethylureidomethyl, -ethyl oder -propyl, Thioureido, Thioureidoniederalkyl, an einem oder beiden Stickstoffatomen durch Niederalkyl, Aryl oder Arylniederalkyl substituiertes Thioureido oder Thioureidoniederalkyl, insbesondere 1- oder 3-Mono-, 1,3- oder 3,3-Di- oder 1,3,3-Triniederalkylthioureido, 1- oder 3-Phenylthioureido, 1- oder 3-Mono-, 1,3- oder 3,3-Di- oder 1,3,3-Triphenylniederalkylthioureido, z. B. 3-Niederalkylthioureido, wie 3-Methyl- oder 3-Ethylthioureido, 1- oder 3-Mono-, 1,3-Di- oder 3,3-Di- oder 1,3,3-Triniederalkylthioureidoniederalkyl, 1- oder 3-Phenylthioureidoniederalkyl, 1- oder 3-Mono-, 1,3- oder 3,3-Di- oder 1,3,3-Triphenylniederalkylthioureidoniederalkyl, z. B. 3-Niederalkylthioureidoniederalkyl, wie 3-Methyl- oder 3-Ethylthioureidoniederalkyl, vor allem 3-Methyl- oder 3-Ethylthioureidomethyl, -ethyl oder -propyl, Hydrazino, Hydrazinoniederalkyl, wie Hydrazinomethyl, -ethyl oder -propyl, an einem oder beiden Stickstoffatomen durch Niederalkyl, Aryl oder Arylniederalkyl substituiertes Hydrazino oder Hydrazinoniederalkyl, insbesondere 1- oder 2-Mono-, 1,2- oder 2,2-Di- oder 1,2,2-Triniederalkylhydrazino, 1- oder 2-Phenylhydrazino, 1- oder 2-Mono-, 1,2- oder 2,2-Di- oder 1,2,2-Triphenylniederalkylhydrazino, z. B. 2,2-Diniederalkylhydrazino, wie 2,2-Dimethyl- oder 2,2-Diethylhydrazino, 1- oder 2-Mono-, 1,2- oder 2,2-Di- oder 1,2,2-Triniederalkylhydrazinoniederalkyl, 1- oder 2-Phenylhydrazinoniederalkyl, 1- oder 2-Mono-, 1,2- oder 2,2-Di- oder 1,2,2-Triphenylniederalkylhydrazinoniederalkyl, z. B. 2,2-Diniederalkylhydrazinoniederalkyl, wie 2,2-Dimethyl- oder 2,2-Diethylhydrazinoniederalkyl, Amidino, Amidinoniederalkyl, wie Amidinomethyl, -ethyl oder -propyl, an einem oder beiden Stickstoffatomen durch Niederalkyl, Aryl oder Arylniederalkyl substituiertes Amidino oder Amidinoniederalkyl, insbesondere N¹- oder N²-Mono-, N¹,N²- oder N¹,N¹-Di- oder N¹,N¹,N²-Triniederalkylamidino, N¹- oder N²-Phenylamidino, N¹- oder N²-Mono-, N¹,N²-, N¹,N¹-Di- oder N¹,N¹,N²-Triphenylniederalkylamidino, z. B. N¹,N¹-Diniederalkylamidino, wie N¹,N¹-Dimethyl- oder N¹,N¹-Diethylamidino, N¹- oder N²-Mono-, N¹,N²- oder N¹,N¹-Di- oder N¹,N¹,N²-Triniederalkylamidinoniederalkyl, N¹- oder N²-Phenylamidinoniederalkyl, N¹- oder N²-Mono-, N¹,N²-, N¹,N¹-Di- oder N¹,N¹,N²-Triphenylniederalkylamidinoniederalkyl, z. B. N¹,N¹-Diniederalkylamidinoniederalkyl, wie N¹,N¹-Dimethyl- oder N¹,N¹-Diethylamidinoniederalkyl, Guanidino, Guanidinoniederalkyl, wie Guanidinomethyl, -ethyl oder -propyl, an einem, zwei oder allen drei Stickstoffatomen durch Niederalkyl, Aryl oder Arylniederalkyl substituiertes Guanidino oder Guanidinoniederalkyl, insbesondere 1-, 2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3,3-Tetra- oder 1,1,2,3,3 -Pentaniederalkyl- oder -phenylniederalkylguanidino, insbesondere 3,3-Diniederalkylguanidino, wie 3,3-Dimethylguanidino oder 3,3-Diethylguanidino, 1-,2- oder 3-Mono-, 1,1-, 3,3-, 1,2-, 1,3- oder 2,3-Di-, 1,1,2-, 1,1,3-, 1,2,3-, 1,3,3- oder 2,3,3-Tri-, 1,1,2,3-, 1,2,3,3- oder 1,1,3,3-Tetra- oder 1,1,2,3,3,-Pentaniederalkyl- oder -Phenylniederalkylguanidinoniederalkyl, insbesondere 3,3-Diniederalkylguanidinoniederalkyl, wie 3,3-Dimethylguanidinoniederalkyl oder 3,3-Diethylguanidinoniederalkyl, Oxo, Oxoniederalkyl, insbesondere Niederalkanoyl, wie Formyl, Acetyl oder Propionyl, Thioxo, Thioxoniederalkyl, Imino, Iminoniederalkyl, Niederalkylimino, Niederalkyliminoniederalkyl, Acylimino, insbesondere Niederalkanoylimino, wie Acetylimino, Acyliminoniederalkyl, insbesondere Niederalkanoyliminoniederalkyl, wie Acetyliminoniederalkyl, Hydroxyimino, Hydroxyiminoniederalkyl, wie Hydroxyiminomethyl, Hydroxyiminoethyl oder Hydroxyiminopropyl, Niederalkoxyimino oder Niederalkoxyiminoniederalkyl, wie Methoxyimino oder Methoxyiminoniederalkyl, Hydrazono, Hydrazononiederalkyl, wie Hydrazonomethyl, Hydrazonoethyl oder Hydrazonopropyl, N-Mono- oder N,N-Diniederalkylhydrazono, N-Mono- oder N,N-Diniederalkylhydrazononiederalkyl, N-Acylhydrazono, insbesondere N-Niederalkanoylhydrazono, wie Acetylhydrazono, oder Niederalkoxycarbonylhydrazono, wie tert-Butoxycarbonylhydrazono, N-Acylhydrazononiederalkyl, insbesondere N-Niederalkanoylhydrazononiederalkyl, wie Acetylhydrazononiederalkyl, oder Niederalkoxycarbonylhydrazononiederalkyl, wie tert-Butoxycarbonylhydrazononiederalkyl, Niederalkylthioimino, wie Methyl- oder Ethylthioimino, und Niederalkylthioiminoniederalkyl, wie Methyl- oder Ethylthioiminoniederalkyl, vor allem Methyl- oder Ethylthioiminomethyl, -ethyl oder -propyl.

Aryl ist vorzugsweise Phenyl oder Naphthyl, wie 1- oder 2-Naphthyl. Die Phenyl- und Naphthylreste können unsubstituiert oder substituiert sein, insbesondere wie nachfolgend für Phenyl angegeben. Aryl ist bevorzugt Phenyl, das unsubstituiert oder durch einen oder mehrere, vorzugsweise bis zu 5, insbesondere einen oder zwei, vor allem einen insbesondere in p-Stellung vorliegenden oder im Falle von Halogen, vor allem Fluor, bis zu 5, Substituenten aus der Gruppe bestehend aus Hydrocarbyl, z.B. Niederalkyl, Niederalkenyl, Niederalkinyl, Niederalkylen (angeknüpft an zwei benachbarten C-Atomen), Cycloalkyl, Phenylniederalkyl oder Phenyl; substituiertes Hydrocarbyl, z.B. Niederalkyl, das z.B. durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert ist; Hydroxy; verethertes Hydroxy, z.B. Niederalkoxy, Halogenniederalkoxy, Phenylniederalkoxy, Phenyloxy, Niederalkenyloxy, Halogenniederalkenyloxy oder Niederalkinyloxy; Niederalkylendioxy (angeknüpft an zwei benachbarten C-Atomen); verestertes Hydroxy, z.B. Niederalkanoyloxy, Phenylniederalkanoyloxy oder Phenylcarbonyloxy (≙ Benzoyloxy); Mercapto; verethertes Mercapto, das gegebenenfalls oxidiert ist, z.B. Niederalkylthio, Phenylniederalkylthio, Phenylthio, Niederalkylsulfinyl [-S(=O)-Niederalkyl], Phenylniederalkylsulfinyl, Phenylsulfinyl, Niederalkylsulfonyl [-S(O₂)-Niederalkyl], Phenylniederalkylsulfonyl oder Phenylsulfonyl; Halogen, Nitro, Amino; Monohydrocarbylamino, z.B. Niederalkylamino, Cycloalkylamino, Phenylniederalkylamino oder Phenylamino; Dihydrocarbylamino, z.B. Diniederalkylamino, N-Niederalkyl-N-phenylamino, N-Niederalkyl-N-phenylniederalkylamino, Niederalkylenamino oder durch -O-, -S- oder -NR'' unterbrochenes Niederalkylenamino (worin R" für Wasserstoff, Niederalkyl oder Acyl, z.B. Niederalkanoyl, steht); Acylamino, z.B. Niederalkanoylamino, Phenylniederalkanoylamino oder Phenylcarbonylamino (≙ Benzoylamino); Acyl, z.B. Niederalkanoyl, Phenylniederalkanoyl oder Phenylcarbonyl (= Benzoyl); Carboxy; verestertes Carboxy, z.B. Niederalkoxycarbonyl; amidiertes Carboxy, z.B. Carbamoyl, N-Niederalkylcarbamoyl, N,N-Diniederalkylcarbamoyl, N-Hydroxycarbamoyl oder N-Phenylcarbamoyl; Cyano, durch zwei unabhängig voneinander aus Hydroxy, Niederalkoxy oder Phenylniederalkoxy, wie Benzyloxy, ausgewählte Reste am Phosphor substituiertes Phosphoryloxy oder durch Phenylen-1,2-dioxy am Phosphor substituiertes Phosphoryloxy (d. h. einen Rest der Formel 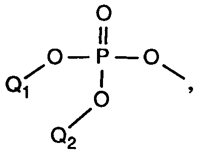
worin Q₁ und Q₂ unabhängig voneinander Wasserstoff, Niederalkyl oder Phenylniederalkyl bedeuten, oder worin Q₁ und Q₂ zusammen ortho-Phenylen bedeuten), Sulfo (SO₃H); verestertes Sulfo, z.B. Niederalkoxysulfonyl; und amidiertes Sulfo, z.B. Sulfamoyl (-SO₂NH₂), N-Niederalkylsulfamoyl, N,N-Diniederalkylsulfamoyl oder N-Phenylsulfamoyl, substituiert ist; wobei in den Substituenten vorkommende Phenylgruppen jeweils unsubstituiert oder durch Niederalkyl, Niederalkoxy, Hydroxy, Halogen und/oder Trifluormethyl substituiert sind; in erster Linie ist Aryl unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxy- oder Ethoxycarbonyl, und Cyano o-, m- oder p-substituiertes Phenyl, oder Pentafluorphenyl.

Niederalkylen, angeknüpft an zwei benachbarten C-Atomen eines Benzolrings, ist vorzugsweise C₃-C₄-Alkylen, z.B. 1,3-Propylen oder 1,4-Butylen.

Niederalkylendioxy, angeknüpft an zwei benachbarten C-Atomen, bedeutet vorzugsweise C₁-C₂-Alkylendioxy, z.B. Methylen- oder 1,2-Ethylendioxy.

Niederalkylenamino ist vorzugsweise C₄-C₇- und insbesondere C₄-C₅-Alkylenamino, z.B. Piperidino. Durch -O-, -S- oder -NR'- unterbrochenes Niederalkylenamino steht vorzugsweise für solches C₄-C₇- und insbesondere C₄-C₅-Alkylenamino, bei dem ein Ringkohlenstoffatom durch die entsprechende Heterogruppe ersetzt ist, und ist insbesondere Morpholino, Thiomorpholino, Piperazino oder 4-Niederalkyl- oder 4-Niederalkanoylpiperazino.

Heteroaryl steht für einen ungesättigten heterocyclischen Rest und ist vorzugsweise über ein Ringkohlenstoffatom verknüpft. Es handelt sich insbesondere um einen 5- oder 6-gliedrigen Ring mit bis zu drei Heteroatomen ausgewählt aus N, O und S, vor allem N, z.B. Imidazolyl, Triazolyl, Pyridyl, Pyrimidinyl oder Triazinyl, und vor allen Dingen um Pyridyl. Diese Reste können unsubstituiert oder z.B. durch Niederalkyl, Hydroxy, Niederalkoxy, Halogen, Cyano und/oder Trifluormethyl substituiert sein.

Pyridyl ist z.B. 2-, 3- oder 4-Pyridyl.

Imidazolyl ist z.B. 2- oder 4(5)-Imidazolyl.

Triazolyl ist z.B. 1,2,4-Triazol-3- oder -4-yl oder 1,2,3-Triazol-4-yl.

Pyrimidinyl ist z.B. 2-, 4- oder 5-Pyrimidinyl.

Triazinyl ist z.B. 1,3,5-Triazin-2-yl.

Heteroaryl ist insbesondere 2-, 3- oder 4-Pyridyl, 2-, 4- oder 5-Pyrimidinyl oder 1,3,5-Triazin-2-yl.

Cycloalkyl ist vorzugsweise C₃-C₈- und insbesondere C₅-C₇-Cycloalkyl, was bedeutet, dass es 3 bis 8 bzw. 5 bis 7 Ringkohlenstoffe enthält, und ist z. B. Cyclopentyl, Cyclohexyl oder Cycloheptyl. Die genannten Cycloalkylreste können auch substituiert sein, z.B. durch Niederalkyl oder Hydroxy oder auch durch Halogen, wie Fluor oder Chlor.

Das bivalente Radikal Q ist entweder ein bivalenter Rest der Formel 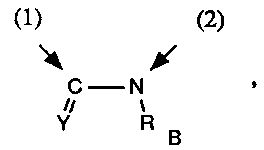
welcher über sein Y tragendes Kohlenstoffatom [Pfeil (1)] an das Q bindende Kohlenstoffatom des Benzolringes und über sein R_{B} tragendes Stickstoffatom [Pfeil (2)] an das R_{A} tragende Stickstoffatom in Formel I gebunden ist, worin Y unabhängig von X für Sauerstoff oder Schwefel steht, so dass entsprechende Verbindungen der Formel I die Formel IA, 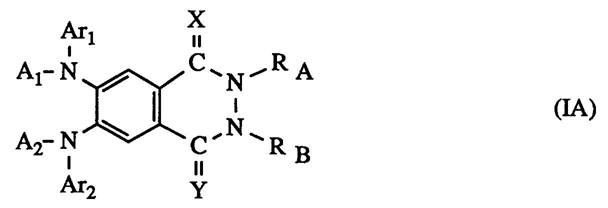
haben, worin die Reste die für Verbindungen der Formel I genannten Bedeutungen haben, oder als Tautomere davon vorliegen, wie unten beschrieben; oder ein bivalenter Rest der Formel 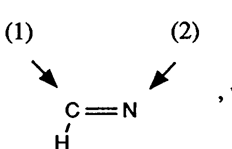
welcher über sein Kohlenstoffatom [Pfeil (1)] an das Q bindende Kohlenstoffatom des Benzolringes und über sein Stickstoffatom [Pfeil (2)] an das R_{A} tragende Stickstoffatom in Formel I gebunden ist, so dass entsprechende Verbindungen der Formel I die Formel IB, 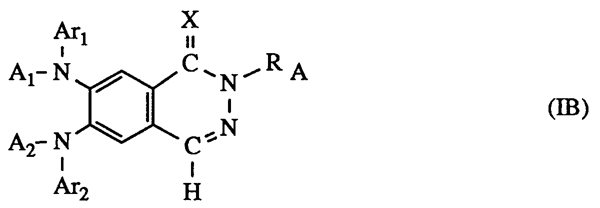
haben, worin die Reste die genannten Bedeutungen haben.

Bei der Definition jeder einzelnen der Gruppen -(C=X)- und -(C=Y)- als -(C=O)- oder -(C=S)- ist -(C=O)- bevorzugt.

Bevorzugt sind die Verbindungen der Formel IA, worin R_{A} und R_{B} jeweils Wasserstoff bedeuten, und die Verbindungen der Formel IB, worin R_{A} Wasserstoff bedeutet; wobei in beiden Fällen die übrigen Reste die für Verbindungen der Formel I genannten Bedeutungen haben.

Soweit vor- und nachstehend von Thioxo die Rede ist, ist dieser Substituent nicht an endständiges Methyl gebunden.

Salze von erfindungsgemässen Verbindungen mit salzbildenden Gruppen sind vor allem pharmazeutisch verwendbare, nicht-toxische Salze. Beispielsweise können Verbindungen der Formel I mit basischen Gruppen, z.B. primären, sekundären oder tertiären Aminogruppen (auch in Hydrazino oder Hydrazono) Säureadditionssalze bilden, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder mit Aminosäuren, wie Arginin oder Lysin. Verbindungen der Formel I mit einer sauren Gruppe, z.B. Carboxy, Sulfo oder Phospho, bilden z.B. Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wie Niederalkylaminen, z.B. Triethylamin, Hydroxyniederalkylaminen, z.B. 2-Hydroxyethylamin, Bis-(2-hydroxyethyl)-amin oder Tris-(2-hydroxyethyl)-amin, basischen aliphatischen Estern von Carbonsäuren, z.B. 4-Aminobenzoesäure-2-diethylaminoethylester, Niederalkylenaminen, z.B. 1-Ethylpiperidin, Cycloalkylaminen, z.B. Dicyclohexylamin, oder Benzylaminen, z.B. N,N'-Dibenzylethylendiamin, Dibenzylamin oder Benzyl-β-phenethylamin. Verbindungen der Formel I mit einer sauren und einer basischen Gruppe können auch in Form von inneren Salzen, d.h. in zwitterionischer Form, vorliegen.

Unter die Salze der erfindungsgemässen Verbindungen fallen auch Komplexe von Verbindungen der Formel I mit Übergangsmetallionen, z.B. Kupfer, Kobalt, Platin oder Mangan.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nicht-toxischen Salze, die deshalb bevorzugt sind.

Die Verbindungen der Formel I der vorliegenden Erfindung können als einzelne Isomere oder Isomerengemische vorliegen, sofern mehrere Isomeren möglich sind. Bei Vorliegen unsymmetrisch substituierter Doppelbindungen oder Ringe, z. B. bei substituiertem Niederalkenyl A₁ oder A₂, können die cis- und/oder die trans-Form vorliegen, bei doppelt gebundenem Stickstoff an Ringsystemen, wie in Hydroxyimino, beispielsweise die syn- oder die anti-Form. Asymmetrisch substituierte Kohlenstoffatome können in der (S)-, (R)- oder (R,S)-Form vorliegen. Es können mithin bei Vorliegen entsprechender struktureller Voraussetzungen Isomerengemische (wie Racemate oder Diastereomerengemische), reine Diastereomeren oder reine Enantiomeren vorliegen.

Soweit von den Verbindungen der vorliegenden Erfindung Tautomere vorliegen können, sei es in reiner Form oder im Gleichgewicht mit anderen Tautomeren, sind diese bei den vor- und nachstehenden Definitionen ebenfalls umfasst. Tautomerisierbare Gruppen liegen z.B. vor, wenn an O, S oder N, welche an ein Kohlenstoffatom gebunden sind, von dem eine Doppelbindung ausgeht, Wasserstoff gebunden ist, was Imin/Enamin- bzw. (Thio-) Keto/Enol-Tautomerie ermöglicht. Dies ist beispielsweise der Fall bei Verbindungen der Formel I, die Thioureido- oder Guanidinoreste oder deren wie oben definierte substituierte Derivate mit mindestens einem Wasserstoffatom an einem Stickstoffatom enthalten. Insbesondere können Tautomere vorliegen, wenn einer der Reste R_{A} und R_{B} in Verbindungen der Formel I Wasserstoff bedeutet, der andere hingegen eine der genannten Bedeutungen ausser Wasserstoff hat: Falls in Verbindungen der Formel IA R_{A} Wasserstoff, X Sauerstoff und R_{B} einen der genannten Reste ausser Wasserstoff bedeuten, wandert vorzugsweise dieser Wasserstoff unter gleichzeitiger Wanderung der Doppelbindung an X (aus -(C=X)-NH-NR_{B}'- (worin R_{B}' die für R_{B} genannten Bedeutungen ausser Wasserstoff hat) wird dann in Formel I -(C-XH)=N-R_{B}'-), falls R_{B} in Verbindungen der Formel IA Wasserstoff, Y Sauerstoff und R_{A} einen der genannten Reste ausser Wasserstoff bedeutet, wandert vorzugsweise dieser Wasserstoff unter gleichzeitiger Wanderung der Doppelbindung an Y (aus -NR_{A}'-NH-(C=Y)- (worin R_{A}' die für R_{A} genannten Bedeutungen ausser Wasserstoff hat) wird dann in Formel I -NR_{A}'-N=(C-YH)-). Es ist auch möglich, dass tautomere Formen, beispielsweise in Lösungen, im Gleichgewicht vorliegen. Alle derartigen Tautomere, deren Auftreten dem Fachmann geläufig ist, sind ebenfalls umfasst. Bevorzugt unter den tautomerisierbaren Verbindungen der Formel I sind solche, die in nur einer tautomeren Form vorliegen oder bei denen eine tautomere Form stark überwiegt.

Alle bisher untersuchten Verbindungen der Formel IA, worin Y Sauerstoff bedeutet, R_{A} einen der genannten Reste ausser Wasserstoff bedeutet und R_{B} Wasserstoff bedeutet, liegen in der tautomeren Form der Formel IC 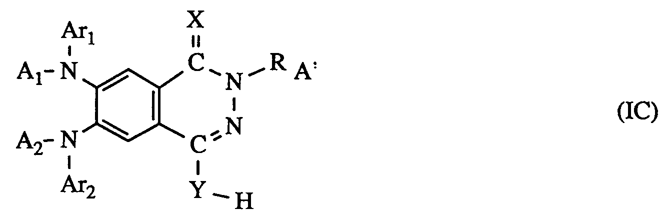
worin Y Sauerstoff bedeutet, R_{A}' einen der für R_{A} genannten Reste ausser Wasserstoff bedeutet und die übrigen Reste die genannten Bedeutungen haben. Analoge Verhältnisse können auch bei entsprechenden Verbindungen, worin Y Schwefel bedeutet, vorliegen.

Die erfindungsgemässen Verbindungen weisen wertvolle, insbesondere pharmakologisch verwendbare, Eigenschaften auf. Insbesondere zeigen sie spezifische Hemmwirkungen, die von pharmakologischem Interesse sind. In erster Linie wirken sie als Protein-Tyrosinkinase-Hemmer und zeigen z.B. eine potente Hemmung der Tyrosinkinaseaktivität des Rezeptors für den epidermalen Wachstumsfaktor (EGF) und der c-erbB2-Kinase. Diese rezeptorspezifischen Enzymaktivitäten spielen eine Schlüsselrolle in der Signalübertragung in einer Vielzahl von Säugetierzellen einschliesslich Humanzellen, insbesondere von Epithelialzellen, Zellen des Immunsystems und Zellen des zentralen und peripheren Nervensystems. Die EGF-induzierte Aktivierung der rezeptorassoziierten Protein-Tyrosinkinase (EGF-R-PTK) ist bei verschiedenen Zelltypen eine Voraussetzung für die Zellteilung und damit für die Proliferation einer Zellpopulation. Durch Zugabe von EGF-Rezeptorspezifischen Tyrosinkinasehemmern wird somit die Vermehrung dieser Zellen gehemmt.

Die Hemmung der EGF-Rezeptor-spezifischen Protein-Tyrosinkinase (EGF-R-PTK) kann z.B. mit der Methode von E. McGlynn et al. (siehe Europ. J. Biochem. 207, 265-275 (1992)) nachgewiesen werden. Die erfindungsgemässen Verbindungen hemmen die Enzymaktivität zu 50 % (IC₅₀) vorzugsweise in Konzentrationen ab etwa 0,01 µM oder höher, insbesondere von 10⁻⁷ bis 10⁻³M, z.B. von 1,2 x 10⁻⁶ bis 10⁻⁴M. Sie zeigen ferner ebenfalls im mikromolaren Bereich, vorzugsweise von 10⁻⁷ bis 10⁻³M, insbesondere von 1 x 10⁻⁶ bis 10⁻⁴M, z.B. auch eine Hemmung des Zellwachstums einer EGF-abhängigen Zellinie, etwa der epidermoiden Maus-Keratinocyten-Zellinie. Um diese Hemmung des Zellwachstums zu messen, wird die EGF-stimulierte Zellproliferation von epidermalen BALB/MK-Keratinocyten verwendet (Beschreibung des Verfahrens in Meyer, T., et al., Int. J. Cancer 43, 851 (1989)). Diese Zellen sind zur Proliferation in hohem Masse auf die Gegenwart von EGF angewiesen (Weissmann, B. E., Aaronson, S. A, Cell 32, 599 (1983). Zur Durchführung des Tests werden BALB/MK-Zellen (10 000/Vertiefung) auf 96-Loch-Mikrotiterplatten übertragen und über Nacht inkubiert. Die Testsubstanzen (gelöst in DMSO) werden in in verschiedenen Konzentrationen (in Verdünnungsreihen) zugegeben, so dass die Endkonzentration an DMSO nicht grösser als 1 % ist. Nach der Zugabe werden die Platten für drei Tage inkubiert, während denen die Kontrollkulturen ohne Testsubstanz wenigstens drei Zellteilungscyclen durchlaufen können. Das Wachstum der MK-Zellen wird gemessen mittels Methylenblau-Färbung. IC₅₀-Werte werden definiert als die Konzentration der jeweiligen Testsubstanz, die zu einer 50 %-igen Abnahme im Vergleich zu den Kontrollkulturen ohne Inhibitor führt.

Die erfindungsgemässen Verbindungen hemmen neben oder anstelle der EGF-R-PTK auch andere Tyrosinkinasen, die in die durch trophische Faktoren vermittelte Signalübertragung involviert sind, z.B. die abl-Kinase (IC₅₀ bis herunter zu etwa 10⁻⁷ M), Kinasen aus der Familie der src-Kinasen (IC₅₀ bis herunter zu etwa 10⁻⁸ M) und die c-erbB2-Kinase (HER-2), sowie Serin/Threonin-Kinasen, z.B. die Proteinkinase C (bis herunter zu 10⁻⁶ M), welche alle in der Wachstumsregulation und Transformation bei Säugetierzellen einschliesslich Humanzellen eine Rolle spielen.

Die Hemmung der c-erbB2-Tyrosinkinase (HER-2) kann z.B. analog der für EGF-R-PTK verwendeten Methode von E. McGlynn et al. (siehe Europ. J. Biochem. 207, 265-275 (1992)) nachgewiesen werden. Die c-erbB2-Kinase kann nach bekannten Protokollen isoliert und ihre Aktivität bestimmt werden, z.B. nach T. Akiyama et al., Science 232, 1644 (1986), oder vorzugsweise nach P. M. Guy et al. (siehe J. Biol. Chem. 267, 13851-13856 (1992)).

Somit sind die erfindungsgemässen Verbindungen auch zur Hemmung der durch diese und verwandte Tyrosinkinasen vermittelten Prozesse geeignet.

Die Antitumoraktivität in vivo wird beispielsweise getestet unter Verwendung des menschlichen Epithelzellkarzinoms A431 (ATCC No. CRL 1555), welches in weibliche BALB/c-Nacktmäuse (Bomholtgard, Dänemark) transplantiert wird. Für die Experimente werden in vivo aufgtretende Tumoren von etwa 1 cm³ Durchmesser unter sterilen Bedingungen aus den Tieren durch Excision gewonnen. Diese Tumoren werden homogenisiert, in 10 Volumina (w/v) Phosphat-gepufferter Saline suspendiert und s.c. (0,2 ml/Maus, z.B. 10⁶ Zellen/Maus) in die linke Flanke der Tiere injiziert. Die Behandlung mit einer Testsubstanz wird 5 bis 8 Tage nach der Transplantation eingeleitet, wenn der Durchmesser der Tumoren 4 bis 5 mm beträgt. Die jeweilige Testsubstanz [beispielsweise gelöst in ®Lauroglykol (1,2-Propylenglykol-monolaurat, Gemisch beider Konstitutionsisomeren; Gattefossé S.A., Saint Priest, Frankreich), ®Gelucire (Glyceride und Teilpolyglyceride von Fettsäure; Gattefossé S.A., Saint Priest, Frankreich) oder Sesamöl] wird p.o. täglich während 15 aufeinanderfolgenden Tagen verabreicht. Das Tumorwachstum wird durch Überwachung des senkrechten Tumordurchmessers bestimmt und das Tumorvolumen nach der Formel π x L x D²/6 berechnet (L = Länge, D = Durchmesser des Tumors senkrecht zur Tumorachse). Die Ergebnisse kann man als Quotient Behandlung/Kontrolle (T/C) in Prozent angeben.

Die erfindungsgemässen Verbindungen sind daher z.B. bei der Behandlung benigner oder maligner Tumoren nützlich. Sie sind in der Lage, Tumorregression zu bewirken und Tumormetastasierung und das Wachstum von Mikrometastasen zu verhindern. Insbesondere sind sie bei epidermaler Hyperproliferation (Psoriasis), bei der Behandlung von Neoplasien epithelialen Charakters, z.B. Mammakarzinomen, und bei Leukämien anwendbar. Ferner sind die Verbindungen bei der Behandlung von Erkrankungen des Immunsystems und von Entzündungen einzusetzen, soweit in diese Erkrankungen Proteinkinasen involviert sind. Auch bei der Behandlung von Erkrankungen des zentralen oder peripheren Nervensystems sind die Verbindungen einzusetzen, soweit Signalübertragung durch Proteinkinasen involviert ist. Schliesslich haben die Verbindungen der vorliegenden Erfindung auch antimikrobielle Eigenschaften, die sie z. B. zur Behandlung von Erkrankungen geeignet machen, die durch Bakterien, wie Salmonella typhimurium, Viren, wie Vacciniavirus, und weitere Mikroben, welche mit auf Wachstumsfaktoren reagierenden Proteinkinasen interagieren, verursacht werden.

Die erfindungsgemässen Verbindungen können alleine oder auch in Kombination mit anderen pharmakologisch wirksamen Substanzen angewandt werden, z.B. zusammen mit (a) Inhibitoren der Enzyme der Polyaminsynthese, (b) Inhibitoren der Proteinkinase C, (c) Inhibitoren anderer Tyrosinkinasen, (d) Cytokinen, (e) negativen Wachstumsregulatoren, z.B. TGF-β oder IFN-β, (f) Aromatasehemmern, (g) Antiöstrogenen oder (h) Cytostatika.

Bei den nachfolgend genannten Gruppen von Verbindungen der Formel I können allgemeine Definitionen, beispielsweise von Substituenten, unabhängig voneinander durch bei den allgemeinen Definitionen genannte spezifischere Definitionen ersetzt werden.

Bevorzugt sind Verbindungen der Formel I, worin A₁ und A₂ unabhängig voneinander Wasserstoff, Niederalkyl; substituiertes Niederalkyl, welches durch bis zu 2 Reste ausgewählt aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mercapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder vorzugsweise unsubstituiert ist, C₃-C₈-Cycloalkylamino, Phenylniederalkylamino, Phenylamino, Niederalkanoylamino, Phenylniederalkanoylamino, Phenylcarbonylamino, Hydroxy, Niederalkoxy, worin der Niederalkylrest ein- oder zweifach wie oben in Diniederalkylaminoniederalkyl A₁ oder A₂ substituiert oder vorzugsweise unsubstituiert ist, Phenylniederalkoxy, Niederalkanoyloxy, Mercapto, Niederalkylthio, worin der Niederalkylrest ein- oder zweifach wie oben in Diniederalkylaminoniederalkyl A₁ oder A₂ substituiert oder vorzugsweise unsubstituiert ist, Phenylniederalkylthio, Niederalkanoylthio, Carboxy, Niederalkoxycarbonyl, Phenylniederalkoxycarbonyl, Cyano, Carbamoyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl substituiertes Ureido, Thioureido, an einem oder beiden Stickstoffatomen durch Niederalkyl substituiertes Thioureido, Hydrazino, an einem oder beiden Stickstoffatomen durch Niederalkyl substituiertes Hydrazino, Amidino, an einem oder beiden Stickstoffatomen durch Niederalkyl substituiertes Amidino, Guanidino, an einem, zwei oder allen drei Stickstoffatomen durch Niederalkyl substituiertes Guanidino, Oxo, welches nicht am Kohlenstoff gebunden ist, welcher an den A₁ oder A₂ tragenden Stickstoff gebunden ist, Thioxo, Imino, Niederalkylimino, Niederalkanoylimino, Hydroxyimino, Niederalkoxyimino, Hydrazono, N-Mono- oder N,N-Diniederalkylhydrazono, N-Niederalkanoylhydrazono, Niederalkoxycarbonylhydrazono oder Niederalkylthioimino substituiert ist; durch einen der für substituiertes Niederalkyl A₁ und/oder A₂ genannten Reste substituiertes oder vorzugsweise unsubstituiertes Niederalkenyl oder Niederalkinyl; Heterocyclylniederalkyl, worin Heterocyclyl ein unsubstituierter oder insbesondere durch Niederalkyl, Niederalkanoyl, Hydroxy, Niederalkoxy, Halogen, Cyano-und/oder Trifluormethyl substituierter, über ein Ringstickstoffatom gebundener Rest ausgewählt aus Pyrrolyl, 2,5-Dihydropyrrolyl, Pyrrolinyl Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydrooxazolyl, Tetrahydro-isoxazolyl, Tetrahydrothiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3 4-Tetrahydrochinolyl und 1,2-Di- oder 1,2,3,4-Tetrahydroisochinolyl ist, der endständig an Niederalkyl gebunden ist; Niederalkanoyl, Halogenniederalkanoyl, Phenylniederalkanoyl, Benzoyl, Carbamoyl, N-Mono- oder N,N-Diniederalkylcarbamoyl, N-Mono- oder N,N-Bis(hydroxyniederalkyl)carbamoyl, Thiocarbamoyl, N-Mono- oder N,N-Diniederalkylthiocarbamoyl, N-Mono- oder N,N-Bis(hydroxyniederalkyl)thiocarbamoyl, Niederalkoxycarbonyl, Phenylniederalkoxycarbonyl, Niederalkylsulfonyl, Benzolsulfonyl oder im Benzolrest durch Niederalkyl, Niederalkoxy, Hydroxy, Halogen und/oder Trifluormethyl, vorzugsweise einen der genannten Reste, substituiertes Benzolsulfonyl bedeuten; oder worin A₁ und A₂ zusammen Niederalkylen bilden, welches unsubstituiert oder substituiert ist durch bis zu 3 Substituenten ausgewählt aus Niederalkyl, Amino, Aminoniederalkyl, Mono- oder Diniederalkylamino, Mono- oder Diniederalkylaminoniederalkyl, worin der endständige Niederalkylrest ein- oder zweifach wie oben in Diniederalkylaminoniederalkyl A₁ oder A₂ substituiert oder vorzugsweise unsubstituiert ist, C₃-C₈-Cycloalkylamino, C₃-C₈-Cycloalkylaminoniederalkyl, Phenylniederalkylamino, Phenylniederalkylaminoniederalkyl, Phenylamino, Phenylaminoniederalkyl, Niederalkanoylamino, Phenylniederalkanoylamino, Phenylcarbonylamino, Niederalkanoylaminoniederalkyl, Phenylniederalkanoylaminoniederalkyl, Phenylcarbonylaminoniederalkyl, Hydroxy, Hydroxyniederalkyl, Niederalkoxy oder Niederalkoxyniederalkyl, worin der endständige Niederalkylrest ein- oder zweifach wie oben in Diniederalkylaminoniederalkyl A₁ oder A₂ substituiert oder vorzugsweise unsubstituiert ist, Phenylniederalkoxy, Phenylniederalkoxyniederalkyl, Niederalkanoyloxy, Niederalkanoyloxyniederalkyl, Mercapto, Mercaptoniederalkyl, Niederalkylthio oder Niederalkylthioniederalkyl, worin der endständige Niederalkylrest ein- oder zweifach wie oben in Diniederalkylaminoniederalkyl A₁ oder A₂ substituiert oder vorzugsweise unsubstituiert ist, Phenylniederalkylthio, Phenylniederalkylthioniederalkyl, Niederalkanoylthio, Niederalkanoylthioniederalkyl, Carboxy, Carboxyniederalkyl, Niederalkoxycarbonyl, Niederalkoxycarbonylniederalkyl, Phenylniederalkoxycarbonylniederalkyl, Cyano, Cyanoniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylniederalkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl substituiertes Ureido oder Ureidoniederalkyl, Thioureido, Thioureidoniederalkyl, an einem oder beiden Stickstoffatomen durch Niederalkyl substituiertes Thioureido oder Thioureidoniederalkyl, Hydrazino, Hydrazinoniederalkyl, an einem oder beiden Stickstoffatomen durch Niederalkyl substituiertes Hydrazino oder Hydrazinoniederalkyl, Amidino, Amidinoniederalkyl, an einem oder beiden Stickstoffatomen durch Niederalkyl substituiertes Amidino oder Amidinoniederalkyl, Guanidino, Guanidinoniederalkyl, an einem, zwei oder allen drei Stickstoffatomen durch Niederalkyl substituiertes Guanidino oder Guanidinoniederalkyl, Oxo, Oxoniederalkyl, Thioxo, Thioxoniederalkyl, Imino, Iminoniederalkyl, Niederalkylimino, Niederalkyliminoniederalkyl, Niederalkanoylimino, Niederalkanoyliminoniederalkyl, Hydroxyimino, Hydroxyiminoniederalkyl, Niederalkoxyimino, Niederalkoxyiminoniederalkyl, Hydrazono, Hydrazononiederalkyl, N-Mono- oder N,N-Diniederalkylhydrazono, N-Mono- oder N,N-Diniederalkylhydrazononiederalkyl, N-Niederalkanoylhydrazono, Niederalkoxycarbonylhydrazono, N-Niederalkanoylhydrazononiederalkyl, Niederalkoxycarbonylhydrazononiederalkyl, Niederalkylthioimino und Niederalkylthioiminoniederalkyl; Ar₁ und Ar₂ unabhängig voneinander unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl, Pentafluorphenyl, über ein Ringstickstoffatom gebundenes Heteroaryl ausgewählt aus Imidazolyl, Triazolyl, Pyridyl, Pyrimidinyl und Triazinyl, welche unsubstituiert oder durch Niederalkyl, Hydroxy, Niederalkoxy, Halogen, Cyano und/oder Trifluormethyl substituiert sind, oder C₃-C₈-Cycloalkyl, welches unsubstituiert oder durch Niederalkoxy oder Hydroxy substituiert ist, bedeuten; X für Sauerstoff oder Schwefel steht; und worin Q einen bivalenten Rest der Formel 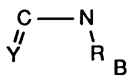
bedeutet, welcher über sein Y tragendes Kohlenstoffatom an das Q bindende Kohlenstoffatom des Benzolringes und über sein R_{B} tragendes Stickstoffatom an das R_{A} tragende Stickstoffatom in Formel I gebunden ist, worin Y unabhängig von X für Sauerstoff oder Schwefel steht und R_{A} und R_{B} unabhängig voneinander Wasserstoff, unsubstituiertes oder substituiertes Niederalkyl, wie oben für A₁ und A₂ definiert, insbesondere unsubstituiertes oder durch bis zu zwei Reste aus Amino, Mono- oder Diniederalkylamino, Hydroxy und Niederalkoxy substituiertes Niederalkyl; Niederalkanoyl, Halogenniederalkanoyl, Phenylniederalkanoyl, Benzoyl; Carbamoyl, N-Mono- oder N,N-Diniederalkylcarbamoyl, N-Mono- oder N,N-Bis(hydroxyniederalkyl)carbamoyl, Thiocarbamoyl, N-Mono- oder N,N-Diniederalkylthiocarbamoyl, N-Mono- oder N,N-Bis(hydroxyniederalkyl)thiocarbamoyl, Niederalkoxycarbonyl oder Phenylniederalkoxycarbonyl bedeuten; oder worin Q (als alternative oder weitere Bedeutung von Q, bezogen auf den zuletzt genannten bivalenten Rest) einen bivalenten Rest der Formel 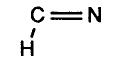
bedeutet, welcher über sein Kohlenstoffatom an das Q bindende Kohlenstoffatom des Benzolringes und über sein Stickstoffatom an das R_{A} tragende Stickstoffatom in Formel I gebunden ist, wobei R_{A} unsubstituiertes oder substituiertes Niederalkyl, vorzugsweise wie oben definiert, bedeutet; Salze davon, sofern salzbildende Gruppen vorliegen, und Tautomere davon, sofern tautomerisierbare Gruppen vorliegen.

Stark bevorzugt sind Verbindungen der Formel I, worin A₁ und A₂ unabhängig voneinander Wasserstoff, Niederalkyl; substituiertes Niederalkyl, welches durch bis zu 2 Reste, vorzugsweise einen Rest ausgewählt aus Amino, Mono- oder Diniederalkylamino, Hydroxy, Niederalkoxy, Carboxy, Niederalkoxycarbonyl, Phenylniederalkoxycarbonyl, Cyano, Carbamoyl, N-Niederalkylcarbamoyl, N,N-Diniederalkylcarbamoyl, Ureido, an einem oder beiden Stickstoffatomen durch Niederalkyl substituiertes Ureido, Thioureido oder an einem oder beiden Stickstoffatomen durch Niederalkyl substituiertes Thioureido substituiert ist; Niederalkenyl oder Niederalkinyl bedeuten; oder worin A₁ und A₂ zusammen Niederalkylen bilden, welches unsubstituiert oder substituiert ist durch Niederalkyl, Amino, Aminoniederalkyl, Mono- oder Diniederalkylamino, Hydroxy, Hydroxyniederalkyl, Niederalkoxy oder Niederalkoxyniederalkyl, Carboxy, Carboxyniederalkyl, Niederalkoxycarbonyl, Niederalkoxycarbonylniederalkyl, Cyano, Cyanoniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, Thiocarbamoyl, Thiocarbamoylniederalkyl, N-Niederalkylthiocarbamoyl, N-Niederalkylthiocarbamoylniederalkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl substituiertes Ureido oder Ureidoniederalkyl, Thioureido, Thioureidoniederalkyl oder an einem oder beiden Stickstoffatomen durch Niederalkyl substituiertes Thioureido oder Thioureidoniederalkyl; Ar₁ und Ar₂ unabhängig voneinander unsubstituiertes oder durch einen Rest ausgewählt aus Niederalkyl, wie Methyl oder Ethyl, Hydroxy, Niederalkoxy, wie Methoxy, Halogen, wie Fluor oder Iod, Carboxy, Niederalkoxycarbonyl, wie Methoxycarbonyl oder Ethoxycarbonyl, und Cyano o-, m- oder p-substituiertes Phenyl, Pentafluorphenyl oder C₃-C₈-Cycloalkyl bedeuten, vorzugsweise beide den gleichen Rest; X für Sauerstoff oder Schwefel steht; und worin Q einen bivalenten Rest der Formel 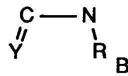
bedeutet, welcher über sein Y tragendes Kohlenstoffatom an das Q bindende Kohlenstoffatom des Benzolringes und über sein R_{B} tragendes Stickstoffatom an das R_{A} tragende Stickstoffatom in Formel I gebunden ist, worin Y unabhängig von X für Sauerstoff oder Schwefel steht, wobei R_{A} und R_{B} unabhängig voneinander Wasserstoff, unsubstituiertes oder durch einen Rest ausgewählt aus Amino, Mono- oder Diniederalkylamino, Hydroxy und Niederalkoxy substituiertes Niederalkyl; Niederalkanoyl, Carbamoyl, N-Mono- oder N,N-Diniederalkylcarbamoyl, N-Mono- oder N,N-Bis(hydroxyniederalkyl)carbamoyl, Thiocarbamoyl, N-Mono- oder N,N-Diniederalkylthiocarbamoyl, N-Mono- oder N,N-Bis(hydroxyniederalkyl)thiocarbamoyl, Niederalkoxycarbonyl oder Phenylniederalkoxycarbonyl bedeuten; oder worin Q (als alternative oder weitere Bedeutung von Q, bezogen auf den zuletzt genannten bivalenten Rest)einen bivalenten Rest der Formel 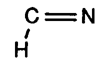
bedeutet, welcher über sein Kohlenstoffatom an das Q bindende Kohlenstoffatom des Benzolringes und über sein Stickstoffatom an das R_{A} tragende Stickstoffatom in Formel I gebunden ist, wobei R_{A} unsubstituiertes oder substituiertes Niederalkyl, vorzugsweise wie oben definiert, bedeutet; Salze davon, sofern salzbildende Gruppen vorliegen, und Tautomere davon, sofern tautomerisierbare Gruppen vorliegen.

Stärker bevorzugt sind Verbindungen der Formel IA, worin A₁ und A₂ unabhängig voneinander Wasserstoff oder Niederalkyl bedeuten, oder worin A₁ und A₂ zusammen Niederalkylen, insbesondere Ethylen, bedeuten; Ar₁ und Ar₂ unabhängig voneinander unsubstituiertes oder durch Halogen oder Niederalkyl in o-, m- oder p-Stellung, insbesondere in p-Stellung, substituiertes Phenyl bedeuten; X und Y unabhängig voneinander Sauerstoff oder Schwefel, vorzugsweise beide das gleiche der genannten Radikale, insbesondere Sauerstoff, bedeuten; und R_{A} und R_{B} unabhängig voneinander Wasserstoff, Niederalkyl, Hydroxyniederalkyl, wie 2-Hydroxyethyl, Carbamoyl oder Thiocarbamoyl bedeuten; und Tautomere davon, sofern tautomerisierbare Gruppen vorliegen.

Stärker bevorzugt sind auch Verbindungen der Formel IB, worin A₁ und A₂ unabhängig voneinander Wasserstoff oder Niederalkyl bedeuten, oder worin A₁ und A₂ zusammen Niederalkylen, insbesondere Ethylen, bedeuten; Ar₁ und Ar₂ unabhängig voneinander unsubstituiertes oder durch Halogen oder Niederalkyl in o-, m- oder p-Stellung, insbesondere in p-Stellung, substituiertes Phenyl oder Cyclohexyl, welches unsubstituiert oder durch Niederalkyl, wie Methyl oder Ethyl, Hydroxy oder Halogen, wie Fluor oder Chlor, substituiert ist, bedeuten; X Sauerstoff oder Schwefel, insbesondere Sauerstoff, bedeuten; und R_{A} Wasserstoff, Niederalkyl, Hydroxyniederalkyl, wie 2-Hydroxyethyl, Carbamoyl oder Thiocarbamoyl bedeutet; und Tautomere davon, sofern tautomerisierbare Gruppen vorliegen. Sofern Y Sauerstoff bedeutet, R_{A} einen der zuletzt genannten Reste ausser Wasserstoff bedeutet und R_{B} Wasserstoff bedeutet, liegt vorzugsweise die tautomere Form der Formel IC mit den entsprechenden Substituenten vor.

Besonders bevorzugt sind Verbindungen der Formel IA, worin A₁ und A₂ Wasserstoff bedeuten, Ar₁ und Ar₂ unabhängig voneinander einen Rest ausgewählt aus Phenyl, 4-Fluorphenyl oder 4-Methylphenyl bedeuten, vorzugsweise beide den gleichen der genannten Reste, X und Y unabhängig voneinander Sauerstoff oder Schwefel, insbesondere jeweils jeder der beiden Sauerstoff, bedeuten und R_{A} und R_{B} unabhängig voneinander Wasserstoff, Methyl, 2-Hydroxyethyl oder Carbamoyl bedeuten; und Tautomere davon, sofern tautomerisierbare Gruppen vorliegen. Sofern Y Sauerstoff bedeutet, R_{A} einen der zuletzt genannten Reste ausser Wasserstoff bedeutet und R_{B} Wasserstoff bedeutet, liegt vorzugsweise die tautomere Form der Formel IC mit den entsprechenden Substituenten vor.

Besonders bevorzugt sind auch Verbindungen der Formel IB, worin A₁ und A₂ Wasserstoff bedeuten, Ar₁ und Ar₂ unabhängig voneinander einen Rest ausgewählt aus Phenyl und Cyclohexyl bedeuten, X Sauerstoff oder Schwefel, insbesondere Sauerstoff bedeutet, und R_{A} Wasserstoff bedeutet.

Von allen bisher genannten Verbindungen der Formel I sind jeweils solche in allererster Linie bevorzugt, worin A₁ und A₂ Wasserstoff bedeuten; oder worin einer der beiden Reste A₁ und A₂ Wasserstoff bedeutet, der andere unter die Definition unsubstituiertes oder durch einen der jeweils genannten Substituenten substituiertes Niederalkyl fällt; oder worin A₁ und A₂ zusammen einen Rest bedeuten, der unter die Definitionen von unsubstituiertem oder durch einen der jeweils genannten Substituenten substituiertem Niederalkylen, insbesondere unsubstituiertem Niederalkylen, fällt; während die übrigen Reste die genannten Bedeutungen haben, pharmazeutisch verwendbare Salze davon, sofern salzbildende Gruppen vorliegen, und Tautomere davon, sofern tautomerisierbare Gruppen vorliegen.

Ganz besonders bevorzugt von allen vorgenannten Verbindungen sind diejenigen, welche durch Formel IA, worin jeder der Reste R_{A} und R_{B} Wasserstoff bedeutet und die übrigen Reste die genannten Bedeutungen haben, oder die Verbindungen der Formel IB, worin die Reste die genannten Bedeutungen haben, Salze davon, sofern salzbildende Gruppen vorliegen, und Tautomere davon, sofern tautomerisierbare Gruppen vorliegen.

Am stärksten bevorzugt sind die einzelnen in den Beispielen genannten Verbindungen der Formel I, deren Salze, sofern salzbildende Gruppen vorliegen, und deren Tautomeren, sofern tautomerisierbare Gruppen vorliegen, und die dort genannten Verfahren zu deren Herstellung; oder pharmazeutische Präparate mit diesen Verbindungen als Wirkstoff, diese Verbindungen zur Anwendung in einem therapeutischen Verfahren zur Behandlung des menschlichen oder tierischen Körpers und die Verwendung dieser Verbindungen zur therapeutischen Behandlung des menschlichen oder tierischen Körpers oder zur Herstellung pharmazeutischer Präparate zur Behandlung von proteinkinastabhängigen Erkrankungen.

Die Verbindungen der Formel I können nach an sich bekannten Verfahren erhalten werden, indem man z.B.
a) zur Herstellung von Verbindungen der Formel I, worin Q einen bivalenten Rest der Formel 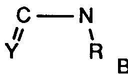 bedeutet, welcher über sein Y tragendes Kohlenstoffatom an das Q bindende Kohlenstoffatom des Benzolringes und über sein R_{B} tragendesStickstoffatom an das R_{A} tragende Stickstoffatom in Formel I gebunden ist, worin Y unabhängig von X für Sauerstoff oder Schwefel steht, R_{A} und R_{B} unabhängig voneinander Wasserstoff, unsubstituiertes oder substituiertes Niederalkyl oder Acyl bedeuten und die übrigen Reste die genannten Bedeutungen haben, eine Dicarbonsäure der Formel II, 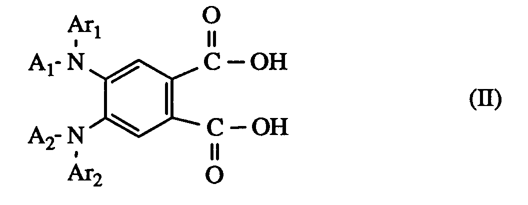 worin A₁, A₂, Ar₁ und Ar₂ die genannten Bedeutungen haben, oder ein reaktionsfähiges Derivat davon, worin die Reste die für Verbindungen der Formel I genannten Bedeutungen haben, mit einer Hydrazinverbindung der Formel III,

   R_{A}-NH-NH-R_{B} (III)

   worin R_{A} und R_{B} die für Verbindungen der Formel I genannten Bedeutungen haben, umsetzt, wobei die Verbindungen der Formeln II und/oder III bei Vorliegen salzbildender Gruppen auch in Form von Salzen eingesetzt werden können und in den Verbindungen der Formeln II und/oder m funktionelle Gruppen, die nicht an der Reaktion teilnehmen sollen, erforderlichenfalls in geschützter Form vorliegen, und vorhandene Schutzgruppen abspaltet, oder
b) zur Herstellung von Verbindungen der Formel I, worin Q einen bivalenten Rest der Formel 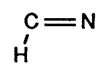 bedeutet, welcher über sein Kohlenstoffatom an das Q bindende Kohlenstoffatom des Benzolringes und über sein Stickstoffatom an das R_{A} tragende Stickstoffatom in Formel I gebunden ist, wobei R_{A} unsubstituiertes oder substituiertes Niederalkyl bedeutet, ein Formylbenzoesäurederivat der Formel XIII, 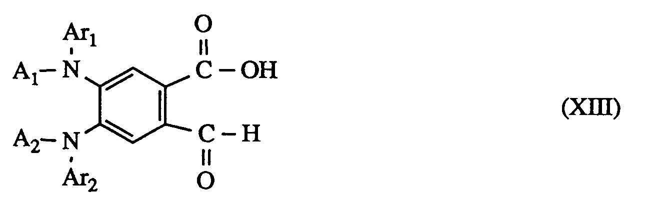 worin die Reste die zuletzt genannten Bedeutungen haben, oder ein reaktionsfähiges Derivat davon, mit einer Verbindung der Formel XIV,

   R_{A}-NH-NH₂ (XIV)

   worin R_{A} die zuletzt genannten Bedeutungen hat, umsetzt, wobei funktionelle Gruppen, die nicht an der Reaktion teilnehmen sollen, erforderlichenfalls geschützt sind, und vorhandene Schutzgruppen abspaltet,
   und gewünschtenfalls als zusätzliche Verfahrensmassnahme eine erhältliche Verbindung der Formel I in eine andere Verbindung der Formel I umwandelt, und/oder ein erhältliches Salz in die freie Verbindung oder in ein anderes Salz umwandelt, und/oder eine erhältliche freie Verbindung der Formel I in ein Salz umwandelt und/oder ein erhältliches Gemisch von Isomeren von Verbindungen der Formel I in die Isomeren auftrennt.

In der folgenden näheren Beschreibung der Verfahren haben die Symbole A₁, A₂, Ar₁, Ar₂, X, Y, R_{A} und R_{B} jeweils die für Verbindungen der Formel I genannten Bedeutungen, sofern nichts anderes angegeben ist.

### Genauere Beschreibung der Verfahren:

### Verfahren a) (Diamidbildung)

Dicarbonsäuren der Formel II liegen frei oder insbesondere als reaktionsfähige Derivate vor, wobei eine oder beide Carboxygruppen derivatisiert sein können, vorzugsweise beide, beispielsweise als aktiviertes Säurederivat oder als reaktionsfähiges inneres Anhydrid. Die reaktionsfähigen Derivate können ferner auch in situ gebildet werden. Es können beide Carboxygruppen gleichzeitig oder der Reihe nach umgesetzt werden. Vorzugsweise werden auf diese Weise Verbindungen der Formel erhalten, in denen R_{A} und R_{B} die genannten Bedeutungen ausser Acyl hat; aus erhaltenen Verbindungen der Formel I, worin R_{A} und/oder R_{B} Wasserstoff bedeutet, können dann im Rahmen der zusätzlichen Verfahrensmassnahmen (siehe unten) solche Verbindungen der Formel I erhalten werden, worin R_{A} und/oder R_{B} Acyl bedeutet. Diese können auch erhalten werden durch Umsetzung geschützter Derivate der Formel III, worin einer der Reste R_{A} und R_{B} Acyl bedeutet, während anstelle des anderen eine Schutzgruppe steht. Nach Abspaltung der Schutzgruppe kann dann in einem weiteren Reaktionschritt der freigesetzte Stickstoff, der ein Wasserstoffatom trägt, mit der noch nicht derivatisierten zweiten Carboxygruppe umgesetzt werden. Auch die Umsetzung einer an einem Carboxyrest geschützten Verbindung der Formel II mit einer Verbindung der Formel III, Abspaltung der Carboxyschutzgruppe und erneute Umsetzung unter Bildung eines Phthalazindionderivates ist möglich.

Die freie Carbonsäure der Formel II kann beispielsweise durch starke Säuren, wie Halogenwasserstoff-, Schwefel-, Sulfon-, oder Carbonsäure oder saure Ionenaustauscher, z.B. durch Chlor-, Bromwasserstoff- oder Iodwasserstoffsäure, Schwefelsäure, eine gegebenenfalls, z.B. durch Halogen, substituierte Alkancarbonsäure oder durch eine Säure der Formel II, vorzugsweise mit einem Überschuss der Säure der Formel II, erforderlichenfalls unter Bindung von entstehendem Reaktionswasser durch wasserbindende Mittel, unter azeotroper Abdestillation des Reaktionswassers oder unter extraktiver Veresterung, durch Säureanhydride, insbesondere anorganische Säureanhydride, beispielsweise Carbonsäureanhydride, z.B. Niederalkancarbonsäureanhydride (ausser Ameisensäureanhydrid), wie Acetanhydrid, oder durch geeignete Aktivierungs- oder Kupplungsreagentien der nachstehend aufgeführten Art, insbesondere auch in situ, aktiviert werden.

Als Aktivierungs- und Kupplungsreagentien zur Aktivierung von Carbonsäuren der Formel II in situ können insbesondere Carbodiimide, z.B. N,N'-Di-C₁-C₄-alkyl- oder N,N'-Di-C₅-C₇-cycloalkyl-carbodiimid, wie Diisopropylcarbodiimid oder N,N'-Dicyclohexylcarbodiimid, vorteilhaft unter Zusatz eines Aktivierungskatalysators, wie N-Hydroxysuccinimid oder gegebenenfalls, z.B. durch Halogen, C₁-C₇-Alkyl oder C₁-C₇-Alkoxy, substituiertes N-Hydroxy-benzotriazol oder N-Hydroxy-5-norbornen-2,3-dicarboxamid, C₁-C₄-Alkylhalogenformiat, z.B. Isobutylchlorformiat, geeignete Carbonylverbindungen, z.B. N,N-Carbonyldiimidazol, geeignete 1,2-Oxazoliumverbindungen, z.B. 2-Ethyl-5-phenyl-1,2-oxazolium-3'-sulfonat oder 2-tert-Butyl-5-methyl-isoxazolium-perchlorat, geeignete Acylaminoverbindungen, z.B. 2-Ethoxy-1-ethoxycarbonyl-1,2-dihydrochinolin, oder geeignete Phosphorylcyanamide bzw. -azide, z.B. Diethylphosphorylcyanamid oder Diphenylphosphorylazid, ferner Triphenylphosphin-disulfid oder 1-C₁-C₄-Alkyl-2-halogeno-pyridinium-halogenide, z.B. 1-Methyl-2-chlorpyridinium-iodid, eingesetzt werden.

Als aktiviertes Säurederivat kommt beispielsweise eine Verbindung der Formel IIa 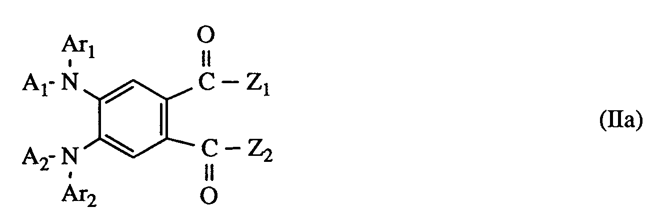
in Betracht, worin Z₁ und/oder Z₂ Hydroxy oder insbesondere reaktionsfähig aktiviertes Hydroxy bedeutet, wobei höchstens einer der beiden Reste Z₁ und Z₂ für Hydroxy steht. Z₁ und/oder Z₂ kann vorzugsweise für eine Azidogruppe (erhältlich beispielsweise durch Umsetzung eines entsprechenden Säureesters über das entsprechende Hydrazid und dessen Behandlung mit salpetriger Säure); Halogen, insbesondere Chlor oder Brom (erhältlich beispielsweise durch Umsetzung mit organischen Säurehalogeniden, insbesondere mit Oxalyldihalogeniden, wie Oxalyldichlorid, oder vor allem mit anorganischen Säurehalogeniden, z.B. mit Säurehalogeniden des Phosphors oder Schwefels, wie Phosphortrichlorid, Phosphortribromid, Phosphorpentachlorid, Phosphorpentabromid, Phosphoroxidchlorid, Phosphoroxidbromid, Thionylchlorid oder Thionylbromid); für Cyanmethoxy, durch elektronenziehende Substituenten, wie Nitro, Chlor oder Fluor in o-, m- und/oder p-Stellung substituiertes Phenyloxy, z.B. Nitrophenoxy, wie 4-Nitrophenoxy oder 2,4-Dinitrophenyoxy, oder Polyhalogenphenoxy, wie Pentachlorphenoxy (herstellbar z.B. durch Behandeln der entsprechenden Säure mit Chloracetonitril in Gegenwart einer Base, bzw. durch Umsetzung der Säure mit dem entsprechenden Phenol, z.B. Nitrophenol oder Polyhalogenphenol, in Gegenwart eines Kondensationsmittels, wie N,N'-Dicyclohexylcarbodiimid); oder für ein asymmetrisches Säureanhydrid stehen, welches beispielsweise durch Einwirkung eines Salzes, z.B. eines Alkalimetallsalzes, wie des Natrium- oder Kaliumsalzes, einer Säure der Formel II oder ihres Reaktionspartners, vorzugsweise einer Niederalkancarbonsäure, wie Essigsäure, auf ein jeweils komplementäres Säurehalogenid, insbesondere im Falle der Reaktion mit einem Salz einer Carbonsäure der Formel II ein Carbonsäurehalogenid, z.B. -Chlorid, oder im Falle der Reaktion eines Carbonsäurehalogenides der Formel IIa, worin Z₁ und Z₂ Halogen, z.B. Chlor oder Brom, bedeuten, mit einem Salz einer Niederalkancarbonsäure, insbesondere Natrium- oder Kaliumacetat, erhalten werden kann. Z₁ und Z₂ bedeuten bevorzugt Halogen, wie Chlor oder Brom, 4-Nitrophenyloxy sowie Acyloxy, z.B. Niederakanoyloxy, wie Acetyloxy.

Innere Anhydride von Dicarbonsäuren der Formel II sind vor allem solche der Formel IIb, 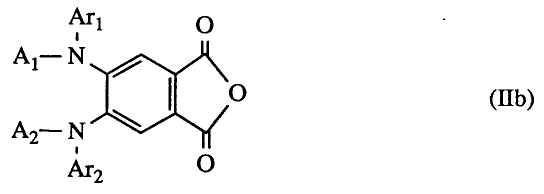
worin A₁, A₂, Ar₁ und Ar₂ die unter Formel I angegebenen Bedeutungen haben. Diese Anhydride eignen sich bei Umsetzung mit Verbindungen der Formel III insbesondere zur Herstellung von Verbindungen der Formel I, worin R_{A} und R_{B} die genannten Bedeutungen ausser Acyl haben. Die Reaktion findet vorzugsweise in inerten Lösungsmitteln, beispielsweise Ethern, z.B. Diniederalkylethern, wie Diethylether, oder cyclischen Ethern, wie Dioxan oder Tetrahydrofuran, in Alkoholen, wie Methanol oder Ethanol, oder in N,N-Diniederalkylcarbonsäureamiden, wie Dimethylformamid oder Dimethylacetamid, bei Temperaturen zwischen 0 °C und Rückflusstemperatur, vorzugsweise zwischen 40 °C und Rückflusstemperatur, z.B. bei etwa 50 °C bis etwa 67 °C oder einer niedrigeren Rückflusstemperatur, statt, erforderlichenfalls in Gegenwart von Schutzgas, wie Stickstoff oder Argon. Innere Anhydride der Formel IIb sind besonders bevorzugt als Säurederivate von Verbindungen der Formel II.

Falls in den Ausgangsmaterialien der Formel III R_{A} und/oder R_{B} Acyl bedeutet, findet die Umsetzung besonders vorteilhaft mit reaktionsfähigen Verbindungen der Formel II, worin Z₁ und Z₂ Halogen, z.B. Chlor oder Brom, bedeuten, in Gegenwart von starken Basen, wie von Erdalkalimetallhydriden, z.B. Natriumhydrid, statt, vorzugsweise in inerten Lösungsmitteln, z.B. Säureamiden, wie Dimethylformamid oder 1,3-Dimethyl-3,4,5,6-tetrahydro-2(1H)-pyrimidinon, bei Temperaturen zwischen 0 und 50 °C, z.B. bei Raumtemperatur.

Die Ausgangsmaterialien der Formel II sind beispielsweise erhältlich durch Hydrolyse von Dicarbonsäureestern der Formel IV, 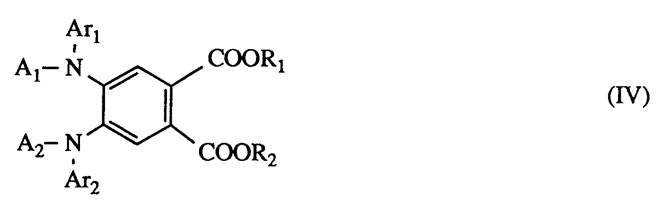
worin R₁ und R₂ Niederalkyl, insbesondere Methyl, bedeuten und die übrigen Reste die genannten Bedeutungen haben, vorzugsweise im sauren oder alkalischen Medium, erhalten. Beispielsweise erfolgt die Hydrolyse der Verbindung der Formel IV in einer wässrigen alkoholischen Lösung einer Hydroxybase, z.B. mit einer Lösung eines Alkalimetallhydroxides, wie Natrium- oder Kaliumhydroxid, in einem Gemisch aus Wasser und Ethanol oder Methanol, bei bevorzugten Temperaturen von 0 °C bis zur Rückflusstemperatur des entsprechenden Reaktionsgemisches, insbesondere zwischen etwa 60 °C und der Rückflusstemperatur. Besonders bevorzugt ist die Reaktionsführung unter Sauerstoffausschluss, beispielsweise unter Schutzgas, wie Argon oder Stickstoff. Sind die Niederalkylreste R₁ und/oder R₂ am bindenden Kohlenstoffatom verzweigt, so ist auch saure Hydrolyse möglich, beispielsweise unter Verwendung von Schwefelsäure oder Halogenwasserstoffsäuren, wie Chlorwasserstoffsäure. Auch die Herstellung aus Analogen der Verbindungen der Formel IV, worin anstelle von R₁ und/oder R₂ Carboxyschutzgruppen, beispielsweise die unten genannten, vorliegen, durch Schutzgruppenabspaltung ist möglich. Die Analogen der Verbindungen der Formel IV, worin anstelle von R₁ und/oder R₂ Carboxyschutzgruppen vorliegen, werden beispielsweise erhalten, indem man unten anstelle der Verbindungen der Formel V analoge Verbindungen verwendet, worin anstelle von R₁ und/oder R₂ eine Carboxyschutzgruppe vorliegt (herstellbar analog wie die Verbindungen der Formel V) und hieraus die den Verbindungen der Formel IV analogen Verbindungen mit Carboxyschutzgruppen anstelle von R₁ und/oder R₂ herstellt durch analoge Reaktionen, wie unten für Verbindungen der Formel IV selbst beschrieben. Durch Schutzgruppenabspaltung unter geeigneten, vorzugsweise den unten genannten Bedingungen zur Schutzgruppenabspaltung, können aus erhaltenen Analogen der Verbindungen der Formel IV die Verbindungen der Formel II gewonnen werden.

Die Ausgangsmaterialien der Formel IV werden z.B. dadurch hergestellt, dass man ein Cyclohexadien der Formel V 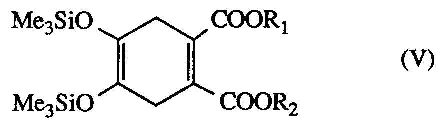
worin Me für Methyl steht (alternativ könnten auch andere Niederalkylreste vorliegen) und worin R₁ und R₂ die für Verbindungen der Formel IV genannten Bedeutungen haben, mit einem Anilin der Formel VI

AHN-Ar (VI)

worin A insbesondere Wasserstoff, unsubstituiertes oder substituiertes Niederalkyl, unsubstituiertes oder substituiertes Niederalkenyl, unsubstituiertes oder substituiertes Niederalkinyl, Heterocyclylniederalkyl, Acyl, Niederalkylsulfonyl oder Arylsulfonyl bedeutet, wie oben für A₁ oder A₂ definiert, und worin Ar Aryl, Heteroaryl oder unsubstituiertes oder substituiertes Cycloalkyl bedeutet, wie oben für Ar₁ oder Ar₂ definiert, oder mit einer Dianilinoniederalkylenverbindung der Formel VI',

Ar₁-NH-K-NH-Ar₂ (VI')

worin Ar₁ und Ar₂ die für Verbindungen der Formel I genannten Bedeutungen haben, z.B. Phenyl bedeuten, und K substituiertes oder vorzugsweise unsubstituiertes Niederalkylen bedeutet, wie oben für aus A₁ und A₂ zusammen gebildetes unsubstituiertes oder substituiertes Niederalkylen definiert, unter Säurekatalyse, beispielsweise in einer Niederalkancarbonsäure, wie Essigsäure, bei Temperaturen zwischen 80 °C und der Rückflusstemperatur, z.B. zwischen 100 und etwa 140 °C, umsetzt [s. Matlin, Stephen A. and Barron, Kenneth, J. Chem. Res. Synop. 8, 246-247 (1990)]. Hierbei sind in den Ausgangsmaterialien funktionelle Gruppen, die nicht an der Reaktion teilnehmen sollen, erforderlichenfalls geschützt. Vorhandene Schutzgruppen können auf einer geeigneten Reaktionsstufe entfernt werden. Schutzgruppen, ihre Einführung und ihre Abspaltung sind unten beschrieben. Substituenten an den Resten Ar können auch nach der Kondensation durch Methoden der klassischen Aromaten- bzw. Heterocyclenchemie oder durch enzymatische Methoden (z.B. 4-Hydroxylierungen) eingeführt werden.

Die Herstellung der Verbindungen der Formel V erfolgt z.B. via einer Diels-Alder-Reaktion aus einem 2,3-Bis(triniederalkylsilyloxy)butadien und einem Acetylendicarbonsäure-diniederalkylester, ist ebenfalls in der angegebenen Literatur (Matlin et al.) beschrieben und analog wie dort durchführbar.

Zur Herstellung von unsymmetrischen Verbindungen der Formel IV, worin A₁ und A₂ und/oder Ar₁ und Ar₂ verschieden sind, können beispielsweise Verbindungen der Formel V mit zwei unterschiedlichen Verbindungen der Formel VI - z.B. stufenweise - umgesetzt und die gewünschten Verbindungen der Formel IV durch eine chromatographische Trennung, z.B. an Kieselgel, isoliert werden.

Weiterhin können zur Herstellung von Verbindungen der Formel IV, worin die Reste die genannten Bedeutungen ausser aus A₁ und A₂ zusammen gebildetem unsubstituiertem oder substituiertem Niederalkylen haben, beispielsweise Verbindungen der Formel IV, worin anstelle von A₁ und A₂ Wasserstoff steht,
1) mit einem Reagens der Formel VII umgesetzt werden,

   W₃-L (VII)

   worin W₃ unsubstituiertes oder substituiertes Niederalkyl, unsubstituiertes oder substituiertes Niederalkenyl, unsubstituiertes oder substituiertes Niederalkinyl oder Heterocyclylniederalkyl bedeutet, wie oben bei der Definition von A₁ und/oder A₂ genannt, umgesetzt werden, wobei L im Falle von substituiertem Niederalkenyl oder substituiertem Niederalkinyl vorzugsweise an ein Kohlenstoffatom gebunden ist, von dem keine Mehrfachbindung ausgeht, und so gewählt ist, dass die Umsetzung durch Substitution schneller erfolgt als die Addition an die Mehrfachbindung; und L eine nukleofuge Gruppe, vorzugsweise Toluolsulfonyloxy oder Halogen, wie Chlor, Brom oder Jod, oder falls der Rest W₃ 2 oder mehr Kohlenstoffatome enthält, Oxa (-O-) oder Thia (-S-), welches an zwei vizinale Kohlenstoffatome gebunden ist (wobei ein Oxiran oder Thiiran gebildet wird, welches bei der Alkylierung, die insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, oder einem Harnstoffderivat, wie 1,3-Dimethyl-3,4,5,6-tetrahydro-2(1H)-pyrimidinon (DMPU), erfolgt, und bei anschliessender Hydrolyse unter Entstehung einer 2-Hydroxy- oder 2-Mercaptoniederalkylgruppe reagiert), oder Aza (-NH-), welches an zwei vizinale Kohlenstoffatome gebunden ist (wobei ein Aziran gebildet wird, welches bei der Umsetzung, die insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, oder einem Harnstoffderivat, wie 1,3-Dimethyl-3,4,5,6-tetrahydro-2(1H)-pyrimidinon (DMPU), erfolgt, und bei anschliessender Hydrolyse unter Bildung einer 2-Aminoniederalkylgruppe reagiert), wobei funktionelle Gruppen in Ausgangsmaterialien, die nicht an der Reaktion teilnehmen sollen, frei oder in geschützter Form vorliegen, und vorhandene Schutzgruppen auf geeigneten Reaktionsstufen erforderlichenfalls abgespalten werden.
   Die Umsetzung findet vorzugsweise in Gegenwart von einer starken Base statt, wie einem Alkalimetallhydrid, z. B. Natriumhydrid, einem Alkalimetallamid, wie Natriumamid, oder einem Alkalimetall-diniederalkylamid, wie Lithiumdiispropylamid, insbesondere von Natriumhydrid oder Natriumamid, welches z.B. als Dispersion in Öl zugesetzt werden kann, unter Verwendung eines gegenüber der molaren Menge der Verbindung der Formel IV, worin anstelle von A₁ und A₂ je ein Wasserstoffatom steht, äquimolaren Menge oder eines Überschusses der Base, beispielsweise der 1- bis 5-fachen molaren Menge, vor allem der 1- bis 2-fachen molaren Menge, bei Temperaturen zwischen Raumtemperatur und der Rückflusstemperatur des Reaktionsgemisches, insbesondere zwischen etwa 80 und etwa 100 °C, in aprotischen, insbesondere polaren Lösungsmitteln, wie Säureamiden, z. B. Dimethylformamid, 1,3-Dimethyl-3,4,5,6-tetrahydro-2(1H)-pyrimidinon (DMPU) oder Hexamethylphosphorsäuretriamid, oder Gemischen von solchen Lösungsmitteln, in An- oder Abwesenheit von Schutzgas, wie Argon oder Stickstoff, wobei im Falle der Verwendung von Alkalimetallamiden als Basen entstehender Ammoniak durch Anlegen eines Vakuums, z. B. von 0,1 bis 100, insbesondere von 0,5 bis 10 Torr, entfernt wird; und/oder
2) mit einer Säure der Formel VII',

   W₃'-L' (VII')

   worin W₃' Acyl, Niederalkylsulfonyl oder Arylsulfonyl bedeutet, wie oben bei der Definition von A₁ und/oder A₂ genannt, oder einem reaktionsfähigen Derivat hiervon umgesetzt werden, wobei L' Hydroxy oder einen Rest wie oben für Z₁ in Verbindungen der Formel IV definiert bedeutet, insbesondere Halogen, unter Bedingungen analog den bei der Umsetzung von Verbindungen der Formel II mit Verbindungen der Formel III beschriebenen.

Sollen Verbindungen der Formel I hergestellt werden, worin nur einer der Reste A₁ und A₂ die oben genannten Bedeutungen ausser Wasserstoff hat, während der andere Wasserstoff bedeutet, wird die Reaktion mit einer molaren Menge der Verbindung der Formel VII oder VII' durchgeführt, welche vorzugsweise der 0,2- bis 2-fachen molaren Menge, z. B. der 1- bis 1,6-fachen molaren Menge, der Verbindung der Formel IV, worin anstelle von A₁ und A₂ Wasserstoff steht, entspricht. Sollen beide Wasserstoffatome in der Verbindung der Formel IV, worin für A₁ und A₂ Wasserstoff steht, durch die bei der Definition von A₁ und A₂ genannten Reste ausser Wasserstoff ersetzt werden, so wird vorzugsweise ein Überschuss, z. B. ein 2- bis 10-, insbesondere ein etwa zwei- bis dreifacher Überschuss der Verbindung der Formel VII oder VII' eingesetzt.

Die Verbindungen der Formeln V, VI, VI', VII und VII' sind bekannt, kommerziell erhältlich oder nach an sich bekannten Verfahren herstellbar.

In den Verbindungen der Formel VII ist Niederalkyl (oder ferner Niederalkenyl oder Niederalkinyl) W₃ vorzugsweise unsubstituiert oder substituiert durch geschütztes Amino, insbesondere in geschütztem Aminoniederalkyl, z.B. Phthalimidoniederalkyl, wie Phthalimidopropyl; beispielsweise durch Niederalkoxycarbonyl, wie tert-Butyloxycarbonyl, geschütztes und im Mononiederalkylrest unsubstituiertes unsubstituiertes oder wie oben substituiertes (und dann erforderlichenfalls an den Substituenten geschütztes) Mononiederalkylamino, insbesondere in entsprechend geschütztem und substituiertem Mononiederalkylaminoniederalkyl; in den beiden N-Niederalkylresten unsubstituiertes oder wie oben definiert substituiertes (und dann erforderlichenfalls an Substituenten geschütztes) Diniederalkylamino, insbesondere in entsprechendem unsubstituiertem oder substituiertem Diniederalkylaminoniederalkyl; N-geschütztes Cycloalkylamino, insbesondere in N-geschütztem Cycloalkylaminoniederalkyl; N-geschütztes Phenylniederalkylamino, insbesondere in N-geschütztem Phenylniederalkylaminoniederalkyl; N-geschütztes Phenylamino, insbesondere in N-geschütztem Phenylaminoniederalkyl; Acylamino, wie in Acylaminoniederalkyl; geschütztes Hydroxy, insbesondere in Hydroxyniederalkyl, worin die Hydroxygruppe geschützt vorliegt; Niederalkoxy, worin der endständige Niederalkylrest unsubstituiert oder wie oben substituiert (und dann erforderlichenfalls mit geschützten Substituenten) vorliegt, wie in entsprechendem substituiertem geschütztem oder unsubstituiertem Niederalkoxyniederalkyl; Phenylniederalkoxy, insbesondere in Phenylniederalkoxyniederalkyl; Acyloxy, insbesondere in Acylniederalkoxyniederalkyl; geschütztes Mercapto, insbesondere in Mercaptoniederalkyl, worin die Mercaptogruppe in geschützter Form vorliegt; Niederalkylthio, worin der endständige Niederalkylrest unsubstituiert oder wie oben substituiert (erforderlichenfalls mit geschützten Substituenten) vorliegt; insbesondere in entsprechend substituiertem geschütztem oder unsubstituiertem Niederalkylthioniederalkyl; Phenylniederalkylthio, wie in Phenylniederalkylthioniederalkyl; Acylthio, wie in Acylthioniederalkyl; geschütztes Carboxy, wie in geschütztem Carboxyniederalkyl; verestertes Carboxy, wie in verestertem Carboxyniederalkyl; Cyano, wie in Cyanoniederalkyl; Oxo, wie in Oxoniederalkyl (erforderlichenfalls geschützt durch Acetalbildung, z.B. mit Niederalkanolen, insbesondere Ethan-1,2-diol, wobei die Schutzgruppe auf der gewünschten Stufe durch Hydrolyse in Gegenwart einer Säure, wie Essigsäure oder Schwefelsäure, abgespalten werden kann); und/oder Thioxo, insbesondere in Thioxoniederalkyl ausser Thioxomethyl (erforderlichenfalls geschützt durch Thioacetalbildung, z. B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann), oder Heterocyclyl, wie in erforderlichenfalls geschütztem Heterocyclylniederalkyl.

Benutzt man anstelle einer Verbindung der Formel VII eine entsprechende Verbindung, in der am Rest W₃ (welcher hier wie oben für A₁ und A₂ definiertes unsubstituiertes oder substituiertes Niederalkyl bedeutet) zusätzlich zu einem der genannten nukleofugen Reste L ein Halogenatom vorliegt, so kann man durch Eliminierung von Halogenwasserstoff in Gegenwart der zu Reaktion verwendeten Base, z. B. von Natriumamid, welche beispielsweise im Überschuss eingesetzt wird, unter den oben genannten Reaktionsbedingungen die entsprechende unsubstituierte oder substituierte Niederalkenylverbindung erhalten. Analog kann eine Verbindung mit unsubstituiertem oder substituiertem Niederalkinylrest aus entsprechenden Analogen der Verbindung der Formel VII erhalten werden, in denen zusätzlich zu einem der genannten nukleofugen Reste L zwei weitere Halogenatome vorliegen.

In erhältlichen Verbindungen der Formel IV können Carboxygruppen, die in A₁ und/oder A₂ als Substituent vorliegen, in Carbamoyl-, N-Mono- oder N,N-Diniederalkylcarbamoyl-, N-Hydroxycarbamoyl- oder N-Phenylcarbamoylgruppen (auch in N-Aryl- und N-Arylniederalkylcarbamoylgruppen) umgewandelt werden, beispielsweise durch Umsetzung mit Ammoniak, einem Niederalkylamin oder einem Diniederalkylamin, Hydroxylamin oder Phenylamin, in Gegenwart eines Kondensationsmittels, z.B. einem Carbodiimid, wie Dicyclohexylcarbodiimid oder einem polaren Derivat davon, in polaren organischen Lösungsmitteln, wie Ethanol, oder in Gegenwart von einem N.N'-Carbonyldiazolid, wie N.N'-Dicarbonylimidazol (vgl. H. A. Staab, Angew. Chem. 74, 407-423 (1962)) in inerten organischen Lösungsmitteln, z.B. chlorierten Kohlenwasserstoffen, wie Methylenchlorid oder Chloroform, oder in Ethern, wie Diethylether, Tetrahydrofuran oder Dioxan, über das entsprechende Carbonsäureazolid. Man erhält so die entsprechenden Verbindungen der Formel IV, worin A₁ und/oder A₂ Carbamoyl, N-Mono- oder N,N-Diniederalkylcarbamoyl, N-Hydroxycarbamoyl oder N-Phenylcarbamoyl enthält. Entsprechende Thiocarbamoyl-,N-Mononiederalkylthiocarbamoyl- oder N,N-Diniederalkylcarbamoylsubstituenten in A₁ und A₂ kann man aus Carboxy durch Umwandlung in ein Carbonylhalogenid, z.B. mit anorganischen Säurehalogeniden, wie Phosphortrichlorid, Phosphorpentachlorid oder Thionylchlorid, oder organischen Säurehalogeniden, wie Oxalyldichlorid, und anschliessende Umsetzung z.B. mit Phosphorpentachlorid, Schwefelwasserstoff und Ammoniak, einem Niederalkylamin oder einem N,N-Diniederalkylamin erhalten.

In Verbindungen der Formel IV, worin A₁ und/oder A₂ z.B. ein Hydroxyniederalkyl bedeuten, kann durch nukleophile Substitution die Hydroxygruppe in eine freie oder durch Niederalkyl, Aryl und/oder Arylniederalkyl N-substituierte Hydrazinogruppe, eine Guanidinogruppe oder eine durch Niederalkyl, Aryl und/oder Arylniederalkyl N-substituierte Guanidinogruppe umgewandelt werden. Beispielsweise kann eine Hydroxyverbindung durch Umsetzung mit aromatischen Sulfonsäuren oder aktivierten Derivaten davon, wie den entsprechenden aromatischen Sulfonsäurehalogeniden, z. B. Toluolsulfonsäurehalogeniden, wie Toluolsulfonsäurechlorid, in Ab- oder vorzugsweise Anwesenheit von geeigneten Basen, z.B. tertiären Stickstoffbasen, wie Triethylamin oder N-Methylmorpholin, in den entsprechenden Ester der aromatischen Sulfonsäure überführt werden. Dann kann dieser Ester mit Hydrazin, Guanidin, oder den entsprechend substituierten Derivaten, oder Salzen davon, wobei auch Schutzgruppen vorliegen können, unter den Bedingungen der nukleophilen Substitution umgesetzt werden, vorzugsweise in Gegenwart von organischen Lösungsmitteln, z.B. Alkoholen, wie Methanol, Ethanol oder Trifluorethanol, Ketonen, wie Aceton, Nitrilen, wie Acetonitril, Estern, wie Essigsäureethylester, Ethern, wie Diethylether, Ethylenglykoldimethylether, Tetrahydrofuran oder Dioxan, Säureamiden, wie Dimethylformamid, Bisalkansulfinen, wie Dimethylsulfoxid, Arylalkoholen, wie Phenol, oder auch von Wasser, oder Gemischen dieser Lösungsmittel, gegebenenfalls (z.B. zur Umsetzung von an Aryl gebundenem Stickstoff) in inerten organischen Lösungsmitteln, wie Dimethylformamid oder 1,3-Dimethyl-3,4,5,6-tetrahydro-2(1H)-pyrimidinon, unter Zugabe starker Basen, wie Natriumamid oder Natriumhydrid. Erforderlichenfalls werden vorhandene Schutzgruppen abgespalten. Man erhält Verbindungen der Formel IV, worin A₁ und/oder A₂ Substituenten ausgewählt aus Hydrazino, an einem oder beiden Stickstoffatomen durch Niederalkyl, Aryl oder Arylniederalkyl substituiertem Hydrazino, Guanidino und an einem, zwei oder allen drei Stickstoffatomen durch Niederalkyl, Aryl oder Arylniederalkyl substituiertem Guanidino enthält. Diejenigen Verbindungen der Formel IV, worin Substituenten ausgewählt aus Hydrazino und am endständigen N-Atom durch Niederalkyl, Aryl und/oder Arylniederalkyl substituiertem Hydrazino vorliegen, kann man auch erhalten ausgehend von einer entsprechenden Oxoverbindung der Formel IV durch deren Umsetzung mit Stickstoffbasen ausgewählt aus Hydrazin und durch Niederalkyl, Aryl und/oder Arylniederalkyl an einem der beiden N-Atome bis zu zweifach substituiertem Hydrazin, wie weiter unten für die Umsetzung einer Oxoverbindung mit Stickstoffbasen beschrieben, und anschliessende Reduktion einer erhaltenen entsprechenden Iminoverbindung, vorzugsweise durch katalytische Hydrierung mit selektiven Hydrierkatalysatoren, insbesondere in Gegenwart von Palladium auf festen Trägermaterialien, z.B. auf Kohle, in polaren organischen oder organisch-wässrigen Lösungsmitteln oder Lösungsmittelgemischen, insbesondere in Ethern, z.B. cyclischen Ethern, wie Tetrahydrofuran oder Dioxan, oder in Alkoholen, wie Niederalkanolen, z.B. Methanol oder Ethanol, oder Gemischen davon, z.B. Methanol/Tetrahydrofuran-Gemischen, bei Temperaturen zwischen -20 und 60 ⁰C, z.B. zwischen 0 und 40 °C, wie bei Raumtemperatur.

In Verbindungen der Formel IV, worin A₁ und/oder A₂ z.B. ein Cyanoniederalkyl bedeuten, können Cyanogruppen z.B. durch partielle Hydrolyse, im Sinne einer Graf-Ritter-Reaktion oder über Imino-niederalkylester-Salze in Carbamoyl- oder N-Niederalkylcarbamoylgruppen in Verbindungen der Formel IV überführt werden. Die Bedingungen bei der Hydrolyse des Cyano-Zwischenproduktes können so gewählt werden, dass die Reaktion auf der Stufe des Amids abgebrochen wird. Zu diesem Zweck ist insbesondere die Hydrolyse mit Säuren geeignet, wobei z.B. 80%-ige Schwefelsäure (unter Erwärmen), Polyphosphorsäure (bei 110-150 °C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen. Ebenfalls mit Hilfe der Graf-Ritter-Reaktion gelingt die Herstellung N-monoalkylierter Amide der Formel IV aus den entsprechenden Nitrilen. Hierzu setzt man die Nitrile in Gegenwart einer starken Säure, vornehmlich 85-90%-iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen oder Alkoholen. Die Imino-niederalkylester der Formel IV erhält man z.B. durch säurekatalysierte Anlagerung von Alkoholen an die Nitrilvorläufer. Diese Anlagerung kann alternativ durch Basen, z.B. Alkoholate wie Natriummethoxid, katalysiert werden. Setzt man anstelle von Alkoholen entsprechende Mercaptane ein, beispielsweise in Gegenwart von Stickstoffbasen, wie Triethylamin oder N-Methylmorpholin, so erhält man die entsprechenden Imino-niederalkylthioester. Aus den Imino-niederalkylestem erhält man die Carbamoylderivate, aus den Imino-niederalkylthioestern die entsprechenden Thiocarbamoylderivate im Sinne einer Pinner-Spaltung durch thermischen Zerfall der Iminoester-Salze bei Temperaturen oberhalb von etwa 80 °C. Die Thiocarbamoylverbindungen können auch direkt erhalten werden durch Umsetzung von Cyanogruppen mit Schwefelwasserstoff analog der partiellen Hydrolyse, beispielsweise in Gegenwart von tertiären Aminen, wie Triethylamin.

Verbindungen der Formel IV, worin A₁ und/oder A₂ z.B. ein Amidinoniederalkyl oder ein an einem Stickstoffatom durch bis zu zwei Reste ausgewählt aus Niederalkyl, Aryl und Arylniederalkyl substituiertes Amidinoniederalkyl bedeuten, lassen sich durch Umsetzung einer entsprechenden Imino-niederalkylester- oder Imino-niederalkylthiolestervorstufe (als Säureadditionssalz, z.B. -(C=NH)-OC₂H₅·HCl bzw. -C(=NH)-SC₂H₅·HI, wie oben beschrieben hergestellt aus einer entsprechenden Cyanoverbindung) mit Ammoniak oder dem entsprechenden Niederalkyl-, Aryl- oder Arylniederalkylamin herstellen. Die Cyanovorstufen können beispielsweise auch durch Umsetzung mit einem Alkalimetallamid, oder durch Reaktion mit einem entsprechenden Ammoniumsalz, z.B. einem entsprechenden Ammoniumhalogenid, in die freien, mono- oder disubstituierten Amidine überführt werden. Verbindungen der Formel IV, worin A₁ und/oder A₂ ein an allen beiden Stickstoffatomen durch Aryl, Arylniederalkyl oder Niederalkyl substituiertes Amidino enthalten, lassen sich aus den (analog wie oben für Niederalkylcarbamoyl beschrieben herstellbaren) Verbindungen herstellen, worin in Formel IV anstelle von Carbamoyl in A₁ und/oder A₂ durch Niederalkyl, Aryl oder Arylniederalkyl N-substituiertes Carbamoyl als Substituent enthalten ist, z. B. durch Umsetzung mit POCl₃ oder PCl₅ in die entsprechenden Imidsäurechloride (z.B. -(C=NH-niederalkyl)-Cl), welche nach Reaktion mit Ammoniak bzw. einem primären oder sekundären Amin substituierte Amidine der Formel IV ergeben (vgl. Chem. Abstr. 81, 91186a (1974)).

In erhältlichen Verbindungen der Formel IV können Aminogruppen, die in A₁ und/oder A₂ als Substituent vorliegen, in Ureido- oder an einem oder beiden Stickstoffatomen durch je bis zu einen Rest ausgewählt aus Niederalkyl, Aryl und Arylniederalkyl substituiertes Ureido umgewandelt werden, indem man Verbindungen der Formel IV, worin z.B. A₁ und/oder A₂ Aminoniederalkyl oder N-Mononiederalkylaminoniederalkyl bedeuten, oder in denen anstelle von einem oder beiden der Reste A₁ und A₂ ein Arylaminoniederalkyl und/oder ein Arylniederalkylaminoniederalkyl stehen (herstellbar z. B. durch Umsetzung von Verbindungen der Formel IV, worin anstelle von A₁ oder A₂ Wasserstoff steht, mit Analogen der Verbindungen der Formel VII, worin ausser der nukleofugen Gruppe L noch eine weitere nukleofuge Gruppe, z.B. Halogen, enthalten ist, unter Bedingungen analog der Umsetzung von Verbindungen der Formel IV, worin anstelle von A₁ und/oder A₂ Wasserstoff steht, unter erneuter Anwendung analoger Reaktionsbedingungen und Substitution des zweiten nukleofugen Restes entweder mit einem Arylamin oder einem Arylniederalkylamin, oder aus Verbindungen der Formel IV, worin A₁ und/oder A₂ ein Hydroxyniederalkyl bedeuten, indem man die Hydroxygruppe in einen nukleofugen Rest, z.B. durch Behandlung mit einem aromatischen Sulfonsäurehalogenid, wie Toluolsulfonsäurechlorid, überführt und diesen dann mit einem Arylamin oder einem Arylniederalkylamin unter Bedingungen analog der Umsetzung von Verbindungen der Formel IV, worin anstelle von A₁ und/oder A₂ Wasserstoff steht, mit Verbindungen der Formel VII umsetzt), mit einem Niederalkyl-, Aryl- oder Arylniederalkylisocyanat oder N-geschütztem Isocyanat (z. B. Benzylisocyanat) umsetzt, vorzugsweise in einem Ether, z.B. einem cyclischen Ether, wie Tetrahydrofuran, bei bevorzugten Temperaturen zwischen -20 und 60 °C, insbesondere etwa bei Raumtemperatur, wobei funktionelle Gruppen, die nicht an der Reaktion teilnehmen sollen, erforderlichenfalls geschützt sind, und vorhandene Schutzgruppen auf einer geeigneten Reaktionsstufe abspaltet.

Analog kann man in Verbindungen der Formel IV Aminogruppen, die in A₁ und/oder A₂ als Substituent vorliegen, in Thioureido- oder an einem oder beiden Stickstoffatomen durch je bis zu einen Rest ausgewählt aus Niederalkyl, Aryl oder Arylniederalkyl substituiertes Thioureido umwandeln, indem man anstelle der Isocyanate entsprechende Thioisocyanate verwendet.

Verbindungen der Formel IV, worin A₁ und/oder A₂ z.B. ein am endständigen Stickstoff durch 2 Reste ausgewählt aus Niederalkyl, Aryl und Arylniederalkyl substituiertes Ureidoniederalkyl bedeuten, können beispielsweise hergestellt werden, indem man entsprechende Aminoniederalkylverbindungen der Formel IV mit Phosgen oder Analogen davon, z. B. N.N'-Carbonyldiazoliden, wie N.N'-Carbonyldiimidazol (vgl. H. A. Staab, Angew. Chem. 74, 407-423 (1962)), umsetzt und die entstehenden Chlorocarbonyl- oder Azolidocarbonyl-aminoverbindungen dann mit durch 2 Reste ausgewählt aus Niederalkyl, Aryl und Arylniederalkyl substituiertem Ammoniak umsetzt, oder die entsprechenden Aminoniederalkylverbindungen der Formel IV mit dem Reaktionsprodukt von durch 2 Reste ausgewählt aus Niederalkyl, Aryl und Arylniederalkyl substituiertem Ammoniak mit Phosgen oder Analogen davon, z.B. den N.N'-Carbonyldiazoliden, wie N.N'-Carbonyldiimidazol, umsetzt unter Erhalt der entsprechend substituierten Ureidoverbindungen. Die Reaktionen werden vorzugsweise in inerten Lösungsmitteln, insbesondere chlorierten Kohlenwasserstoffen, wie Methylenchlorid oder Chloroform, Ethern, wie Diethylether, Tetrahydrofuran oder Dioxan, oder Säureamiden, wie Dimethylformamid, bei Temperaturen zwischen -20 °C und der Rückflusstemperatur, insbesondere zwischen 0 und 30 °C, durchgeführt.

Verbindungen der Formel IV, worin A₁ und/oder A₂ z.B. ein am endständigen Stickstoff durch 2 Reste ausgewählt aus Niederalkyl, Aryl und Arylniederalkyl substituiertes Thioureidoniederalkyl enthalten, können auf analoge Weise beispielsweise hergestellt werden, indem man entsprechende Aminoniederalkylverbindungen der Formel IV mit Thiophosgen oder Analogen davon, z. B. N.N'-Thiocarbonyldiazoliden, wie N.N'-Thiocarbonyldiimidazol (vgl. H. A. Staab, Angew. Chem. 74, 407-423 (1962), umsetzt und die entstehenden Chlorothiocarbonyl- oder Azolidothiocarbonyl-aminoverbindungen dann mit durch 2 Reste ausgewählt aus Niederalkyl, Aryl und Arylniederalkyl substituiertem Ammoniak umsetzt, oder umgekehrt die entsprechenden Aminoniederalkylverbindungen der Formel IV mit dem Reaktionsprodukt von durch 2 Reste ausgewählt aus Niederalkyl, Aryl und Arylniederalkyl substituiertem Ammoniak mit Thiophosgen oder Analogen davon, z.B. den N.N'-Thiocarbonyldiazoliden, wie N.N'-Thiocarbonyldiimidazol, umsetzt.

In Verbindungen der Formel IV kann z.B. ein Hydroxyniederalkyl A₁ und/oder A₂ zur entsprechenden Oxo-Verbindung oxidiert werden. Im Falle von primären Alkoholen ist hier-zu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstempertur, z.B. bei etwa 0 bis 40 °C, statt. Im Falle von sekundären Alkoholen kann ausserdem mit weniger selektiven Oxidationsmitteln, wie Chromsäure, Dichromat/Schwefelsäure, Dichromat/Eisessig, Salpetersäure, Braunstein, Selendioxid oder Dimethylsulfoxid in Gegenwart von Oxalylchlorid, in Wasser, wässrigen oder organischen Lösungsmitteln, wie halogenierten Kohlenwasserstoffen, z.B. Methylenchlorid, oder Carbonsäureamiden, wie Dimethylformamid, oxidiert werden, vorzugsweise bei Temperaturen zwischen -50 °C und Rückflusstemperatur, insbesondere zwischen - 10 und 50 °C. Man erhält Verbindungen der Formel IV, in denen der Rest A₁ und/oder A₂ Oxo trägt.

Verbindungen der Formel IV, worin A₁ und/oder A₂ Imino, Niederalkylimino, Acylimino, Hydroxyimino, Niederalkoxyimino, Hydrazono, N-Mono- oder N,N-Diniederalkylhydrazono und/oder N-Acylhydrazono enthalten, z.B. als Subsituenten in einem substituierten Niederalkyl, können aus entsprechenden Oxo-Verbindungen der Formel IV hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in welchem die (vorzugsweise wie zuletzt beschrieben durchgeführte) Oxidation einer Hydroxy- zu einer Oxoverbindung durchgeführt wird.

So kann eine Oxoverbindung durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in ein entsprechendes Iminoderivat überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder -dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt werden kann; in Wasser (in An- oder Abwesenheit von Tensiden), einem wässrigen Lösungsmittelgemisch, wie einem Gemisch von Wasser mit einem oder mehreren Alkoholen, z. B. Methanol, Ethanol oder Isopropanol, Diniederalkylsulfoxiden, wie Dimethylsulfoxid, oder Diniederalkylniederalkanoylamiden, wie Dimethylformamid, organischen Lösungsmitteln, wie Alkoholen, z. B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen - 20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur; umgesetzt wird.

Oxoverbindungen der Formel IV können in die entsprechenden Thioxoverbindungen überführt werden, beispielsweise durch Umsetzung mit Phosphorpentasulfid oder vorzugsweise Phosphorpentasulfid-Ersatzstoffen, wie Laweson's Reagenz (= 2,4-Bis-(4-methoxyphenyl)-2,4-dithioxo-1,3,2,4-dithiaphosphetan), wobei die Reaktion in inerten organischen Lösungsmitteln, beispielsweise in einem halogenierten Kohlenwasserstoff, wie Dichlormethan, bei Temperaturen von 30 °C bis zur Rückflusstemperatur, insbesondere unter Rückfluss, durchgeführt wird.

Verbindungen der Formel IV, worin A₁ und/oder A₂ Acyliminosubstituenten enthalten, z.B. als Substituenten in einem substituierten Niederalkyl, können aus entsprechenden Iminoausgangsmaterialien erhalten werden durch deren Umsetzung mit einer freien Säure, welche den Acylrest enthält, beispielsweise in Gegenwart von Kondensationsmitteln, wie Carbodiimiden, z. B. Dicyclohexylcarbodiimid, oder mit einem aktivierten Säurederivat davon, z. B. einem Carbonsäurehalogenid, gegebenenfalls in Gegenwart einer geeigneten Base, z.B. eines tertiären Amins, wie bereits definiert, vorzugsweise unter Feuchtigkeitsausschluss.

Verbindungen der Formel IV, worin A₁ und/oder A₂ Niederalkylthioiminosubstituenten tragen, z.B. an substituiertem Niederalkyl, können vorzugsweise hergestellt werden, indem man entsprechende Iminoausgangsmaterialien der Formel IV mit Niederalkylsulfenylhalogeniden (herstellbar z. B. aus Sulfensäuren mit Halogenwasserstoff oder durch Chlorolyse, Bromolyse oder Jodolyse von entsprechenden Organo-Schwefel-Verbindungen, wobei die Herstellung auch in situ erfolgen kann), insbesondere Niederalkylsulfenyl-halogeniden, wie Methylsulfenylchlorid, umsetzt, vorzugsweise unter Verwendung der Salze der Iminoverbindungen oder in Gegenwart von Alkalihydroxiden, wie Natrium- oder Kaliumhydroxid, vorzugsweise in organischen Lösungsmitteln, z.B. Kohlenwasserstoffen, wie Heptan, Ethern, wie Diethylether, Dioxan oder Tetrahydrofuran, oder Carbonsäureamiden, wie Dimethylformamid, bei bevorzugten Temperaturen zwischen 0 °C und der Rückflusstemperatur, insbesondere zwischen 0 und 30 °C.

Verbindungen der Formel IV, worin A₁ und/oder A₂ zusätzlich zu oder anstelle von substituiertem Niederalkyl aus substituiertem Niederalkenyl und substituiertem Niederalkinyl ausgewählt sind, können, auch wenn diese in den Abschnitten für die Herstellung von Verbindungen der Formel IV nicht explizit erwähnt sind, in sinnvoller und zweckmässig Weise durch analoge Herstellungsverfahren dargestellt werden können, wie für die Verbindungen der Formel IV mit substituierten Niederalkykesten beschrieben.

Verbindungen der Formel IV, worin A₁ und/oder A₂ z.B. Heterocyclylniederalkyl bedeuten, können aus entsprechenden Verbindungen der Formel IV, worin wenigstens einer der Substituenten A₁ und A₂ Wasserstoff bedeutet oder aus entsprechenden Analogen, worin anstelle von A₁ und A₂ jeweils Wasserstoff steht, erhalten werden, vorzugsweise durch Umsetzung mit Verbindungen der Formel VIII,

Heterocyclylniederalkyl-L'' (VIII)

worin Heterocyclylniederalkyl wie oben definiert ist und L'' eine nukleofuge Gruppe bedeutet, wie oben für L bei Verbindungen der Formel VII definiert, unter nukleophiler Substitution der nukleofugen Gruppe L''. Die Reaktionsbedingungen entsprechen dabei vorzugsweise den bei der Alkylierungsreaktion mit Verbindungen der Formel VII genannten Bedingungen in Gegenwart einer starken Base.

Zur Herstellung von Verbindungen der Formel IV, worin A₁ und A₂ zusammen unsubstituiertes oder substituiertes Niederalkylen bedeuten, werden beispielsweise Verbindungen der Formel IV, worin anstelle von A₁ und A₂ Wasserstoff steht, mit einem bisalkylierenden Reagens der Formel IX,

L₁-B-L₂ (IX)

worin B unsubstituiertes oder substituiertes Niederalkylen als bivalentes, jeweils über eines seiner Kohlenstoffatome gebundenes Radikal (unsubstituiert oder beispielsweise substituiert durch die bei der Definition von aus A₁ und A₂ zusammen gebildetem substituiertem Niederalkylen genannten Substituenten, vorzugsweise einen dieser Substituenten) bedeutet, und worin L₁ und L₂ unabhängig voneinander eine nukleofuge Gruppe bedeuten, wie oben für L bei der Definition von Verbindungen der Formel VII beschrieben, umgesetzt, wobei erforderlichenfalls solche funktionelle Gruppen in den Ausgangsmaterialien, die nicht an der Reaktion teilnehmen sollen, durch Schutzgruppen geschützt sind, die auf einer geeigneten Reaktionsstufe entfernt werden. Die bevorzugten Schutzgruppen, ihre Einführung und ihre Abspaltung wurden oben erwähnt.

L₁ ist vorzugsweise eine nukleofuge Gruppe, insbesondere aliphatisch - oder aromatisch - substituiertes Sulfonyloxy, z.B. Methansulfonyloxy oder p-Toluolsulfonyloxy (Tosyloxy), Halogen, wie Chlor, Brom oder Jod, oder Cyano, während L₂, falls ein Niederalkylenrest B zwei oder mehr Kohlenstoffatome enthält, Oxa (-O-) oder Thia (-S-), welches an zwei vizinale Kohlenstoffatome gebunden ist (wobei ein Oxiran oder Thiiran gebildet wird, welches bei der Alkylierung, welche insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, erfolgt, und bei anschliessender Hydrolyse unter Entstehung einer 1-Hydroxy- oder 1-Mercaptoniederalkylgengruppe reagiert), oder Aza (-NH-), welches an zwei vizinale Kohlenstoffatome gebunden ist (wobei ein Aziran gebildet wird, welches bei der Alkylierung, welche insbesondere in Gegenwart einer starken Base, wie Lithium-Diisopropylamid, Natriumamid oder vor allem Natriumhydrid, bei Temperaturen zwischen 50 °C und dem Siedepunkt des Reaktionsgemisches, z. B. bei 80 bis 100 °C, in einem Säureamid, wie Dimethylformamid, erfolgt, und bei anschliessender Hydrolyse unter Bildung einer 1-Aminoniederalkylengruppe reagiert), bedeuten kann.

Die Umsetzung erfolgt vorzugsweise unter den Reaktionsbedingungen, die für die Umsetzung von Verbindungen der Formel IV, worin anstelle von A₁ und A₂ jeweils ein Wasserstoffatom steht, mit Verbindungen der Formel VII als bevorzugt beschrieben sind.

Es ist auch möglich, dass L₁ und L₂ zusammen am endständigen Kohlenstoff von B gebundenes Oxo bedeuten (die Verbindung der Formel IX ist dann ein Aldehyd), oder jeweils Niederalkoxy bedeuten (die Verbindung der Formel IX ist dann ein Acetal). Vorzugsweise ist B in Verbindungen der Formel II dann augewählt aus verestertem Carboxymethylen, wie Niederalkoxycarbonylmethylen, oder Niederalkanoylmethylen. Die Umsetzung findet beispielsweise in Gegenwart von Säuren, z.B. Halogenwasserstoffsäuren, wie Chlorwasserstoffsäure, oder vorzugsweise in Gegenwart von Lewis-Säuren, insbesondere SnCl₂ (z.B. als Hydrat), in geeigneten Lösungsmitteln oder Lösungsmittelgemischen, z.B. Ethern, wie Diniederalkoxyniederalkanen, insbesondere in 1,2-Dimethoxyethan, bei bevorzugten Temperaturen zwischen 0 und 50 °C, z.B. etwa bei Raumtemperatur, statt, erforderlichenfalls unter Wasserzusatz.

Die Verbindung der Formel IX wird vorzugsweise in der äquimolaren Menge oder im Überschuss gegenüber der Verbindung der Formel IV eingesetzt, worin anstelle von A₁ und A₂ je ein Wasserstoffatom steht, insbesondere in der 1- bis 3-fachen molaren Menge, z.B. der 1- bis 1,5-fachen molaren Menge, wie der etwa 1,2-fachen Menge. Die zur Reaktion eingesetzte starke Base wird vorzugsweise im Überschuss gegenüber der Verbindung der Formel IV eingesetzt, worin A₁ und A₂ je ein Wasserstoffatom bedeuten, insbesondere in der 2- bis 10-fachen molaren Menge, z.B. in der 2- bis 3-fachen molaren Menge.

Die Reaktion kann so geführt werden, dass die nukleofugen Gruppen L₁ und L₂ praktisch gleichzeitig in einem Ansatz substituiert werden, oder die nukleofugen Gruppen L₁ und L₂ können der Reihe nach in verschiedenen Ansätzen substituiert werden.

In den Verbindungen der Formel IX ist B vorzugsweise unsubstituiertes oder durch einen oder mehrere, insbesondere einen der folgenden Substituenten substituiertes Niederalkylen: Niederalkyl, geschütztes Amino oder Aminoniederalkyl, z.B. Phthalimido oder Phthalimidoniederalkyl, wie Phthalimidopropyl oder, beispielsweise durch Niederalkoxycarbonyl, wie tert-Butyloxycarbonyl, geschütztes und im Mononiederalkykest unsubstituiertes oder wie oben substituiertes (und dann erforderlichenfalls an den Substituenten geschütztes) Mononiederalkylamino oder Mononiederalkylamino-niederalkyl, in den beiden N-Niederalkylresten unsubstituiertes oder wie oben definiert substituiertes (und dann erforderlichenfalls an Substituenten geschütztes) Diniederalkylamino oder Diniederalkylaminoniederalkyl, N-geschütztes Cycloalkylamino oder Cycloalkylaminoniederalkyl, N-geschütztes Phenylniederalkylamino oder Phenylniederalkylaminoniederalkyl, N-geschütztes Phenylamino oder Phenylaminoniederalkyl, Acylamino oder Acylaminoniederalkyl, Hydroxy (zusätzlich zu der Definition von aus A₁ und A₂ gebildetem substituiertem Niederalkylen in Zwischenprodukten möglich) oder Hydroxyniederalkyl, wobei die Hydroxygruppe geschützt vorliegt, Niederalkoxy oder Niederalkoxyniederalkyl, worin der endständige Niederalkylrest unsubstituiert oder wie oben substituiert (und dann erforderlichenfalls mit geschützten Substituenten) vorliegt, Phenylniederalkoxy oder Phenylniederalkoxyniederalkyl, Acyloxy oder Acyloxyniederalkyl, Mercapto oder Mercaptoniederalkyl, worin die Mercaptogruppe in geschützter Form vorliegt, Niederalkylthio oder Niederalkylthioniederalkyl, worin der endständige Niederalkylrest unsubstituiert oder wie oben substituiert (und dann erforderlichenfalls mit geschützten Substituenten) vorliegt, Phenylniederalkylthio oder Phenylniederalkylthioniederalkyl, Acylthio oder Acylthioniederalkyl, geschütztes Carboxy oder Carboxyniederalkyl, verestertes Carboxy oder Carboxyniederalkyl, Cyano oder Cyanoniederalkyl, Oxo oder Oxoniederalkyl (erforderlichenfalls geschützt durch Acetalbildung, z. B. mit Niederalkanolen, insbesondere mit Ethan-1,2-diol, wobei die Schutzgruppe auf der gewünschten Stufe durch Hydrolyse in Gegenwart einer Säure, wie Essigsäure oder Schwefelsäure, abgespalten werden kann), oder Thioxo oder Thioxoniederalkyl (erforderlichenfalls geschützt durch Thioacetalbildung, z.B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann).

In erhältlichen Verbindungen der Formel IV kann Carboxy in einer Carboxy- oder Carboxyniederalkylgruppe (die in einem aus A₁ und A₂ zusammen gebildetem substituiertem Niederalkylen vorliegt) in Carbamoyl, N-Mono- oder N,N-Diniederalkylcarbamoyl, N-Hydroxycarbamoyl oder N-Phenylcarbamoyl(auch in N-Aryl- und N-Arylniederalkyl- carbamoylgruppen) umgewandelt werden, beispielsweise durch Umsetzung mit Ammoniak, einem Niederalkylamin oder einem Diniederalkylamin, Hydroxylamin oder Phenylamin (auch einem N-Aryl- oder N-Arylniederalkyl-amin), oder jeweils einem Salz davon, in Gegenwart eines Kondensationsmittels, z.B. einem Carbodiimid, wie Dicyclohexylcarbodiimid oder einem polaren Derivat davon, in polaren organischen Lösungsmitteln, wie Ethanol, oder einem N.N'-Carbonyldiazolid, wie N.N'-Dicarbonylimidazol (vgl. H. A. Staab, Angew. Chem. 74, 407-423 (1962)) in inerten organischen Lösungsmitteln, z.B. chlorierten Kohlenwasserstoffen, wie Methylenchlorid oder Chloroform, oder in Ethern, wie Diethylether, Tetrahydrofuran oder Dioxan, über das entsprechende Carbonsäureazolid. Man erhält so entsprechende Verbindungen der Formd IV, in denen ein aus A₁ und A₂ zusammen gebildetes substituiertes Niederalkylen vorliegt, welches durch Carbamoyl, N-Mono- oder N,N-Diniederalkylcarbamoyl, N-Hydroxycarbamoyl und/oder N-Phenylcarbamoyl substituiert ist. Entsprechende Thiocarbamoylsubstituenten in aus A₁ und A₂ zusammen gebildetem substituiertem Niederalkylen können aus Carboxy durch Umwandlung in ein Carbonylhalogenid, z. B. mit einem anorganischen Säurehalogenid, wie Phosphortrichlorid, Phosphorpentachlorid oder Thionylchlorid, oder einem organischen Säurehalogenid, wie Oxalyldichlorid, und anschliessende Umsetzung z.B. mit Phosphorpentachlorid, Schwefelwasserstoff und Ammoniak, einem Niederalkylamin oder einem Diniederalkylamin, Hydroxylamin oder Phenylamin erhalten werden.

In Verbindungen der Formel IV, worin ein aus A₁ und A₂ zusammen gebildetes substituiertes Niederalkylen durch Hydroxy und/oder Hydroxyniederalkyl substituiert ist, kann durch nukleophile Substitution die Hydroxygruppe in eine Hydrazino-, durch Niederalkyl, Aryl und/oder Arylniederalkyl N-substituierte Hydrazinogruppe, eine Guanidinogruppe oder eine durch Niederalkyl, Aryl und/oder Arylniederalkyl N-substituierte Guanidino- gruppe umgewandelt werden. Beispielsweise kann Hydroxy durch Umsetzung mit aromatischen Sulfonsäuren oder aktivierten Derivaten davon, wie den entsprechenden aromatischen Sulfonsäurehalogeniden, z. B. Toluolsulfonsäurehalogeniden, wie Toluolsulfonsäurechlorid, in Ab- oder vorzugsweise Anwesenheit von geeigneten Basen, z.B. tertiären Stickstoffbasen, wie Triethylamin oder N-Methylmorpholin, in durch die entsprechende aromatische Sulfonsäure verestertes Hydroxy überführt werden und dann dieser Ester mit Hydrazin, Guanidin, den entsprechend substituierten Derivaten, oder Salzen davon, wobei auch Schutzgruppen vorliegen können, unter den Bedingungen einer nukleophilen Substitution umgesetzt werden, vorzugsweise in Gegenwart von organischen Lösungsmitteln, z.B. Alkoholen, wie Methanol, Ethanol oder Trifluorethanol, Ketonen, wie Aceton, Nitrilen, wie Acetonitril, Estern, wie Essigsäureethylester, Ethern, wie Diethylether, Ethylenglykoldimethylether, Tetrahydrofuran oder Dioxan, Säureamiden, wie Dimethylformamid, Bisalkansulfinen, wie Dimethylsulfoxid, Arylalkoholen, wie Phenol, oder auch von Wasser, oder Gemischen dieser Lösungsmittel, erforderlichenfalls (z. B. zur Umsetzung von an Aryl gebundenem Stickstoff) in inerten organischen Lösungsmitteln, wie Dimethylformamid oder 1,3-Dimethyl-3,4,5,6-tetrahydro2(1H)-pyrimidinon, unter Zugabe starker Basen, wie Natriumamid oder Natriumhydrid. Falls sinvoll und zweckmässig, werden vorhandene Schutzgruppen abgespalten. Man erhält Verbindungen der Formel IV, worin A₁ und A₂ zusammen ein substituiertes Niederalkylen mit Substituenten ausgewählt aus Hydrazino, an einem oder beiden Stickstoffatomen durch Niederalkyl, Aryl oder Arylniederalkyl substituiertem Hydrazino, Guanidino und an einem, zwei oder allen drei Stickstoffatomen durch Niederalkyl, Aryl oder Arylniederalkyl substituiertem Guanidino bilden.

In Verbindungen der Formel IV, worin A₁ und A₂ zusammen ein durch Cyano und/oder Cyanoniederalkyl substituiertes Niederalkylen bilden, können Cyanogruppen z.B. durch partielle Hydrolyse, im Sinne einer Graf-Ritter-Reaktion, oder über Imino-niederalkylester-Salze in Carbamoyl- oder N-Niederalkylcarbamoylgruppen in Verbindungen der Formel IV überführt werden. Die Bedingungen bei der Hydrolyse des Cyano-Zwischenproduktes können so gewählt werden, dass die Reaktion auf der Stufe des Amids abgebrochen wird. Zu diesem Zweck ist insbesondere die Hydrolyse mit Säuren geeignet, wobei z.B. 80%-ige Schwefelsäure (unter Erwärmen), Polyphosphorsäure (bei 110-150 °C), Bromwasserstoff/Eisessig (Raumtemperatur, Ameisensäure oder ohne Lösungsmittel), HCl-Gas in etherischer Lösung gefolgt von der Zugabe von Wasser oder wässriger Salzsäure, oder Borhalogenide in Frage kommen. Mit Hilfe der Graf-Ritter-Reaktion gelingt die Herstellung N-monoalkylierter Amide der Formel IV aus den entsprechenden Nitrilen. Hierzu setzt man die Nitrile in Gegenwart einer starken Säure, vornehmlich 85-90%-iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen oder Alkoholen. Die Imino-niederalkylester der Formel IV erhält man z.B. durch säurekatalysierte Anlagerung von Alkoholen an die Cyanoverbindungen (als Salze). Diese Anlagerung kann alternativ durch Basen, z.B. Alkoholate wie Natriummethoxid, katalysiert werden. Setzt man anstelle von Alkoholen entsprechende Mercaptane ein, beispielsweise in Gegenwart von Stickstoffbasen, wie Triethylamin oder N-Methylmorpholin, so erhält man die entsprechenden Iminoniederalkylthioester. Aus den Imino-niederalkylestern erhält man die Carbamoylderivate, aus den Imino-niederalkylthioestern die entsprechenden Thiocarbamoylderivate im Sinne einer Pinner-Spaltung durch thermischen Zerfall der Iminoester-Salze bei Temperaturen oberhalb von etwa 80 °C. Die Thiocarbamoylverbindungen können auch direkt erhalten werden durch Umsetzung von Cyanogruppen mit Schwefelwasserstoff analog der partiellen Hydrolyse, beispielsweise in Gegenwart von tertiären Stickstoffbasen, wie Triethylamin.

Verbindungen der Formel IV, worin ein aus A₁ und A₂ zusammen gebildetes substituiertes Niederalkylen durch Amidino, Amidinoniederalkyl, an einem Stickstoffatom durch bis zu zwei Reste ausgewählt aus Niederalkyl, Aryl und Arylniederalkyl substituiertes Amidino und/oder an einem Stickstoffatom durch bis zu zwei Reste ausgewählt aus Niederalkyl, Aryl und Arylniederalkyl substituiertes Amidinoniederalkyl substituiert ist, lassen sich durch Umsetzung der wie oben aus entsprechenden Cyano- oder Cyanoniederalkylausgangsmaterialien hergestellten Imino-niederalkylester oder Imino-niederalkylthiolester (als Säuredditionssalze, z.B. -(C=NH)-OC₂H₅·HCl bzw. -C(=NH)-SC₂H₅·HI) herstellen, indem mit Ammoniak oder entsprechenden primären oder sekundären Niederalkyl-, Aryl- und/oder Arylniederalkylaminen umgesetzt wird. Die entsprechenden Cyanovorstufen können beispielsweise auch durch Umsetzung mit einem Alkalimetallamid, oder durch Reaktion mit einem primären oder sekundären Ammoniumsalz, z.B. einem primären oder sekundären Ammoniumhalogenid, in die entsprechenden freien, mono- oder disubstituierten Amidine überführt werden. Verbindungen der Formel IV, worin A₁ und A₂ zusammen ein substituiertes Niederalkylen bedeuten, welches ein an allen beiden Stickstoffatomen durch Aryl, Arylniederalkyl oder Niederalkyl substituiertes Amidino oder Amidinoniederalkyl als Substituenten trägt, lassen sich auch aus den (wie oben für Niederalkylcarbamoyl beschrieben herstellbaren) entsprechenden Verbindungen herstellen, worin in Formel IV durch Niederalkyl, Aryl oder Arylniederalkyl N-substituiertes Carbamoyl steht, z.B. durch Umsetzung mit POCl₃ oder PCl₅ in die entsprechenden imidsäurechloride (z.B. -(C=NH-niederalkyl)-Cl) , welche nach Reaktion mit Ammoniak bzw. einem entsprechenden Amin substituierte Amidine der Formel IV ergeben (vgl. Chem. Abstr. 81, 91186a (1974)).

In erhältlichen Verbindungen der Formel IV können in Amino- und/oder Aminoniederalkylresten (die als Substituent in einem aus A₁ und A₂ zusammen gebildeten substituierten Niederalkylen vorliegen) vorkommende Aminoreste in Ureido, Ureidoniederalkyl oder an einem oder beiden Stickstoffatomen durch je bis zu einen Rest ausgewählt aus Niederalkyl, Aryl oder Arylniederalkyl substituiertes Ureido oder Ureidoniederalkyl umgewandelt werden, indem man entsprechende Aminoverbindungen der Formel IV, in denen Aminoreste vorliegen, solche, in denen N-Mononiederalkylaminoreste vorliegen oder solche, in denen anstelle von Amino Arylamino oder Arylniederalkylamino steht (herstellbar z. B. durch Umsetzung von Verbindungen der Formel IV, worin das aus A₁ und A₂ zusammen gebildete substituierte Niederalkylen Hydroxy enthält, welches durch Veresterung, z. B. durch Umsetzung mit einem aromatischen Sulfonsäurehalogenid, wie Toluolsulfonsäurechlorid, in einen nukleofugen Rest, z.B. aromatisches Sulfonyloxy, überführt wird, analog der Umsetzung von Verbindungen der Formel IV, worin anstelle von A₁ und A₂ Wasserstoff steht, mit Verbindungen der Formel VII unter nukleophiler Substitution des nukleofugen aromatischen Sulfonyloxy entweder mit einem Arylamin oder einem Arylniederalkylamin), mit einem Niederalkyl-, Aryl- oder Arylniederalkylisocyanat oder einem N-geschützten Isocyanat (z. B. Benzylisocyanat) umsetzt, vorzugsweise in einem Ether, z.B. einem cyclischen Ether, wie Tetrahydrofuran, bei bevorzugten Temperaturen zwischen -20 und 60°C, insbesondere etwa bei Raumtemperatur, wobei funktionelle Gruppen, die nicht an der Reaktion teilnehmen sollen, erforderlichenfalls geschützt sind, und vorhandene Schutzgruppen gewünschtenfalls abspaltet.

Analog lassen sich in Verbindungen der Formel IV Aminogruppen (die als Substituent in einem aus A₁ und A₂ zusammen gebildeten substituierten Niederalkylen vorliegen) in Thioureido oder an einem oder beiden Stickstoffatomen durch je bis zu einen Rest ausgewählt aus Niederalkyl, Aryl oder Arylniederalkyl substituiertes Thioureido umwandeln, indem man anstelle der Isocyanate entsprechende Thioisocyanate verwendet.

Verbindungen der Formel IV, worin als Substituent in einem aus A₁ und A₂ zusammen gebildeten substituierten Niederalkylen am endständigen Stickstoff durch bis zu 2 Reste ausgewählt aus Niederalkyl, Aryl und Arylniederalkyl substituiertes Ureido und/oder Ureidoniederalkyl vorliegt, können beispielsweise hergestellt werden, indem man entsprechende Aminoverbindungen der Formel IV mit Phosgen oder Analogen davon, z. B. N.N'-Carbonyldiazoliden, wie N.N'-Carbonyldiimidazol (vgl. H. A. Staab, Angew. Chem. 74, 407-423 (1962), umsetzt und die entstehenden Chlorocarbonyl- oder Azolidocarbonyl-aminoverbindungen dann mit durch bis zu 2 Reste ausgewählt aus Niederalkyl, Aryl und Arylniederalkyl substituiertem Ammoniak umsetzt, oder umgekehrt die entsprechenden Aminoverbindungen der Formel IV mit dem Reaktionsprodukt von durch 2 Reste ausgewählt aus Niederalkyl, Aryl und Arylniederalkyl substituiertem Ammoniak mit Phosgen oder Analogen davon, z.B. den N.N'-Carbonyldiazoliden, wie N.N'-Carbonyldiimidazol, umsetzt unter Erhalt der entsprechend substituierten Ureidoverbindungen. Die Reaktionen werden vorzugsweise in inerten Lösungsmitteln, insbesondere chlorierten Kohlenwasserstoffen, wie Methylenchlorid oder Chloroform, Ethern, wie Diethylether, Tetrahydrofuran oder Dioxan, oder Säureamiden, wie Dimethylformamid, bei Temperaturen zwischen -20 °C und Rückflusstemperatur, insbesondere zwischen 0 und 30 °C, durchgeführt. Setzt man anstelle der Aminoverbindungen der Formel IV Analoge ein, in denen Mono-Niederalkylamino, Arylamino und/oder Arylniederalkylamino vorliegt, kann man auch die entsprechenden Verbindungen der Formel IV erhalten, worin bis zu drei Substituenten ausgewählt aus Niederalkyl, Aryl und Arylniederalkyl an den beiden Stickstoffatomen von Ureidogruppen vorliegen.

Verbindungen der Formel IV, worin als Substituent in einem aus A₁ und A₂ gebildeten substituierten Niederalkylen am endständigen Stickstoff durch 2 Reste ausgewählt aus Niederalkyl, Aryl und Arylniederalkyl substituiertes Thioureidoniederalkyl vorliegt, können auf analoge Weise beispielsweise hergestellt werden, indem man entsprechende Aminoverbindungen der Formel IV mit Thiophosgen oder Analogen davon, z. B. N.N'-Thiocarbonyldiazoliden, wie N.N'-Thiocarbonyldiimidazol (vgl. H. A. Staab, Angew. Chem 74, 407-423 (1962), umsetzt und die entstehenden Chlorothiocarbonyl- oder Azolido-thiocarbonylaminoverbindungen dann mit durch 2 Reste ausgewählt aus Niederalkyl, Aryl und Arylniederalkyl substituiertem Ammoniak umsetzt, oder umgekehrt die entsprechenden Aminoverbindungen der Formel IV mit dem Reaktionsprodukt von durch 2 Reste ausgewählt aus Niederalkyl, Aryl und Arylniederalkyl substituiertem Ammoniak mit Thiophosgen oder Analogen davon, z.B. den N.N'-Thiocarbonyldiazoliden, wie N.N'-Thiocarbonyldiimidazol, umsetzt.

Eine Verbindung der Formel IV, worin als Substituent in einem aus A₁ und A₂ zusammen gebildeten substituierten Niederalkylen Hydroxy und/oder Hydroxyniederalkyl vorliegt, kann zu einer entsprechenden Oxo-Verbindung oxidiert werden. Im Falle von primären Alkoholen ist hierzu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstemperatur, z.B. bei etwa 0 bis 40 °C, statt. Im Falle von sekundären Alkoholen kann ausserdem mit weniger selektiven Oxidationsmitteln, wie Chromsäure, Dichromat/Schwefelsäure, Dichromat/Eisessig, Salpetersäure, Braunstein und Selendioxid oxidiert werden. Man erhält Verbindungen der Formel IV, in denen ein aus A₁ und A₂ zusammen gebildetes substituiertes Niederalkylen durch Oxo substituiert ist.

Verbindungen der Formel IV, worin in einem aus A₁ und A₂ zusammen gebildeten substituierten Niederalkylen ein oder mehrere Substituenten ausgewählt aus Imino, Niederalkylimino, Acylimino, Hydroxyimino, Niederalkoxyimino, Hydrazono, N-Mono- oder N,N-Diniederalkylhydrazono, N-Acylhydrazono und einem durch Imino, Niederalkylimino, Acylimino, Hydroxyimino, Niederalkoxyimino, Hydrazono, N-Mono- oder N,N-Diniederalkylhydrazono und/oder N-Acylhydrazono substituierten Niederalkyl vorliegen, können aus entsprechenden Oxo-Verbindungen der Formel IV hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in dem die Oxidation einer Hydroxy- zur Oxoverbindung durchgeführt wird, welche beispielsweise wie zuletzt beschrieben erfolgt.

So können die Oxoverbindungen durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in die entsprechenden Iminoderivate überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder -dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt werden kann; in Wasser, einem wässrigen Lösungsmittelgemisch, wie einem Gemisch von Wasser mit einem oder mehreren Alkoholen, z. B. Methanol, Ethanol oder Isopropanol, Diniederalkylsulfoxiden, wie Dimethylsulfoxid, oder Diniederalkyiniederalkanoylamiden, wie Dimethylformamid, organischen Lösungsmitteln, wie Alkoholen, z. B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen -20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur; umgesetzt wird.

Verbindungen der Formel IV, worin als Substituent in einem aus A₁ und A₂ gebildeten substituierten Niederalkylen Acylimino und/oder Acyliminoniederalkyl vorliegt, können aus den entsprechenden Iminoverbindungen erhalten werden durch Umsetzung mit den entsprechenden freien Säuren, welche den Acylrest enthalten, beispielsweise in Gegenwart von Kondensationsmitteln, wie Carbodiimiden, z. B. Dicyclohexylcarbodiimid, oder aktivierten Säurederivaten davon, z. B. Carbonsäurehalogeniden, gegebenenfalls in Gegenwart einer geeigneten Base, z.B. eines tertiären Amins, wie bereits definiert, vorzugsweise unter Feuchtigkeitsausschluss.

Verbindungen der Formel IV, worin aus A₁ und A₂ zusammen gebildetes Niederalkylen Niederalkylthioiminosubstituenten trägt, können vorzugsweise hergestellt werden, indem man entsprechende Iminoausgangsmaterialien der Formel IV mit Niederalkylsulfenylhalogeniden (herstellbar z. B. aus Sulfensäuren mit Halogenwasserstoff oder durch Chlorolyse, Bromolyse oder Jodolyse von entsprechenden Organo-Schwefel-Verbindungen, wobei die Herstellung auch in situ erfolgen kann), insbesondere Niederalkylsulfenylhalogeniden, wie Methylsulfenylchlorid, umsetzt, vorzugsweise unter Verwendung der Salze der Iminoverbindungen oder in Gegenwart von Alkalihydroxiden, wie Natrium- oder Kaliumhydroxid, vorzugsweise in organischen Lösungsmitteln, z.B. Kohlenwasserstoffen, wie Heptan, Ethern, wie Diethylether, Dioxan oder Tetrahydrofuran, oder Carbonsäureamiden, wie Dimethylformamid, bei bevorzugten Temperaturen zwischen 0 °C und der Rückflusstemperatur, insbesondere zwischen 0 und 30 °C.

Aus den erhältlichen Verbindungen der Formel IV werden die Verbindungen der Formel II vorzugsweise durch Hydrolyse, z.B. im sauren oder alkalischen Medium, erhalten. Vorzugsweise erfolgt die Hydrolyse der Verbindung der Formel IV in einer wässrigen alkoholischen Lösung einer Hydroxybase, z.B. mit einer Lösung eines Alkalimetallhydroxides, wie Natrium- oder Kaliumhydroxid, in einem Gemisch aus Wasser und Ethanol oder Methanol, bei bevorzugten Temperaturen von 0 °C bis zur Rückflusstemperatur des entsprechenden Reaktionsgemisches, insbesondere zwischen etwa 40 °C und der Rückflusstemperatur. Besonders bevorzugt ist die Reaktionsführung unter Sauerstoffausschluss, beispielsweise unter Schutzgas, wie Argon oder Stickstoff.

Aus den Verbindungen der Formel II können die Verbindungen der Formeln IIa und IIb hergestellt werden.

Die Herstellung der Verbindungen der Formel IIa erfolgt beispielsweise wie oben beschrieben.

Innere Anhydride der Formel IIb lassen sich beispielsweise aus einer freien Dicarbonsäure der Formel II durch Umsetzung mit Säureanhydriden der Formel X,

R₃-(C=O)-O-(C=O)-R₃' (X)

worin R₃ und R₃' unabhängig voneinander für Wasserstoff oder Niederalkyl, nicht jedoch beide für Wasserstoff, stehen, insbesondere Acetanhydrid, herstellen.

Die Ausgangsmaterialien der Formel III sind bekannt, kommerziell erhältlich oder nach an sich bekannten Verfahren herstellbar.

Verbindungen der Formel III, worin einer oder beide der Reste R_{A} und R_{B} unsubstituiertes oder substituiertes Niederalkyl bedeuten, lassen sich beispielsweise herstellen, indem man eine Verbindung der Formel XI,

RA'-Q (XI)

worin R_{A}' unsubstituiertes oder substituiertes Niederalkyl, wie in Formel I für R_{A} definiert, bedeutet, und Q eine Abgangsgruppe bedeutet, mit einer Verbindung der Formel XII,

H₂N-NH-R_{B} (XII)

worin R_{B} die für Verbindungen der Formel I genannten Bedeutungen hat, umsetzt, oder indem man eine Verbindung der Formel XI'

R_{B}'-Q (XI')

worin R_{B}' die gleichen Bedeutungen hat wie R_{A}' in Verbindungen der Formel XI und Q die gleiche Bedeutung hat wie in Verbindungen der Formel XI, mit einer Verbindung der Formel XII',

R_{A}-NH-NH₂ (XII')

worin R_{A} die für Verbindungen der Formel I genannten Bedeutungen hat, umsetzt.

Die Umsetzung erfolgt unter den Bedingungen einer nukleophilen Substitution erster oder zweiter Ordnung. Beispielsweise kann man eine der Verbindungen der Formel XI oder XI', worin Q für eine Abgangsgruppe mit hoher Polarisierbarkeit der Elektronenhülle, z.B. für Brom oder Iod, steht, in einem polaren aprotischen Lösungsmittel, z.B. Aceton, Acetonitril, Nitromethan, Dimethylsulfoxid oder Dimethylformamid, umsetzen. Die Umsetzung kann auch in Wasser, dem erforderlichenfalls als Lösungsvermittler ein organisches Lösungsmittel, z.B. Ethanol, Tetrahydrofuran, Dimethylsulfoxid, Dimethylformamid oder Aceton, beigemischt ist, erfolgen. Die Substitutionsreaktion wird gegebenenfalls bei erniedrigter oder erhöhter Temperatur, z.B. in einem Temperaturbereich von etwa -40 °C bis etwa 100 °C, bevorzugt von etwa -10 °C bis etwa 50 °C, und gegebenenfalls unter Inertgas, z.B. Stickstoff- oder Argonatmosphäre, durchgeführt.

Acyl R_{A} und/oder R_{B} kann beispielsweise durch Umsetzung mit Säuren der Formeln R_{A}''-OH oder R_{B}''-OH, worin R_{A}'' und R_{B}'' jeweils die für Acyl R_{A} oder R_{B} genannten Bedeutungen haben, oder reaktionsfähigen Derivaten davon, z.B. Anhydriden, 4-Nitrophenylestern oder Säurehalogeniden, wie -chloriden oder -bromiden mit freiem Hydrazin oder einer der Verbindungen der Formeln XII oder XII' eingeführt werden unter Acylierungsbedingungen analog den für die Umsetzung von Verbindungen der Formel II oder deren reaktionsfähigen Derivaten mit Verbindungen der Formel III beschriebenen, wobei, falls R_{A}'' und/oder R_{B}'' den Rest eines Halbesters der Kohlensäure bedeutet, stets nur reaktionsfähige Derivate in Frage kommen, hergestellt beispielsweise aus dem entsprechenden Alkohol und Phosgen; während zur Einführung von Carbamoyl oder einem Acylrest einer N-monosubstituierten Carbaminsäure oder eines Thiocarbamoyl- oder N-mono-substituierten Thiocarbamoylrestes insbesondere die Umsetzung mit einem N-substituierten Isocyanat oder einem N-geschützten Isocyanat (z. B. Benzylisocyanat, aus dem durch Abspaltung der Benzylschutzgruppe Carbamoyl erhalten werden kann) oder entsprechenden Thioisocyanaten geeignet ist, welche vorzugsweise in einem Ether, z.B. einem cyclischen Ether, wie Tetrahydrofuran, bei bevorzugten Temperaturen zwischen -20 und 60 °C, insbesondere etwa bei Raumtemperatur, stattfindet, oder zur Einführung von N-disubstituiertem Carbamoyl oder Thiocarbamoyl beispielsweise die Umsetzung mit Hydrazin oder den entsprechenden Verbindungen der Formeln XII oder XII'mit Phosgen oder Analogen davon, z. B. N.N'-Carbonyldiazoliden, wie N.N'-Carbonyldiimidazol (vgl. H. A. Staab, Angew. Chem. 74, 407-423 (1962)), bzw. den entsprechenden Thioanalogen, wie Thiophosgen etc., und anschliessende Umsetzung der entstehenden Chloro(thio)- carbonyl- oder Azolido(thio)carbonyl-aminoverbindungen mit dem entsprechenden durch 2 Reste substituierten Ammoniak, oder umgekehrt durch Umsetzung der Hydrazine der Formeln XII oder XII' mit dem Reaktionsprodukt von durch 2 Reste substituiertem Ammoniak mit(Thio)Phosgen oder Analogen davon, z.B. den N,N'-(Thio-)Carbonyldiazoliden, wie N.N'-Carbonyldiimidazol die Reaktion wird vorzugsweise in inerten Lösungsmitteln, insbesondere chlorierten Kohlenwasserstoffen, wie Methylenchlorid oder Chloroform, Ethern, wie Diethylether, Tetrahydrofuran oder Dioxan, oder Säureamiden, wie Dimethylformamid, bei Temperaturen zwischen -20 °C und der Ruckflusstemperatur, insbesondere zwischen 0 und 30 °C, durchgeführt.

Verbindungen der Formeln XII oder XII', worin R_{A} oder R_{B} die für Verbindungen der Formel I genannten Bedeutungen ausser Wasserstoff haben, können auf analoge Weise unter Einführung des komplementären Rests R_{B}' oder R_{A}' alkyliert oder unter Einführung des komplementären Rests R_{B}'' oder R_{A}'' acyliert werden.

Freies Hydrazin (auch als Salz) kann auch unter simultaner Einführung von R_{A} und R_{B} umgesetzt werden.

Die Verbindungen der Formeln XI und XI' sind bekannt, kommerziell erhältlich oder nach an sich bekannten Verfahren herstellbar. Substituenten in substituiertem Niederalkyl R_{A}' und/oder R_{B}' können beispielsweise durch analoge Methoden eingeführt und/oder modifiziert werden, wie bei der Herstellung von Verbindungen der Formel IV mit substituiertem Niederalkyl A₁ und/oder A₂ für die Substitution oder Modifikation des Niederalkylrestes beschrieben. Anstelle von Q kann in den Ausgangsmaterialien auch eine geschützte Hydroxygruppe vorliegen, die erforderlichenfalls erst (nach Schutzgruppenabspaltung) in eine Abgangsgruppe überführt wird, beispielsweise mit Toluolsulfonylchlorid etc.

Alle übrigen Ausgangsmaterialien sind bekannt, kommerziell erhältlich oder nach an sich bekannten Verfahren herstellbar.

Alle genannten Ausgangsmaterialien (d.h. Ausgangsstoffe oder Zwischenverbindungen) können an fünktionellen Gruppen, die nicht an den beschriebenen Reaktionen teilnehmen sollen, erforderlichenfalls geschützt sein, wobei vorhandene Schutzgruppen zu einem geeigneten Zeitpunkt abgespalten werden. Erhaltene Verbindungen der Formel I mit Schutzgruppen können durch Schutzgruppenabspaltung in die freien Verbindungen der Formel I überführt werden, oder den unten genannten zusätzlichen Verfahrensmassnahmen unterworfen werden.

Zu den Schutzgruppen für funktionelle Gruppen in Ausgangsmaterialien, deren Umsetzung vermieden werden soll, insbesondere Carboxy-, Amino-, Hydroxy-, Mercapto- und Sulfogruppen, zählen insbesondere diejenigen Schutzgruppen (conventional protecting groups), die üblicherweise bei der Synthese von Peptid-Verbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Verlauf von Umsetzungen bewirken. Charakteristisch für Schutzgruppen ist, dass sie leicht, d. h. ohne unerwünschte Nebenreaktionen abspaltbar sind, beispielsweise solvolytisch, reduktiv, photolytisch oder auch enzymatisch, z. B. auch unter physiologischen Bedingungen. Charakteristisch für Schutzgruppen ist auch, dass sie in den Endstoffen nicht vorliegen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (E. Gross und J. Meienhofer, Herausg.), Academic Press, London und New York 1981, in "Methoden der organischen Chemie", Houben-Weyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z. B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, ist beispielsweise Methoxycarbonyl oder Ethoxycarbonyl.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z. B. tert-Butoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise Arylmethoxycarbonyl mit einem oder zwei Arylresten, worin Aryl unsubstituiertes oder z. B. durch Niederalkyl, z. B. tert-Niederalkyl, wie tert-Butyl, Niederalkoxy, z. B. Methoxy, Hydroxy, Halogen, z. B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z. B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl, ferner durch eine Niederalkylgruppe verestertes Carboxy, wobei die Niederalkylgruppe in 1- oder 2-Stellung durch geeignete Substituenten substituiert ist, wie 1-Niederalkoxyniederalkoxycarbonyl, z. B. Methoxymethoxycarbonyl, 1-Methoxyethoxycarbonyl oder 1-Ethoxyethoxycarbonyl, 1-Niederalkylthioniederalkoxycarbonyl, z. B. 1-Methylthiomethoxycarbonyl oder 1-Ethylthioethoxycarbonyl, Aroylmethoxycarbonyl, worin die Aroylgruppe gegebenenfalls beispielsweise durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-Iodethoxycarbonyl, sowie 2-(trisubstituiertes Silyl)-niederalkoxycarbonyl, worin die Substituenten unabhängig voneinander je einen gegebenenfalls, z. B. durch Niederalkyl, Niederalkoxy, Aryl, Halogen, und/oder Nitro substituierten aliphatischen, araliphatischen, cycloaliphatischen oder aromatischen Kohlenwasserstoffrest bedeuten, beispielsweise gegebenenfalls wie oben substituiertes Niederalkyl, Phenylniederalkyl, Cycloalkyl oder Phenyl, z. B. 2-Triniederalkylsilylniederalkoxycarbonyl, wie 2-Triniederalkylsilylethoxycarbonyl, z. B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe wird auch als organische Silyloxycarbonylgruppe geschützt. Eine organische Silyloxycarbonylgruppe ist beispielsweise eine Triniederalkylsilyloxycarbonylgruppe, z. B. Trimethylsilyloxycarbonyl. Das Siliciumatom der Silyloxycarbonylgruppe kann auch durch zwei Niederalkyl, z. B. Methylgruppen, und eine Amino- oder Carboxygruppe eines zweiten Moleküls der Formel I substituiert sein. Verbindungen mit solchen Schutzgruppen lassen sich z. B. mit entsprechenden Halogensilanen wie Dimethylchlorsilan als Silylierungsmittel herstellen.

Eine Carboxygruppe wird auch in Form eines inneren Esters mit einer in geeignetem Abstand, z. B. in γ-Stellung, zur Carboxygruppe im Molekül vorliegenden Hydroxygruppe, d. h. in Form eines Lactons, vorzugsweise eines γ-Lactons, geschützt.

Eine geschützte Carboxygruppe ist bevorzugt tert-Niederalkoxycarbonyl, z. B. tert-Butoxycarbonyl, Benzyloxycarbonyl, 4-Nitrobenzyloxycarbonyl, 9-Fluorenylmethoxycarbonyl oder Diphenylmethoxycarbonyl, oder eine in Form eines Lactons, insbesondere eines γ-Lactons, geschützte Carboxygruppe.

Die Freisetzung von geschütztem Carboxy erfolgt nach üblichen, z. B. den in den oben genannten Standardwerken über Schutzgruppen genannten Verfahren.

So kann geschütztes Carboxy, z. B. tert-Niederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure, Chlorwasserstoff oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z. B. durch Behandeln mit einem Alkalimetall-, wie Natrium-dithionit, oder mit einem reduzierenden Metall, z. B. Zink, oder einem reduzierenden Metallsalz, wie einem Chrom-II-salz, z. B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-(trisubstituiertes Silyl)-niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z. B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylniederalkylammoniumfluorid, z. B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z. B. Trimethylsilyloxycarbonyl, geschütztes Carboxy kann in üblicher Weise solvolytisch, z. B. durch Behandeln mit Wasser, einem Alkohol oder Säure, oder ausserdem Fluorid, wie oben beschrieben, freigesetzt werden. Verestertes Carboxy kann auch enzymatisch freigesetzt werden, beispielsweise durch Esterasen oder geeignete Peptidasen, z. B. verestertes Arginin oder Lysin, wie Lysinmethylester, mittels Trypsin. Als innerer Ester, wie als γ-Lacton, geschütztes Carboxy kann durch Hydrolyse in Gegenwart eines Hydroxid-haltigen Base, wie Erdalkalihydroxides oder insbesondere eines Alkalimetallhydroxides, z. B. NaOH, KOH oder LiOH, besonders LiOH, freigesetzt werden, wobei gleichzeitig die entsprechend geschützte Hydroxygruppe freigesetzt wird.

Eine geschützte Aminogruppe ist durch eine Aminoschutzgruppe geschützt, z. B. in Form einer Acylamino-, Arylmethylamino-, veretherten Mercaptoamino-, 2-Acyl-niederalk-1-enylamino oder Silylaminogruppe oder als Azidogruppe.

In einer Acylaminogruppe ist Acyl beispielsweise der Acylrest einer organischen Carbonsäure mit z. B. bis zu 18 Kohlenstoffatomen, insbesondere einer gegebenenfalls, z.B durch Halogen oder Aryl, substituierten Niederalkancarbonsäure oder gegebenenfalls, z. B. durch Halogen, Niederalkoxy oder Nitro, substituierten Benzoesäure, oder bevorzugt eines Kohlensäurehalbesters. Solche Acylgruppen sind vorzugsweise Niederalkanoyl, wie Formyl, Acetyl, Propionyl oder Pivaloyl, Halogenniederalkanoyl, z. B. 2-Halogenacetyl, wie 2-Chlor-, 2-Brom-, 2-Iod-, 2,2,2-Trifluor- oder 2,2,2-Trichloracetyl, gegebenenfalls z. B. durch Halogen, Niederalkoxy oder Nitro substituiertes Benzoyl, wie Benzoyl, 4-Chlorbenzoyl, 4-Methoxybenzoyl oder 4-Nitrobenzoyl, Niederalkoxycarbonyl, vorzugsweise in 1-Stellung des Niederalkylrestes verzweigtes oder in 1- oder 2-Stellung geeignet substituiertes Niederalkoxycarbonyl, z. B. tert-Niederalkoxycarbonyl, wie tert-Butoxycarbonyl, Arylmethoxycarbonyl mit einem, zwei oder drei Arylresten, die gegebenenfalls, z. B. durch Niederalkyl, insbesondere tert-Niederalkyl, wie tert-Butyl, Niederalkoxy, wie Methoxy, Hydroxy, Halogen, wie Chlor, und/oder Nitro mono- oder polysubstituiertes Phenyl darstellen, z. B. Benzyloxycarbonyl, 4-Nitrobenzyloxycarbonyl, Diphenylmethoxycarbonyl, 9-Fluorenylmethoxycarbonyl oder Di-(4-methoxyphenyl)methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Halogen-niederalkoxycarbonyl, z. B. 2,2,2-Trichlorethoxycarbonyl, 2-Bromethoxycarbonyl oder 2-Iodethoxycarbonyl, 2-(trisubstituiertes Silyl)-niederalkoxycarbonyl, z. B. 2-Triniederalkylsilyiniederalkoxycarbonyl, wie 2-Trimethylsilylethoxycarbonyl oder 2-(Di-n-butyl-methyl-silyl)-ethoxycarbonyl, oder Triarylsilylniederalkoxycarbonyl, z. B. 2-Triphenylsilylethoxycarbonyl.

In einer Arylmethylaminogruppe, z. B. einer Mono-, Di- oder insbesondere Triarylmethylaminogruppe, sind die Arylreste insbesondere gegebenenfalls substituierte Phenylreste. Solche Gruppen sind z. B. Benzyl-, Diphenylmethyl- oder insbesondere Tritylamino.

In einer veretherten Mercaptoaminogruppe liegt die Mercaptogruppe in erster Linie als substituiertes Arylthio oder Arylniederalkylthio, worin Aryl beispielsweise gegebenenfalls z.B. durch Niederalkyl, wie Methyl oder tert.-Butyl, Niederalkoxy, wie Methoxy, Halogen, wie Chlor, und/oder Nitro substituiertes Phenyl ist, z. B. 4-Nitrophenylthio, vor.

In einem als Aminoschutzgruppe verwendbaren 2-Acyl-niederalk-1-enylrest ist Acyl z. B. der entsprechende Rest einer Niederakancarbonsäure, einer gegebenenfalls z. B. durch Niederalkyl, wie Methyl oder tert-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-niederalk-1-en-2-yl, z. B. 1-Niederalkanoyl-prop-1-en-2-yl, wie 1-Acetyl-prop-1-en-2-yl, oder Niederalkoxycarbonyl-niederalk-1en-2-yl, z. B. Niederalkoxycarbonyl-prop-1-en-2-yl, wie 1-Ethoxycarbonyl-prop-1-en-2-yl.

Eine Silylaminogruppe ist beispielsweise eine Triniederalkylsilylaminogruppe, z. B. Trimethylsilylamino oder tert-Butyl-dimethylsilylamino. Das Siliciumatom der Silylaminogruppe kann auch nur durch zwei Niederalkylgruppen, z. B. Methylgruppen, und die Aminogruppe oder Carboxylgruppe eines zweiten Moleküls der Formel I substituiert sein. Verbindungen mit solchen Schutzgruppen lassen sich z. B. mit den entsprechenden Chlorsilanen, wie Dimethylchlorsilan, als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Niederalkoxycarbonyl, Phenylniederalkoxycarbonyl, Fluorenylniederalkoxycarbonyl, 2-Niederalkanoyl-niederalk-1-en-2-yl oder Niederalkoxycarbonyl-niederalk-1-en-2-yl, besonders bevorzugt tert-Butoxycarbonyl oder Benzyloxycarbonyl.

Bevorzugt sind auch bivalente Aminoschutzgruppen, wie mono- oder disubstituierte Methylidengruppen, wie 1-Niederalkoxy (beispielsweise Methoxy oder Ethoxy)-niederalkyliden (beispielsweise Ethyliden oder 1-n-Butyliden), z.B. =C(CH₃)(OC₂H₅), ferner z.B. =C(CH₃)₂ oder =CH-Phenyl, und insbesondere Bisacylreste, z.B. der Phthalylrest, welcher zusammen mit dem zu schützenden Stickstoffatom ein 1H-isoindol-1,3(2H)-dion (Phthalimidogruppe) bildet.

Eine geschützte Aminogruppe setzt man in an sich bekannter und je nach Art der Schutzgruppen in verschiedenartiger Weise, vorzugsweise mittels Solvolyse oder Reduktion, frei. Niederalkoxycarbonylamino, wie tert-Butoxycarbonylamino, kann in Gegenwart von Säuren, beispielsweise Mineralsäuren, z. B. Halogenwasserstoff, wie Chlorwasserstoff oder Bromwasserstoff, insbesondere Bromwasserstoff, oder von Schwefel- oder Phosphorsäure, vorzugsweise von Chlorwasserstoff, in polaren Lösungsmitteln, wie Wasser oder einer Carbonsäure, wie Essigsäure, oder Ethern, bevorzugt cyclischen Ethern, wie Dioxan, 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z. B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagenz, wie Natriumthiophenolat, und 4-Nitrobenzyloxycarbonylamino auch durch Behandeln mit einem Alkalimetall-, z. B. Natrium-dithionit, gespalten werden. Gegebenenfalls substituiertes Diphenylmethoxycarbonylamino, tert-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z. B. Ameisenoder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Palladiumkatalysators, bevorzugt in polaren Lösungsmitteln, wie Diniederalkylniederalkanoylamiden, z. B. Dimethylformamid, Ethern, wie cyclischen Ethern, z. B. Dioxan, oder Alkoholen, wie Methanol, Ethanol oder Propanol, wobei Methanol besonders bevorzugt ist, gegebenenfalls substituiertes Triarylmethylamino oder Formylamino z. B. durch Behandeln mit einer Säure, wie Mineralsäure, z.B. Chlorwasserstoffsäure, oder einer organischen Säure, z. B. Ameisen-, Essig- oder Trifluoressigsäure, gegebenenfalls in Gegenwart von Wasser, gespalten werden, und eine als Silylamino geschützten Aminogruppe kann z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionssproduktes freigesetzt werden. Eine durch 2-(trisubstituiertes Silyl)-niederalkoxycarbonyl, wie 2-Triniederalkylsilylniederalkoxycarbonyl, geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten. Die Abspaltung der Phthalylgruppe kann z.B. mittels Hydrazinhydrat geschehen oder mittels einer Säure, z.B. einer Mineralsäure, wie Chlorwasserstoffsäure, oder einer organischen Säure, wie Essigsäure, gegebenenfalls in Gegenwart von organischen Lösungsmitteln, z.B. Methanol oder Tetrahydrofuran.

In Form einer Azidogruppe geschütztes Amino wird z. B. durch Reduktion in freies Amino überführt, beispielsweise durch katalytische Hydrierung mit Wasserstoff in Gegenwart eines Hydrierkatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20 °C bis 25 °C, oder auch unter Kühlen oder Erwärmen, durchgeführt.

Eine Hydroxygruppe kann beispielsweise durch eine Acylgruppe, z. B. unsubstituiertes oder durch Halogen, wie Chlor, substituiertes Niederalkanoyl, wie Acetyl oder 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Triphenylmethoxycarbonyl. Eine Hydroxygruppe kann ferner durch Triniederalkylsilyl, z. B. Trimethylsilyl, Triisopropylsilyl oder tert-Butyl-dimethylsilyl, eine leicht abspaltbare verethernde Gruppe, z. B. eine Alkylgruppe, wie tert-Niederalkyl, z. B. tert-Butyl, einen oxa- oder einen thiaaliphatischen oder -cycloaliphatischen, insbesondere 2-oxa- oder 2-thiaaliphatischen oder -cycloaliphatischen, Kohlenwasserstoffrest, beispielsweise 1-Niederalkoxyniederalkyl oder 1-Niederalkylthioniederalkyl, wie Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, wie 2-Tetrahydrofuryl oder 2-Tetrahydropyranyl, oder ein entsprechendes Thiaanaloges, sowie durch 1-Phenylniederalkyl, wie Benzyl, Diphenylmethyl oder Trityl, wobei die Phenylreste beispielsweise durch Halogen, z. B. Chlor, Niederalkoxy, z. B. Methoxy, und/oder Nitro substituiert sein können, geschützt sein.

Zwei in einem Molekül vorkommende, insbesondere benachbarte Hydroxylgruppen oder eine benachbarte Hydroxy- und Aminogruppe können beispielsweise durch bivalente Schutzgruppen, wie eine vorzugsweise, etwa durch ein oder zwei Niederalkylreste oder Oxo, substituierte Methylengruppe, geschützt sein, z. B. durch unsubstituiertes oder substituiertes Alkyliden, z. B. Niederalkyliden, wie Isopropyliden, Cycloalkyliden, wie Cyclohexyliden, eine Carbonylgruppe oder Benzyliden.

Eine in Nachbarstellung zu einer Carboxygruppe stehende Hydroxygruppe kann durch Bildung eines inneren Esters (Lacton), insbesondere eines γ-Lactones, geschützt sein.

Bevorzugt ist eine geschützte Hydroxygruppe durch Triniederalkylsilyl oder als Lacton geschützt, insbesondere durch tert-Butyl-dimethylsilyl.

Eine Mercaptogruppe kann insbesondere durch S-Alkylierung mit gegebenenfalls substituierten Alkylresten, Silylierung, Thioacetalbildung, S-Acylierung oder durch die Bildung asymmetrischer Disulfidgruppierungen geschützt sein. Bevorzugte Mercaptoschutzgruppen sind beispielsweise gegebenenfalls im Phenylrest, z. B. durch Methoxy oder Nitro, substituiertes Benzyl, wie 4-Methoxybenzyl, gegebenenfalls im Phenylrest, z. B. durch Methoxy, substituiertes Diphenylmethyl, wie Di-(4-methoxyphenyl)-methyl, Triphenylmethyl, Pyridyldiphenylmethyl, Trimethylsilyl, Benzylthiomethyl, Tetrahydropyranyl, Acylaminomethyl, wie Acetamidomethyl, iso-Butyrylacetamidomethyl oder 2-Chloracetamidomethyl, Benzoyl, Benzyloxycarbonyl oder Alkyl-, insbesondere Niederalkylaminocarbonyl, wie Ethylaminocarbonyl, sowie Niederalkylthio, wie S-Ethylthio oder S-tert-Butylthio, oder S-Sulfo.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert-Niederalkyl oder durch einen 2-oxa- oder 2-thia-aliphatischen oder -cycloaliphatischen Kohlenwasserstoffrest geschützte Hydroxy- bzw. Mercaptogruppe durch Acidolyse, z. B. durch Behandeln mit einer Mineralsäure oder einer starken Carbonsäure, z. B. Trifluoressigsäure, freigesetzt. Durch Pyridyldiphenylmethyl geschütztes Mercapto kann z. B. durch Quecksilber-II-salze bei pH 2-6 oder durch Zink/Essigsäure oder elektrolytische Reduktion, Acetamidomethyl und iso-Butyrylamidomethyl z. B. durch Reaktion mit Quecksilber-II-salzen bei pH 2-6, 2-Chloracetamidomethyl z. B. durch 1-Piperidinothiocarboxamid, S-Ethylthio, S-tert-Butylthio und S-Sulfo z. B. durch Thiolyse mit Thiophenol, Thioglycolsäure, Natrium-thiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z. B. einer durch Niederalkyl ein- oder zweifach substituierten Methylengruppe, wie durch Niederalkyliden, z.B. Isopropyliden, Cycloyalkyliden, z. B. Cyclohexyliden, oder Benzyliden, geschützt sind, können durch saure Solvolyse, besonders in Gegenwart einer Mineralsäure oder einer starken organischen Säure, freigesetzt werden. Eine Triniederalkylsilylgruppe wird ebenfalls durch Acidolyse, z. B. durch Mineralsäure, bevorzugt Fluorwasserstoffsäure, oder eine starke Carbonsäure abgespalten. 2-Halogenniederalkoxycarbonyl wird durch die oben genannten Reduktionsmittel, z. B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt. Veresterte Hydroxygruppen, z. B. Niederalkanoyloxy, wie Acetyloxy, können auch durch Esterasen freigesetzt werden, acyliertes Amino beispielsweise durch geeignete Peptidasen.

Eine Sulfogruppe kann beispielsweise durch Niederalkyl, z. B. Methyl oder Ethyl, durch Phenyl oder als Sulfonamid, beispielsweise als Imidazolid, geschützt sein.

Eine als Sulfonsäureester oder Sulfonamid geschützte Sulfogruppe wird beispielsweise durch saure Hydrolyse, z. B. in Gegenwart von Mineralsäure, oder bevorzugt durch basische Hydrolyse, z. B. mit Alkalimetallhydroxid oder Alkalimetallcarbonat, beispielsweise Natriumcarbonat, freigesetzt.

Oxo wird erforderlichenfalls geschützt durch Acetalbildung, z. B. mit Niederalkanolen, insbesondere mit Ethan-1,2-diol, wobei die Schutzgruppe auf der gewünschten Stufe durch Hydrolyse in Gegenwart einer Säure, wie Essigsäure oder Schwefelsäure, abgespalten werden kann, Thioxoniederalkyl wird erforderlichenfalls geschützt durch Thioacetalbildung, z. B. mit Niederalkylmercaptanen, wie Ethan-1,2-dithiol, wobei die Schutzgruppe zum geeigneten Zeitpunkt durch Hydrolyse in Gegenwart einer Säure, wie Essigsäure oder Schwefelsäure, abgespalten werden kann.

Die Temperaturen für die Freisetzung der geschützten funktionellen Gruppen liegen vorzugsweise zwischen -80 und 100 °C, besonders bevorzugt zwischen -20 und 50 °C, beispielsweise zwischen 10 und 35 °C, wie im Bereich der Raumtemperatur oder bei Rückflusstemperatur.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Trifluoressigsäure, oder mit Wasserstoff und einem Hydrierkatalysator, wie einem Palladium-Kohle-Katalysator. Umgekehrt können die Gruppen auch so gewählt werden, dass sie nicht alle gleichzeitig, sondern in gewünschter Reihenfolge abgespalten werden können, wobei die entsprechenden Zwischenprodukte erhalten werden.

### Verfähren b) (Cyclisierung von 2-Formylbenzoesäurederivaten mit Hydrazinen)

Formylbenzoesäurederivate der Formel XIII liegen frei oder insbesondere als reaktionsfähige Derivate vor, wobei entweder die Carboxygruppe oder die Formylgruppe oder beide derivatisiert sein können. Die Formyl- und die Carboxygruppe können simultan oder sukzessive, evtl. auch in verschiedenen Ansätzen, mit der Verbindung der Formel XIV umgesetzt werden.

Die Carboxygruppe kann beispielsweise als aktivierte Säuregruppe vorliegen, z.B. in Form eines Anhydrids oder eines Derivates, worin anstelle der Carboxygruppe ein Rest Z₁-(C=O)- vorliegt, worin Z₁ reaktionsfähig aktiviertes Hydroxy bedeutet, wie es oben für Verbindungen der Formel IIa definiert ist.

In einer reaktionsfähigen Verbindung der Formel XIII kann die Formylgruppe in reaktionsfähig derivatisierter Form vorliegen, beispielsweise in Acetalform, insbesondere als Diniederalkoxymethyl, wie Dimethoxymethyl oder Diethoxyethyl, als Bis(arylniederalkoxy)methyl beispielsweise Dibenzyloxymethyl, oder als Niederalkylendioxymethyl, wie Ethylen-1,2-dioxymethyl.

Die Reaktionsbedingungen entsprechen dabei den unten genannten allgemeinen Reaktionsbedingungen.

Vorzugsweise wird ein reaktionsfähiges Derivat einer Verbindung der Formel XIII eingesetzt, welches die Formel XIIIa 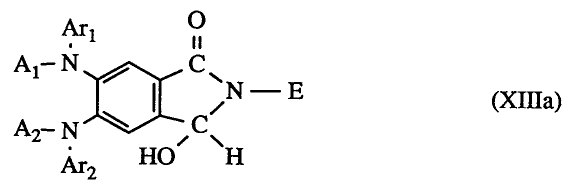
hat, worin E Wasserstoff oder Niederalkyl bedeutet und die übrigen Reste die genannten Bedeutungen haben. Die Reaktion einer Verbindung der Formel XIIIa findet vorzugsweise in einem inerten Lösungsmittel statt, z.B. in einem Ether, insbesondere einem Diniederalkyether, wie Diethylether, oder in erster Linie in einem cyclischen Ether, wie Tetrahydrofuran oder vor allem Dioxan, vorzugsweise unter erhöhtem Druck in einem Druckrohr, bei bevorzugten Temperaturen zwischen 50 und 150 °C, vorzugsweise bei etwa 120 °C. Vorzugsweise liegt die Hydrazinverbindung der Formel XIV im Überschuss gegenüber der Verbindung der Formel XIII vor, beispielsweise im 1,1- bis 10-fachen molaren Überschuss.

In den Ausgangsmaterialien der Formel XIII und deren reaktionsfähigen Derivaten und in den Ausgangsmaterialien der Formel XIV liegen funktionelle Gruppen, die nicht an der Reaktion teilnehmen sollen, erforderlichenfalls in geschützter Form vor. Die Schutzgruppen und ihre Einführung sind wie unter Verfahren a) beschrieben. Die Abspaltung von Schutzgruppen erfolgt ebenfalls vorzugsweise nach den unter Verfahren a) beschriebenen Methoden.

Die Ausgangsmaterialien der Formel XIII sind nach an sich bekannten Verfahren herstellbar. Beispielsweise können sie aus einer Verbindung der Formel IV, die wie oben beschrieben hergestellt werden kann und worin die Reste die oben genannten Bedeutungen haben, durch Umsetzung mit Ammoniak oder einem Niederalkylamin und anschliessende Reduktion der erhaltenen Verbindungen hergestellt werden, wobei man Verbindungen der Formel XIIIa erhält.

Die Umsetzung mit Ammoniak oder einem Niederalkylamin (Aminolyse) findet bei Temperaturen zwischen 10 und 150 °C, vorzugsweise bei 100 bis 150 °C, statt. Bevorzugt ist die Reaktion von Diniederalkylestern der Formel IV, insbesondere in einem hochsiedenden Alkohol, z.B. einem Diol, wie Ethylenglykol; oder in einem anderen Lösungsmittel, z.B. einem Niederalkanol, wie Methanol oder Ethanol, oder in Abwesenheit von Lösungsmitteln, in einem Autoklaven bei erhöhtem Druck.

Man erhält so Phthalimidderivate der Formel XV, 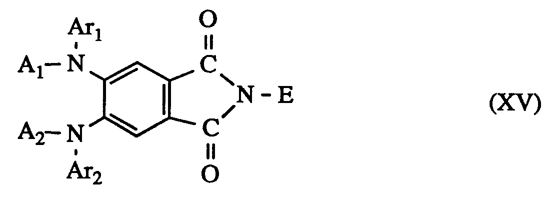
worin A₁, A₂, Ar₁, Ar₂ und E die genannten Bedeutungen haben. Die Reduktion dieser Verbindungen findet beispielsweise mit geeigneten komplexen Hydriden, insbesondere mit Lithiumaluminiumhydrid, in geeigneten Lösungsmitteln, vorzugsweise in Ethern, wie Diniederalkylethern oder cyclischen Ethern, wie Dioxan oder insbesondere Tetrahydrofuran, bei bevorzugten Temperaturen zwischen 0 und 50 °C, insbesondere etwa bei Raumtemperatur, statt, wobei das komplexe Hydrid vorzugsweise im Überschuss, beispielsweise im 1,1- bis 10-fachen molaren Überschuss gegenüber der Verbindung der Formel XV, eingesetzt wird. Man erhält so die cyclischen 2-Formylbenzoesäurederivate der Formel XIIIa.

Sofern nicht zu unerwünschten Produkten füllende hydrolyseempfindliche weitere Substituenten in Verbindungen der Formel XIIIa vorliegen, kann man freie Verbindungen der Formel XIII dann unter geeigneten Bedingungen durch Abspaltung des Restes -NH-E (Freisetzung der Carboxygruppe) unter an sich bekannten Bedingungen zur Hydrolyse, z.B. in Gegenwart wässriger Laugen, z.B. in Gegenwart von Erdalkalimetallhydroxiden, wie Natriumhydroxid oder Kaliumhydroxid; oder in Gegenwart von Säuren, wie Halogenwasserstoffsäuren, z.B. Chlorwasserstoffsäure, Schwefelsäure oder Phosphorsäure, insbesondere von schwachen Säuren wie Essigsäure oder Ameisensäure; in An- oder Abwesenheit weiterer Lösungsmittel, wie Tetrahydrofuran oder Dimethylformamid, bei Temperaturen zwischen 0 °C und Rückflusstemperatur, z.B. zwischen etwa 20 und 50 °C, aus Verbindungen der Formel XIIIa gewinnen.

Die freien Verbindungen können dann nach an sich bekannten Verfahren in weitere reaktionsfähige Derivate überführt werden. Beispielsweise kann man den Formylrest mit Niederalkylalkoholen, wie Methanol oder Ethanol, mit Arylniederalkylalkoholen, wie Benzylalkohol, oder mit Diolen, wie Ethan-1,2-diol (Ethylenglykol) in Acetalform überführen (vorzugsweise unter Säurekatalyse, z.B. in Gegenwart von Toluolsulfonsäure, Schwefelsäure, Phosphorsäure, Halogenwasserstoffsäuren, wie Chlorwasserstoffsäure, oder Halogenessigsäure, wie Trifluor- oder Trichloressigsäure; in geeigneten Lösungsmitteln, z.B. aromatischen Lösungsmitteln, wie Benzol, Toluol oder Xylol, oder in halogenierten Kohlenwasserstoffen, wie Chloroform, Trichlorethylen oder Dichlormethan, bei erhöhten Temperaturen, z.B. unter Rückfluss), und/oder die Carboxygruppe in aktivierte Form bringen, beispielsweise, wie unter Verfahren a) für reaktionsfähige Ausgangsmaterialien der Formel IIa beschrieben.

Die geeignete Reihenfolge und geeignete Reaktionsbedingungen sind dabei dem Fachmann geläufig; erforderlichenfalls sind funktionelle Gruppen zu schützen, die nicht an den Reaktionen teilnehmen sollen.

Die Ausgangsmaterialien der Formel XIV sind bekannt oder analog den Ausgangsverbindungen der Formel III herstellbar.

### Zusätzliche Verfahrensmassnahmen:

Verbindungen der Formel I können in an sich bekannter Weise in andere Verbindungen der Formel I umgewandelt werden.

Es ist möglich, einzelne der genannten Umwandlungen durchzuführen, oder aber auch geeignete Kombinationen zu wählen, also zwei oder mehr Umwandlungen mit einer Verbindung der Formel I durchzuführen. Funktionelle Gruppen in Ausgangsmaterialien der Formel I und anderen Ausgangsmaterialien, die nicht an der jeweiligen Umsetzung teilnehmen sollen, liegen erforderlichenfalls in geschützter Form vor. Die Schutzgruppen werden zu geeigneten Zeitpunkten abgespalten. Die Einführung der Schutzgruppen, die Schutzgruppen selbst und ihre Abspaltung sind wie oben beschrieben.

Zum Beispiel kann eine Verbindung der Formel I, worin in Acylsubstituenten Carbonylgruppen vorliegen, mit einem geeigneten Reagens umgesetzt werden, so dass eine andere Verbindung der Formel I, worin anstelle der entsprechenden Carbonyl- eine Thiocarbonylgruppe steht, erhalten wird. Ein geeignetes Reagens für die Ueberführung von -C(=O)- in -C(=S)- sind z.B. Phosphorpentasulfid oder vorzugsweise Phosphorpentasulfid-Ersatzstoffe, z.B. das Lawesson-Reagens (= 2,4-Bis-(4-methoxyphenyl)-2,4-dithioxo-1,3,2,4-dithiaphosphetan), wobei die Reaktion beispielsweise in einem halogenierten Kohlenwasserstoff, wie Dichlormethan, bei Temperaturen von 30 °C bis zur Rückflusstemperatur, insbesondere bei Rückflusstemperatur, durchgeführt wird.

Verbindungen der Formel I, worin einer der Reste A₁ und A₂ Wasserstoff bedeutet, können durch Umsetzung mit geeigneten Reagentien in andere Verbindungen der Formel I, worin keiner der Reste A₁ und A₂ mehr Wasserstoff bedeutet, überführt werden.

Zur Einführung von A₁ oder A₂ = unsubstituiertes oder substituiertes Niederalkyl kommt z.B. die Behandlung mit der Base Lithiumdiisopropylamid (LDA) und anschliessende Umsetzung mit einem unsubstituierten oder substituierten Diniederalkylether oder einem unsubstituierten oder substituierten Niederalkylhalogenid in Frage. Substituiertes Niederalkyl ist dabei wie für den entsprechenden Rest A₁ und/oder A₂ in Verbindungen der Formel I definiert.

Verbindungen der Formel I, worin wenigstens einer der Reste Ar₁ und Ar₂ Phenyl bedeutet, können durch Hydrierung in Verbindungen der Formel I überführt werden, worin anstelle des/der Phenylrestes ein Cyclohexylrest steht. Die Hydrierung erfolgt vorzugsweise in Gegenwart eines Katalysators, der die selektive Hydrierung von aromatischen Doppelbindungen, beispielsweise in Gegenwart nicht umzusetzender Amidgruppen, erlaubt, insbesondere eines Katalysators aus Schwermetalloxiden, wie eines Rh(III)/Pt(VI)-Oxidkatalysators nach Nishimura (S. Nishunura, Bull. Chem. Soc. Japan 33, 566(1960), in geeigneten Lösungsmitteln, insbesondere Wasser, Alkoholen, wie Methanol oder Ethanol, Estern, wie Essigsäureethylester, oder Ethern, wie Dioxan, oder vorzugsweise in Gegenwart von PtO₂ in Niederalkancarbonsäuren, wie Essigsäure, bei Temperaturen zwischen 0 °C und Rückflusstemperatur oder bis zu 150 °C, vorzugsweise bei 10 bis 50 °C, z.B. bei Raumtemperatur, und bei Wasserstoffdrucken von 1 bis 50 bar, z.B. etwa bei Normaldruck.

Verbindungen der Formel I, worin Ar₁ und/oder Ar₂ durch Halogen, vorzugsweise Brom, substituiertes Aryl, insbesondere Phenyl oder Naphthyl, bedeutet, können in die entsprechenden Derivate umgewandelt werden, in denen eines oder alle der in Aryl Ar₁ und/oder Ar₂ vorhandenen Halogenatome durch Cyano ersetzt sind, beispielsweise durch Umsetzung mit einem Cyanidsalz eines Übergangsmetalles, insbesondere CuCN, bei Temperaturen zwischen 50 und 150 °C, vorzugsweise zwischen 60 und 140 °C, in einem inerten polaren Lösungsmittel, wie einem N,N-Diniederalkyl-niederalkancarbonsäureamid, z. B. Dimethylformamid, ohne oder mit anschliessender Zugabe eines Katalysators, z. B. eines Übergangsmetallhalogenides, wie Eisen(III)chlorid, in wässriger Lösung, (siehe auch Rosenmund et al., Ber. **52**, 1749 (1916); von Braun et al., Ann. 488, 111 (1931).

In Verbindungen der Formel I können die Reste Ar₁ und/oder Ar₂, welche unsubstituiertes oder substituiertes Aryl bedeuten, vorzugsweise unsubstituiertes Phenyl oder Naphthyl, unabhängig voneinander unter Einführung einer oder mehrerer Nitrogruppen nitriert werden, beispielsweise unter üblichen Bedingungen zur Einführung einer Nitrogruppe in Aromaten, z. B. mit konzentrierter oder 100%-iger Salpetersäure bei Temperaturen zwischen 0 und 100 °C, vorzugsweise zwischen 10 und 40 °C, in einem inerten Lösungsmittel, z. B. einem organischen Säureanhydrid, wie Acetanhydrid. Entstehen dabei mehrere verschiedene Produkte mit unterschiedlicher Position und Anzahl der Nitrogruppen, so können diese nach üblichen Methoden, beispielsweise säulenchromatographisch, getrennt werden.

Nitrosubstituenten innerhalb der Reste Ar₁ und/oder Ar₂ können zu Aminogruppen reduziert werden, beispielsweise durch Hydrierung unter üblichen Bedingungen, z. B. durch Hydrierung in Gegenwart eines zur selektiven Reduktion der Nitrogruppen geeigneten Hydrierkatalysators, wie Raney-Nickel, in einem inerten Lösungsmittel, z. B. einem cyclischen oder acyclischen Ether, wie Tetrahydrofuran, unter Normaldruck oder bei auf bis zu 5 bar erhöhtem Druck.

Verbindungen der Formel I mit veretherten Hydroxygruppen, beispielsweise mit Niederalkoxyresten als Substituenten innerhalb von Ar₁ und/oder Ar₂ oder an A₁ und/oder A₂, können durch Etherspaltung in die entsprechenden Hydroxy-substituierten Verbindungen der Formel I überführt werden. Die Etherspaltung erfolgt unter an sich bekannten Bedingungen, beispielsweise in Gegenwart von Halogenwasserstoffsäuren, wie Bromwasserstoff oder Iodwasserstoff, in An- oder Abwesenheit von Lösungsmitteln, wie Carbonsäuren, z. B. Niederalkancarbonsäuren, wie Essigsäure, bei Temperaturen zwischen 20 °C und der Rückflusstemperatur des Reaktionsgemisches, oder vorzugsweise unter schonenden Bedingungen mit Borhalogeniden, insbesondere Bortribromid, in einem inerten Lösungsmittel, wie einem chlorierten Kohlenwasserstoff, z. B. Methylenchlorid oder Chloroform, bei Temperaturen zwischen -80 und 0 °C, insbesondere zwischen -50 und -20 °C.

Verbindungen der Formel I, in denen Hydroxy vorliegt, z.B. in A₁, A₂ und/oder aus diesen beiden Resten zusammen gebildetem substituiertem Niederalkylen, können durch Oxidation zu den entsprechenden Carbonylverbindungen und direkt anschliessende oder erst nach der Isolierung der Carbonylverbindung erfolgende Umsetzung mit Hydroxylamin oder einem Salz davon oder weiteren Aminoverbindungen in die entsprechenden Iminoverbindungen, wie Hydroxyiminoverbindungen, überführt werden. Die weiteren Substituenten, Reagentien und bevorzugten Reaktionsbedingungen finden sich bei der Beschreibung der Herstellung von Verbindungen der Formel IV worin als Substituent in aus A₁ und A₂ zusammen gebildetem substituiertem Niederalkylen Imino, Niederalkylimino, Acylimino, Hydroxyimino, Niederalkoxyimino, Hydrazono, N-Mono- oder N,N-Diniederalkylhydrazono und/oder N-Acylhydrazono vorliegt, aus entsprechenden Oxo-Verbindungen der Formel IV, wobei an Stelle der Oxoverbindungen der Formel IV solche der Formel I einzusetzen sind.

Verbindungen der Formel I, in denen Hydroxyimino als Substituent in A₁ und/oder A₂ oder einem aus diesen beiden Resten zusammen gebildetem Niederalkylen vorliegt, können unter Hydrierung zu den entsprechenden Aminoverbindungen umgesetzt werden. Die Hydrierung erfolgt dabei vorzugsweise katalytisch mit selektiven Hydrierkatalysatoren, insbesondere in Gegenwart von Palladium auf festen Trägermaterialien, z.B. auf Kohle, in polaren organischen oder organisch-wässrigen Lösungsmitteln oder Lösungsmittelgemischen, insbesondere in Ethern, z.B. cyclischen Ethern, wie Tetrahydrofuran oder Dioxan, oder in Alkoholen, z.B. Niederalkanolen, wie Methanol oder Ethanol, oder Gemischen davon, z.B. in Methanol/Tetrahydrofuran-Gemischen, bei Temperaturen zwischen -20 und 60 °C, vorzugsweise zwischen 0 und 40 °C, z.B. etwa bei Raumtemperatur.

In Verbindungen der Formel I mit primären Hydroxygruppen, beispielsweise solchen Verbindungen der Formel I, in denen substituiertes Niederalkyl, substituiertes Niederalkenyl oder substituiertes Niederalkinyl A₁ und/oder A₂ primäre Hydroxygruppen enthalten, wie in Hydroxyethyl, oder in denen ein aus A₁ und A₂ zusammen gebildetes substituiertes Niederalkylen vorliegt, welches als Substituent Hydroxyniederalkyl, wie Hydroxymethyl, enthält, können primäre Hydroxygruppen zu entsprechenden Carboxy- oder Carboxyniederalkylresten oxidiert werden, beispielsweise durch Oxidation mit Chromsäure, Dichromat/Schwefelsäure, Salpetersäure, Braunstein oder Kaliumpermanganat, vorzugsweise mit Kaliumpermanganat in neutralem oder alkalischem Medium, z.B. in wässriger alkoholischer Lösung, bei bevorzugten Temperaturen zwischen -20 und 50 °C, insbesondere zwischen 0 °C und Raumtemperatur. Man erhält entsprechende Carboxy-substituierte Verbindungen der Formel I.

In Verbindungen der Formel I, worin ein substituiertes Niederalkyl A₁ und/oder A₂ Carboxy enthält, wie in Carboxymethyl A₁ und/oder A₂, oder worin ein aus A₁ und A₂ zusammen gebildetes substituiertes Niederalkylen vorliegt, welches als Substituent Carboxy- oder Carboxyniederalkyl enthält, beispielsweise in solchen Verbindungen, die nach dem zuletzt beschriebenen Verfahren aus Verbindungen der Formel I mit primären Hydroxygruppen hergestellt sind, können Carboxygruppen durch Reaktion mit Diazoniederalkanen, wie Diazomethan (ergibt Methyloxycarbonyl) oder mit Niederalkanolen, wie Methanol oder Ethanol, in die entsprechenden Niederalkoxycarbonylgruppen überführt werden. Die Reaktion mit Diazomethan erfolgt beispielsweise in wässrig-alkoholischer Lösung, z.B. in Wasser/Methanol, oder vorzugsweise in Ether, in Gegenwart einer etherischen Lösung von Diazomethan, z.B. Diazomethan in Diethylether, bei Temperaturen zwischen -20 und 30 °C, z.B. zwischen 0 °C und Raumtemperatur. Die Reaktion mit Niederalkanolen erfolgt vorzugsweise in Gegenwart von Kondensationsmitteln, wie Carbodiimiden, z.B. Dicyclohexylcarbodiimid, in dem betreffenden Niederalkanol, dem ein weiteres inertes organisches Lösungsmittel, vorzugsweise Dimethylformamid oder Dimethylacetamid, zugesetzt sein kann, bei Temperaturen zwischen 0 ° und der Rückflusstemperatur, vorzugsweise zwischen 10 und 40 °C. Man erhält entsprechende Niederalkoxycarbonylverbindungen der Formel I.

Verbindungen der Formel I, worin als Substituent in einem aus A₁ und A₂ zusammen gebildeten substituierten Niederalkylen Hydroxy und/oder Hydroxyniederalkyl vorliegt, können zu einer entsprechenden Oxo-Verbindung oxidiert werden. Im Falle primärer Alkohole ist hierzu die Verwendung von selektiven Oxidationsmitteln erforderlich, z. B. von Kaliumferrat (K₂FeO₄) in wässrigen sowie Braunstein in organischen Lösungsmitteln, tert-Butylchromat, Pyridiniumdichromat oder insbesondere Pyridiniumchlorochromat in inerten organischen Lösungsmitteln, z.B. chlorierten Kohlenwasserstoffen, wie Dichlormethan oder Chloroform. Die Reaktion findet vorzugsweise bei Temperaturen zwischen -20 °C und der Rückflusstemperatur, z.B. bei etwa 0 bis 40 °C, statt. Im Falle von sekundären Alkoholen kann ausserdem mit weniger selektiven Oxidationsmitteln, wie Chromsäure, Dichromat/Schwefelsäure, Dichromat/Eisessig, Salpetersäure, Braunstein, Selendioxid oder Dimethylsulfoxid in Gegenwart von Oxalylchlorid, in Wasser, wässrigen oder organischen Lösungsmitteln, wie halogenierten Kohlenwasserstoffen, z.B. Methylenchlorid, oder Carbonsäureamiden, wie Dimethylformamid, oxidiert werden, vorzugsweise bei Temperaturen zwischen - 50 °C und Rückflusstemperatur, insbesondere zwischen - 10 und 50 °C.

Man erhält Verbindungen der Formel I, in denen aus A₁ und A₂ zusammen gebildetes substituiertes Niederalkylen und/oder substituiertes Methyl Z durch Oxo substituiert ist.

Verbindungen der Formel I, worin in einem aus A₁ und A₂ zusammen gebildeten substituierten Niederalkylen ein oder mehrere Substituenten ausgewählt aus Imino, Niederalkylimino, Acylimino, Hydroxyimino, Niederalkoxyimino, Hydrazono, N-Mono- oder N,N-Diniederalkylhydrazono, N-Acylhydrazono und durch Imino, Niederalkylimino, Acylimino, Hydroxyimino, Niederalkoxyimino, Hydrazono, N-Mono- oder N,N-Diniederalkylhydrazono und/oder N-Acylhydrazono substituiertem Niederalkyl vorliegt, und/oder Verbindungen der Formel I, worin Z durch Imino, Niederalkylimino, Acylimino, Hydroxyimino, Niederalkoxyimino, Hydrazono, N-Mono- oder N,N-Diniederalkylhydrazono oder N-Acylhydrazono substituiertes Methyl bedeutet, können aus entsprechenden Oxo-Verbindungen der Formel I hergestellt werden, entweder nach Isolierung der Oxoverbindungen oder vorzugsweise durch deren direkte Weiterverwendung als Rohprodukt, beispielsweise nach partiellem Eindampfen zur Entfernung des Lösungsmittels, in dem die Oxidation einer Hydroxy- zur Oxoverbindung durchgeführt wird, welche beispielsweise wie zuletzt beschrieben erfolgt.

So können die Oxoverbindungen durch Umsetzung mit Stickstoffbasen ausgewählt aus Ammoniak, Niederalkylaminen, Hydroxylamin, Niederalkoxyamin, Hydrazin, N-Mono- oder N,N-Diniederalkylhydrazin und N-Acylhydrazin in die entsprechenden Iminoderivate überführt werden. Die Reaktionsbedingungen entsprechen dabei den zur Umsetzung von Carbonylverbindungen mit Stickstoffbasen üblichen Bedingungen, wobei beispielsweise die Stickstoffbase als Salz einer Säure, z. B. von einer Halogenwasserstoffsäure, wie Fluorwasserstoff, Chlorwasserstoff, Bromwasserstoff oder Jodwasserstoff, besonders bevorzugt Chlorwasserstoff, von Schwefelsäure oder einem Hydrogensulfat, wie einem Alkalimetallhydrogensulfat, z. B. Natriumhydrogensulfat, von Phosphorsäure, einem Hydrogenphosphat oder einem Dihydrogenphosphat, z. B. einem Alkalimetallhydrogenphosphat oder -dihydrogenphosphat, wie Natriumhydrogenphosphat, Dinatriumhydrogenphosphat, Kaliumhydrogenphosphat oder Dikaliumhydrogenphosphat, oder als Salz mit einer organischen Säure, insbesondere mit einer Carbonsäure, wie einer im Niederalkylrest unsubstituierten oder, vorzugsweise durch Halogen, wie Fluor oder Jod, substituierten Niederalkancarbonsäure, z. B. Essigsäure, Chloressigsäure, Dichloressigsäure oder Trifluor- oder Trichloressigsäure, oder mit einer Sulfonsäure, wie einer Niederalkylsulfonsäure, z. B. Methansulfonsäure, Ethansulfonsäure oder Ethandisulfonsäure, oder einer Arylsulfonsäure, wie Benzol- oder Naphthalinsulfonsäure oder Naphthalin-1,5-disulfonsäure, verwendet wird; wobei auch ein Salz einer der genannten Stickstoffbasen mit einer Säure erst in situ, vor allem aus dem entsprechenden Salz einer leicht flüchtigen, durch eine starke Säure, wie Schwefelsäure oder in erster Linie eine der genannten Halogenwasserstoffsäuren, austreibbaren schwachen Säure, wie einer Niederalkancarbonsäure, z. B. Essigsäure, oder insbesondere Kohlensäure oder Hydrogencarbonat, durch Austreiben der schwachen Säure hergestellt werden kann; in Wasser (in An- oder Abwesenheit von Tensiden), einem wässrigen Lösungsmittelgemisch, wie einem Gemisch von Wasser mit einem oder mehreren Alkoholen, z.B. Methanol, Ethanol oder Isopropanol, Diniederalansulfinen, wie Dimethylsulfoxid, oder Diniederalkylniederalkanoylamiden, wie Dimethylformamid, organischen Lösungsmitteln, wie Alkoholen, z.B. Methanol oder Ethanol, Diniederalkylsulfoxiden, wie Dimethylsulfoxid, Diniederalkylniederalkanoylamiden, wie Dimethylformamid, oder in ausreichend inerten Nitrilen, wie Acetonitril, einem Gemisch solcher organischer Lösungsmittel, oder ohne Lösungsmittel in einer Schmelze, vorzugsweise in einer alkoholischen Lösung, wie in Methanol, Ethanol oder insbesondere Isopropanol; vorzugsweise bei Temperaturen zwischen - 20 °C und der Rückflusstemperatur des Reaktionsgemisches in Gegenwart eines Lösungsmittels, bei Schmelzen bis zu 220 °C, insbesondere bei Temperaturen von 0 bis 50 °C in Gegenwart eines Lösungsmittels, vor allem etwa bei Raumtemperatur; umgesetzt wird.

Verfahrensgemäss erhältliche freie Verbindungen der Formel I mit salzbildenden Eigenschaften können in an sich bekannter Weise in ihre Salze überführt werden, Verbindungen mit basischen Eigenschaften z.B. durch Behandeln mit Säuren oder geeigneten Derivaten davon, Verbindungen mit sauren Eigenschaften z.B. durch Behandeln mit Basen oder geeigneten Derivaten davon.

Erfindungsgemäss erhältliche Gemische von Isomeren können in an sich bekannter Weise in die einzelnen Isomeren aufgetrennt werden, Diastereomere z.B. durch Verteilung zwischen mehrphasigen Lösungsmittelgemischen, Umkristallisieren und/oder chromatographische Trennung, beispielsweise an Kieselgel, Racemate z.B. durch Bilden von Salzen mit optisch reinen salzbildenden Reagentien und Auftrennen des so erhältlichen Diastereomerengemisches, z.B. mittels fraktionierter Kristallisation, oder durch Chromatographie an optisch aktiven Säulenmaterialien.

### Allgemeine Reaktionsbedingungen:

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab- oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -80°C bis etwa 250°C, vorzugsweise von etwa -20°C bis etwa 150°C, z.B. bei Raumtemperatur bis zu der Rückflusstemperatur, bei Schmelzen bei bis zu 220 °C, unter atmosphärischem Druck oder in einem geschlossenen Gefäss, gegebenenfalls unter Druck, z.B. bei dem Druck, der im Reaktionsgemisch unter den Reaktionsbedingungen in einem geschlossenen Rohr entsteht, und/oder in einer inerten Atmosphäre, z.B. unter einer Argon- oder Stickstoffatmosphäre. Bevorzugt sind spezifisch genannte Reaktionsbedingungen.

Lösungs- und Verdünnungsmittel sind beispielsweise Wasser, Alkohole, z.B. Niederalkanole wie Methanol, Ethanol oder Propanol, Diole, wie Ethylenglykol, Triole, wie Glycerin, oder Arylalkohole, wie Phenol, Säureamide, z.B. Carbonsäuramide, wie Dimethylformamid, Dimethylacetamid oder 1,3-Dimethyl-3,4,5,6-tetrahydro-2(1H)-pyrimidinon (DMPU), oder Amide anorganischer Säuren, wie Hexamethylphosphorsäuretriamid, Ether, z.B. cyclische Ether, wie Tetrahydrofuran oder Dioxan, oder acyclische Ether, wie Diethylether oder Ethylenglykoldimethylether, halogenierte Kohlenwasserstoffe, wie Haloniederalkane, z.B. Methylenchlorid oder Chloroform, Ketone, wie Aceton, Nitrile, wie Acetonitril, Säureanhydride, wie Acetanhydrid, Ester, wie Essigsäureethylester, Bisalkansulfine, wie Dimethylsulfoxid, Stickstoffheterocyclen, wie Pyridin, Kohlenwasserstoffe, z.B. Niederalkane, wie Heptan, oder Aromaten, wie Benzol oder Toluol, oder Gemische dieser Lösungsmittel, wobei die jeweils geeigneten Lösungsmittel für die oben genannten Reaktionen ausgewählt werden können.

Infolge der engen Beziehung zwischen den Verbindungen der Formel I und ihren Vorstufen in freier Form und in Form von Salzen und/oder Tautomeren sind vor- und nachstehend unter den freien Verbindungen und Ausgangsmaterialien bzw. ihren Salzen und/oder Tautomeren sinn- und zweckmässig gegebenenfalls auch die entsprechenden Salze bzw. freien Verbindungen und/oder Tautomeren zu verstehen, sofern die Verbindungen eine oder mehrere salzbildende Gruppen, z. B. basische Gruppen, wie Amino- oder Iminogruppen, auch solche, die an nicht mehr als ein ungesättigtes Kohlenstoffatom, wie die Gruppen -NA₁Ar₁ und/oder -NA₂Ar₂ am C-Atom des zentralen Phenylringes Rest, worin Ar₁ und A₁ und/oder Ar₂ und A₂ nicht über ein ungesättigtes Kohlenstoffatom gebunden sind, und/oder saure Gruppen, wie Carboxy oder Sulfo (SO₃H), und/oder tautomerisierbare Gruppen enthalten. Sofern im Zusammenhang mit Ausgangsmaterialien, Zwischenprodukten oder Verbindungen der Formel I vor- und nachstehend ein Substituent, eine Verbindung, ein Tautomeres, ein Salz, Substituenten, Verbindungen, Tautomere oder Salze erwähnt werden, ist dies, soweit sinnvoll und zweckmässig, unabhängig von der Verwendung des Singulars oder Plurals zu verstehen als "ein oder mehrere". Ausgangsmaterialien können auch in geschützter Form verwendet werden, soweit dies erforderlich, sinnvoll und zweckmässig ist, wobei die Schutzgruppen zu geeigneten Zeitpunkten abgespalten werden können. Schutzgruppen, ihre Einführung und ihre Abspaltung sind insbesondere wie oben unter Verfahren a) definiert.

Die Verbindungen, einschliesslich ihrer Salze, können auch in Form von Hydraten erhalten werden, oder ihre Kristalle können z.B. das zur Kristallisation verwendete Lösungsmittel einschliessen.

Im Verfahren der vorliegenden Erfindung werden vorzugsweise solche Ausgangsmaterialien eingesetzt, die zu den eingangs als bevorzugt beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Reihenfolge und Reaktionsbedingungen aller beschriebenen Umsetzungen sind vorzugsweise so zu wählen, wie es für den Fachmann als sinn- und zweckmässig anzusehen ist.

### Pharmazeutische Präparate und Verfahren

Die vorliegende Erfindung betrifft ebenfalls pharmazeutische Präparate, die als Wirkstoff Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Bevorzugt ist eine pharmazeutische Zusammensetzung, welche geeignet ist zur Verabreichung an einen Warmblüter, insbesondere Menschen, der an einer Erkrankung leidet, die auf eine Hemmung einer Proteinkinase anspricht, beispielsweise Psoriasis oder ein Tumor, umfassend eine zur Hemmung der Proteinkinase wirksame Menge einer Verbindung der Formel I oder eines Salzes davon, falls salzbildende Gruppen vorliegen, zusammen mit mindestens einem pharmazeutisch annehmbaren Trägermaterial.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,05 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon oder Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, z.B. fetten Oelen, ®Lauroglykol (Gattefossé S.A., Saint Priest, France), ®Gelucire (Gattefossé S.A., Saint Priest, France) oder Sesamöl, Paraffinöl oder flüssigen Polyethylenglykolen, wie PEG 300 oder 400 (Fluka, Schweiz), gelöst oder suspendiert, wobei ebenfalls Stabilisatoren oder Detergentien zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zur parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände, insbesondere solcher, die auf eine Hemmung von Proteinkinasen ansprechen. Die Verbindungen der vorliegenden Erfindung können prophylaktisch oder therapeutisch verabreicht werden, vorzugsweise in einer gegen die genannten Krankheiten wirksamen Menge an einen Warmblüter, z. B. Menschen, der einer derartigen Behandlung bedarf, wobei man sie vorzugsweise in Form von pharmazeutischen Präparaten verwendet. Dabei wird bei einem Körpergewicht von etwa 70 kg eine tägliche Dosis von 1 mg bis 5000 mg, z.B. etwa 0,1 g bis etwa 5 g, vorzugsweise von etwa 0,5 g bis etwa 2 g, einer Verbindung der vorliegenden Erfindung verabreicht.

Die nachfolgenden Beispiele illustrieren die vorliegende Erfindung; Temperaturen werden in Grad Celsius angegeben. Folgende Abküzungen werden verwendet: ber. = berechnet; Ether = Diethylether; FAB-MS = Fast Atom Bombardment Mass Spectroscopy; FD-MS = Felddesorptions-Massenspektroskopie; gef. = gefunden; gesätt. = gesättigt; mbar = Druckeinheit (1 mbar = 1 Hectopascal); ¹H-NMR = Protonen-Kenmagnetische Resonanz; Smp. = Schmelzpunkt.

Mengenverhältnisse von Lösungsmittel-/Elutionsmittelgemischen beziehen sich, wenn nicht anders angegeben, auf Volumenverhältnisse (v/v).

### Beispiel 1: 6,7-Dianilino-2H,3H-phthalazin-1,4-dion

1,2 g 4,5-Dianilinophthalsäureanhydrid werden in 60 ml Tetrahydrofuran mit 1,2 g Hydrazinhydrat während 90 min unter Rückfluss erhitzt. Dann dampft man das Reaktionsgemisch im Vakuum ein und kristallisiert den Eindampfrückstand dreimal aus Methanol/Ether aus. Man erhält so die Titelverbindung in Form von farblosen Kristallen,
Smp. 268-270 °C.
C₂₀H₁₆N₄O₂: Molekulargewicht ber. 344, gef. 344 (FD-MS).

Die Ausgangsmaterialien werden wie folgt hergestellt:
a) 4,5-Dianilino-phthalsäureanhydrid
   Eine Lösung von 2 g 4,5-Dianilino-phthalsäure in Essigsäureanhydrid wird 30 min auf 60°C erhitzt, wobei starke Gelbfärbung auftritt. Nach dem Eindampfen bleiben gelbe Kristalle von 4,5-Dianilno-phthalsäureanhydrid zurück, die aus Aceton/Ether umkristallisiert werden und dann bei 196-197°C schmelzen.
   C₂₀H₁₄N₂O₃: Molekulargewicht ber. 330, gef. 330 (FD-MS).
b) 4,5-Dianilino-phthalsäure
   Man erhitzt 4,5-Dianilino-phthalsäuredimethylester (3,05 g) in einem Gemisch aus Methanol (500 ml) und 1N Natronlauge (100 ml) während 2 h unter Sauerstoffausschluss und Rückfluss. Danach dampft man das Methanol im Vakuum ab und säuert die alkalische Lösung des Reaktionsprodukts mit Salzsäure an. Die 4,5-Dianilinophthalsäure scheidet sich rasch kristallin ab und wird nach Umkristallisieren aus Methylenchlorid in schwach gelblichen spitzen Prismen erhalten, die bei 169°C unter Zersetzung schmelzen.
   C₂₀H₁₆N₂O₄: Molekulargewicht ber. 348, gef. 348 (FD-MS).
c) 4,5-Dianilino-phthalsäuredimethylester
   Eine Lösung von 5,6 g (15 mmol) 4,5-Bis(trimethylsilyloxy)cyclohexa-1,4-dien-1,2-dicarbonsäuredimethylester und 5,5 ml (60 mmol) Anilin in 60 ml Eisessig wird während 4 h unter Rückfluss gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Das Rohprodukt wird aus Ethanol umkristallisiert. Auf diese Weise wird die Titelverbindung in Form gelber Kristalle erhalten, Smp. 178°, FAB-MS: 377 [M⁺+H].
d) ) 4,5-Bis(trimethylsilyloxy)cyclohexa-1,4-dien-1,2-dicarbonsäuredimethylester
   Unter Argon wird eine Lösung von 7,1 g (50 mmol) Acetylendicarbonsäuredimethylester (Fluka, Schweiz) in 30 ml Toluol zu 12,5 g (50 mmol) 2,3-Bis(trimethylsilyloxy)-1,3-butadien (Aldrich, Bundesrepublik Deutschland) (95%) getropft und anschliessend 19 h unter Rückflussbedingungen gekocht. Das Reaktionsgemisch wird abgekühlt, das Lösungsmittel abgedampft und der Rückstand im Hochvakuum (0,1 mbar, 124-127°) destilliert. Auf diese Weise wird die Titelverbindung als gelbes, hochviskoses Oel erhalten, ¹H-NMR (CDCl₃): δ = 0.18 (s, 18H), 3.09 (s, 4H), 3.78 (s, 6H).

### Beispiel 2: 6.7-Bis(4-fluoranilino)-2H,3H-Phthalazin-1.4-dion

Man erhitzt 0,60 g 4,5-Bis(4-fluoranilino)phthalsäureanhydrid in 30 ml Tetrahydrofuran mit 0,60 g Hydrazinhydrat 90 min unter Rückfluss, dampft dann das Reaktiongemisch im Vakuum ein und kristallisiert den Eindampfrückstand mehrmals aus Methanol. Man erhält die Titelverbindung in feinen, weissen Prismen, Smp. 165-168 °C.

| Elementaranalyse: C₂₀H₁₄F₂N₄O₂ (380,35): | | | |
|---|---|---|---|
| ber.: | C 63,16%, | H 3,71%, | N 14,73%; |
| gef.: | C 62.88% | H 3,98% | N 14,77% |

Die Ausgangsmaterialien werden wie folgt hergestellt:
a) 4,5-Bis(4-fluoranilino)-phthalsäureanhydrid
   Bis(4-fluoranilino)-phthalsäuredimethylester (2 g) wird analog Beispiel 1 b) mit wässrig-methanolischer Natronlauge verseift. Die entstandene freie Dicarbonsäure wird ohne weitere Reinigung direkt mit Essigsäureanhydrid analog Beispiel 1 a) in das Anhydrid überführt (Erwärmen auf 50 ⁰C, bis Anhydridbildung vollständig, dann Eindampfen und Filtration des Eindampfrückstandes gelöst in Ethylacetat durch eine geringe Menge Kieselgel, um dunkle Verunreinigungen zu entfernen). Das erhaltene 4,5-Bis(4-fluoranilino)-phthalsäureanhydrid bildet aus Ethylacetat/Ether gelbe, glänzende Kristalle vom Smp. 225-227 ⁰C.
   C₂₀H₁₂N₂F₂O₃: Molekulargewicht ber. 366, gef. 366 (FD-MS).
b) 4,5-Bis(4-fluoranilino)phthalsäuredimethylester
   Eine Lösung von 2,4 g (6 mmol) 4,5-Bis(trimethylsilyloxy)cyclohexa-1,4-dien-1,2-dicarbonsäuremethylester (Beispiel 1d) und 2,3 ml (24 mmol) 4-Fluoranilin in 60 ml Eisessig wird während 2 h unter Rückflussbedingungen gekocht. Das Reaktionsgemisch wird abgekühlt, das Lösungsmittel abgedampft, der dunkelbraune Rückstand in Dichlormethan gelöst und die Lösung nacheinander mit 20 ml 1N HCl, 50 ml gesätt. NaHCO₃ und zweimal 20 ml Wasser gewaschen, mit Natriumsulfat getrocknet und eingedampft. Der Eindampfrückstand wird mit Ethylacetat/Hexan 2:1 an Kieselgel chromatographiert, die Produktfraktionen eingedampft und aus Ethylacetat/Hexan umkristallisiert. Auf diese Weise wird die Titelverbindung in Form gelber Kristalle erhalten, ¹H-NMR (CDCl₃): δ = 7.40 (s, 2H), 7.10-6.80 (m, 8H), 5.70 (br s, 2H), 3.83 (s, 6H).

### Beispiel 3: 6,7-Bis(4-methylanilino)-2H,3H-phthalazin-1,4-dion

Analog der in Beispiel 1 beschriebenen Reaktion erhält man beim Umsetzen von 0,56 g 4,5-Bis(4-methyanilino)-phthalsäureanhydrid mit 0,6 g Hydrazinhydrat in 50 ml Tetrahydrofuran die Titelverbindung, welche aus Methanol in farblosen, fein verfilzten Nadeln kristallisiert. Smp. ca. 170 °C.
C₂₂H₂₀N₄O₂: Molekulargewicht ber. 372, gef. 372 (FD-MS).

Die Ausgangsmaterialien werden wie folgt hergestellt:
a) 4,5-Bis(4-methylanilino)-phthalsäureanhydrid
   Man erhitzt 4,5-Bis(4-methylanilino)-phthalsäuredimethylester (4,45 g, 0,011 mol) mit 50 ml 2M Natronlauge und 1000 ml Methanol unter Rückfluss und Stickstoffatmosphäre. Danach wird auf 300 ml eingeengt, mit 5M HCl angesäuert und die gebildete Dicarbonsäure mehrmals mit Essigsäureethylester extrahiert. Die vereinigten Extrakte werden getrocknet (Dinatriumsulfat) und eingedampft. Der bräunliche Eindampfrückstand wird in Essigsäureanhydrid gelöst und 10 min auf 40 °C erwärmt. Danach wird wieder eingedampft und der Rückstand durch Filtration über Kieselgel (Lösungsmittel Essigester/Hexan 1:1) gereinigt. Man erhält das 4,5-Bis(4-methylanilino)phthalsäureanhydrid als stark gelbgefärbtes Filtrat. Nach Eindampfen wird aus Diethylether kristallisiert unter Erhalt der Titelverbindung: Smp. 221-223 °C; C₂₂H₂₈N₂O₃: Molekulargewicht ber. 358, gef. 358 (FD-MS).
b) 4,5-Bis(4-methylanilino)-phthalsäuredimethylester
   Analog Beispiel 1 c) erhält man ausgehend von 23,1 g (60,8 mmol) 4,5-Bis(trimethylsilyloxy)cyclohexa-1,4-dien-1,2-dicarbonsäuredimethylester und 22,7 g (0,21 mol) 4-Toluidin die Titelverbindung, welche aus Ethylacetat/Ether in Form gelblicher, glänzender Kristallplättchen erhalten wird. Smp. 170-172 °C.

### Beispiel 4: 6,7-Dianilino-2,3-dimethyl-2H,3H-phthalazin-1,4-dion

0,250 g 4,5-Dianilinophthalsäureanhydrid (Beispiel 1 a) werden mit 0,110 g N,N'-Dimethylhydrazin-dihydrochlorid (Fluka, Schweiz) und 0,2155 g Ethyldiisopropylamin in 50 ml Dimethylformamid während 14 h auf 80 ⁰C erhitzt. Dann dampft man das Reaktionsgemisch im Vakuum ein. Der Eindampfrückstand wird an Kieselgel durch Chromatographie getrennt. Mit Hexan/Ethylacetat 2:1 wird eine gelbe Verunreinigung entfernt. Die Hauptmenge wird mit Ethylacetat/Methanol 9:1 eluiert. Nach dem Eindampfen des Eluates erhält man die Titelverbindung aus Methanol/Ether in farblosen Kristallen. Smp. 217-218 ⁰C.
C₂₂H₂₀N₄O₂: Molekulargewicht ber. 372, gef. 372 (FD-MS)

### Beispiel 5: 6,7-Dianilino-4-hydroxy-2-methyl-2H-phthalazin-1-on

Analog dem in Beispiel 1 beschriebenen Verfahren wird durch Erhitzen von 0,20 g 4,5-Dianilinophthalsäureanhydrid (Beispiel 1 a) mit überschüssigem N-Methylhydrazin (0,20 g; Fluka, Schweiz) in 30 ml Tetrahydrofuran während 90 min die Titelverbindung erhalten. Sie bildet nach Auskristallisieren aus Tetrahydrofuran/Ether farblose Kristalle.
Smp. 276-278 °C.
C₂₁H₁₈N₄O₂: Molekulargewicht ber. 358, gef. 358 (FD-MS).

### Beispiel 6: 2-Aminothiocarbonyl-6,7-dianilino-4-hydroxy-2H-phthalazin-1-o n

Man erhitzt eine Lösung von 0,250 g 4,5-Dianilinophthalsäureanhydrid (Beispiel 1 a) und 0,069 g Thiosemicarbazid (Fluka, Schweiz) in 70 ml Methanol 3 h unter Rückfluss. Der nach dieser Zeit aus dem Reaktionsgemisch abgeschiedene gelbliche Feststoff wird abfiltriert und zweimal aus Dimethylformamid kristallisiert. Man erhält die Titelverbindung in Form von blassgelblichen Kristallen. Smp. 230-232 °C.
C₂₁H₁₇N₅O₂S: Molekulargewicht ber. 403, gef. 403 (FD-MS).

### Beispiel 7: 6,7-Dianilino-4-hydroxy-2-(2-hydroxyethyl)-2H-phthalazin-1-on

Analog dem in Beispiel 1 beschriebenen Verfahren wird durch Erhitzen von 0,23 g 4,5-Dianilinophthalsäureanhydrid (Beispiel 1 a) mit überschüssigem 2-Hydroxyethylhydrazin (0,12 g) in 50 ml Tetrahydrofuran während 2 h die Titelverbindung erhalten.
C₂₂H₂₀N₄O₃: Molekulargewicht ber. 388, gef. 388 (FD-MS).

### Beispiel 8: 6,7-Dianilino-2H-phthalazin-1-on

Eine Lösung von 6,7-Dianilino-3-hydroxy-*2H*-phthalazin-1-on (0,078 g, 0,235 mmol) in Dioxan (10 ml) wird im Druckrohr mit Hydrazinhydrat (0,1 ml, etwa 2 mmol) während 4 h auf 120 °C erhitzt. Das Reaktionsgemisch wird nach dem Eindampfen an Kieselgel mit dem Laufmittel Essigsäureethylester/Hexan 2:1 gereinigt. Man erhält dabei ein Material vom R_{f}=0,35 (Dünnschichtchromatographie auf Kieselgel 60, Merck, Darmstadt), das nach zweimaligem Kristallisieren aus Diethylether die reine Titelverbindung ergibt, welche bei 250-255 °C unter teilweiser Zersetzung schmilzt (cremefarbene Plättchen).
C₂₀H₁₆N₄O, Molekulargewicht ber. 328, gef. 328 (FD-MS).

Das Ausgangsmaterial wird folgendermassen hergestellt:
a) 4,5-Bis(anilino)phthalimid
   Eine Suspension von 230 mg (0,7 mmol) 4,5-Bis(anilino)phthalsäuredimethylester (Beispiel 1 c)) in 23 ml Ethylenglykol wird auf 120° erwärmt; unter Rühren wird während 24 h Ammoniak-Gas durchgeleitet. Das Reaktionsgemisch wird abgekühlt und mit Ethylacetat extrahiert. Die Ethylacetat-Phasen werden nacheinander dreimal mit Wasser und einmal mit gesätt. Kochsalzlösung gewaschen, mit Natriumsulfat getrocknet und eingedampft. Der Eindampfrückstand wird mit Dichlormethan/Methanol 40:1 an Kieselgel chromatographiert, die Produktfraktionen vereinigt und eingedampft. Auf diese Weise wird die Titelverbindung in Form gelber Kristalle erhalten, Smp. 215-217°, FAB-MS: 330 [M⁺+H].
b) 6,7-Dianilino-3-hydroxy-*2H*-phthalazin-1-on:
   Zu einer Lösung von 4,5-Bisanilino-phthalimid (0,493 g, 1,49 mmol) in Tetrahydrofuran (150 ml) fügt man unter Rühren portionsweise festes Lithiumaluminiumhydrid (gesamt 0,34 g, 8,9 mmol). Danach ist die anfänglich stark gelbgefärbte Lösung nur mehr schwach gelblich gefärbt. Nun setzt man Wasser und soviel Zitronensäurelösung zu, dass ein pH von etwa 4,5 erreicht wird. Man filtriert durch ®Celite (Filterhilfsmittel auf Kieselgurbasis, Fluka, Schweiz), wäscht mit Tetrahydrofuran und Essigester nach und extrahiert darauf das Filtrat mit Essigester. Nach dem Trocknen und Eindampfen bleibt ein amorpher Lack zurück, der beim Anreiben mit einer geringen Menge Essigsäureethylester kristallisiert. Man erhält so schwach gelblich gefärbte Kristalle der Titelverbindung, die zur Reinigung aus Methanol/Ether umkristallisiert werden. Smp. ab 220 °C (Zers.);
   C₂₀H₁₇N₃O₂, Molekulargewicht ber. 331, gef. 331 (FD-MS).

### Beispiel 9: (A) 6-Anilino-7-cyclohexylamino-2H-phthalazin-1-on und (B) 6,7-Dicyclohexylamino-2H-phthalazin-1-on

6,7-Dianilino-*2H*-phthalazin-1-on (Beispiel 8; 0,092 g, 0,028 mmol) wird in Eisessig (10 ml) mit Platindioxid (PtO₂; 0,002 g) während 24 h hydriert. Das bei der Hydrierung entstehende Material wird durch Chromatographie an Kieselgel mit dem Elutionsmittel Essigsäureethylester/Hexan 1:1 in zwei Komponenten getrennt. Die rascher wandernde Substanz bildet aus Isopropylalkohol/Diethylether farblose, sechseckige Kristalle vom Smp. 265-266 °C (Titelverbindung (A)), C₂₀H₂₂N₄O: Molekulargewicht ber. 334, gef. 335 (M+H)⁺ (FAB-MS). Die später eluierte, wenig langsamer wandernde Substanz kristallisiert beim Eindampfen ihrer Lösung; nach dem Waschen der Kristalle mit Diethylether erhält man die Titelverbindung (B), Smp. 285 °C, farblose Kristalle;
C₂₀H₂₈N₄O, Molekulargewicht ber. 340, gef. 341 (M+H)⁺ (FAB-MS).

### Beispiel 10: Kapseln

Es werden 5000 Kapseln hergestellt, die je 0,25 g Wirkstoff, z.B. eine der in den Beispielen 1-9 hergestellten Verbindungen, enthalten:

| Zusammensetzung | |
|---|---|
| Wirkstoff | 1250 g |
| Talk | 180 g |
| Weizenstärke | 120 g |
| Magnesiumstearat | 80 g |
| Laktose | 20 g |

Verfahren: Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

## Patentansprüche

1. Verbindungen der Formel I, 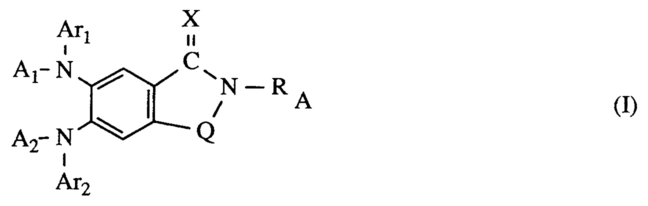 worin A₁ und A₂ unabhängig voneinander Wasserstoff, unsubstituiertes oder substituiertes Niederalkyl, unsubstituiertes oder substituiertes Niederalkenyl, unsubstituiertes oder substituiertes Niederalkinyl, Heterocyclylniederalkyl, Acyl, Niederalkylsulfonyl oder Arylsulfonyl bedeuten, oder worin A₁ und A₂ zusammen unsubstituiertes oder substituiertes Niederalkylen bedeuten; Ar₁ und Ar₂ unabhängig voneinander Aryl, Heteroaryl oder unsubstituiertes oder substituiertes Cycloalkyl bedeuten; X für Sauerstoff oder Schwefel steht; und worin Q einen bivalenten Rest der Formel 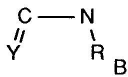 bedeutet, welcher über sein Y tragendes Kohlenstoffatom an das Q bindende Kohlenstoffatom des Benzolringes und über sein R_{B} tragendes Stickstoffatom an das R_{A} tragende Stickstoffatom in Formel I gebunden ist, worin Y unabhängig von X für Sauerstoff oder Schwefel steht und R_{A} und R_{B} unabhängig voneinander Wasserstoff, unsubstituiertes oder substituiertes Niederalkyl oder Acyl bedeuten; oder worin Q einen bivalenten Rest der Formel 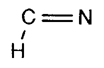 bedeutet, welcher über sein Kohlenstoffatom an das Q bindende Kohlenstoffatom des Benzokinges und über sein Stickstoffatom an das R_{A} tragende Stickstoffatom in Formel I gebunden ist, wobei R_{A} eine der genannten Bedeutungen ausser Acyl hat; Salze davon, sofern salzbildende Gruppen vorliegen, und Tautomere davon, sofern tautomerisierbare Gruppen vorliegen.

2. Verbindungen der Formel I gemäss Anspruch 1, worin R_{A} und, falls vorhanden, R_{B} jeweils Wasserstoff bedeuten und die übrigen Reste die genannten Bedeutungen haben, Salze davon, sofern salzbildende Gruppen vorliegen, und Tautomere davon, sofern tautomerisierbare Gruppen vorliegen.

3. Verbindungen der Formel I gemäss Anspruch 1, worin A₁ und A2 unabhängig voneinander Wasserstoff, Niederalkyl; substituiertes Niederalkyl, welches durch bis zu 2 Reste ausgewählt aus Amino, Mono- oder Diniederalkylamino, worin der Niederalkylrest ein- oder zweifach durch Hydroxy, Niederalkoxy, Phenylniederalkoxy, Niederalkanoyloxy, Halogen, Amino, Niederalkylamino, Diniederalkylamino, Mereapto, Niederalkylthio, Niederalkylsulfinyl, Niederalkylsulfonyl, Carboxy, Niederalkoxycarbonyl, Carbamoyl, N-Niederalkylcarbamoyl, N,N-Diniederalkylcarbamoyl und/oder Cyano substituiert oder unsubstituiert ist, C₃-C₈-Cycloalkylamino, Phenylniederalkylamino, Phenylamino, Niederalkanoylamino, Phenylniederalkanoylamino, Phenylcarbonylamino, Hydroxy, Niederalkoxy, worin der Niederalkylrest ein- oder zweifach wie oben in Diniederalkylaminoniederalkyl A₁ oder A₂ substituiert oder unsubstituiert ist, Phenylniederalkoxy, Niederalkanoyloxy, Mercapto, Niederalkylthio, worin der Niederalkylrest ein- oder zweifach wie oben in Diniederalkylaminoniederalkyl A₁ oder A₂ substituiert oder unsubstituiert ist, Phenylniederalkylthio, Niederalkanoylthio, Carboxy, Niederalkoxycarbonyl, Phenylniederalkoxycarbonyl, Cyano, Carbamoyl, N-Niederalkylcarbamoyl, N,N-Diniederalkylcarbamoyl, N-Hydroxycarbamoyl, N-Phenylcarbamoyl, Thiocarbamoyl, N-Niederalkylthiocarbamoyl, N,N-Diniederalkylthiocarbamoyl, Ureido, an einem oder beiden Stickstoffatomen durch Niederalkyl substituiertes Ureido, Thioureido, an einem oder beiden Stickstoffatomen durch Niederalkyl substituiertes Thioureido, Hydrazino, an einem oder beiden Stickstoffatomen durch Niederalkyl substituiertes Hydrazino, Amidino, an einem oder beiden Stickstoffatomen durch Niederalkyl substituiertes Amidino, Guanidino, an einem, zwei oder allen drei Stickstoffatomen durch Niederalkyl substituiertes Guanidino, Oxo, welches nicht am Kohlenstoff gebunden ist, welcher an den A₁ oder A₂ tragenden Stickstoff gebunden ist, Thioxo, Imino, Niederalkylimino, Niederalkanoylimino, Hydroxyimino, Niederalkoxyimino, Hydrazono, N-Mono- oder N,N-Diniederalkylhydrazono, N-Niederalkanoylhydrazono, Niederalkoxycarbonylhydrazono oder Niederalkylthioimino substituiert ist; durch einen der für substituiertes Niederalkyl A₁ und/oder A₂ genannten Reste substituiertes oder unsubstituiertes Niederalkenyl oder Niederalkinyl; Heterocyclylniederalkyl, worin Heterocyclyl ein unsubstituierter oder durch Niederalkyl, Niederalkanoyl, Hydroxy, Niederalkoxy, Halogen, Cyano und/oder Trifluormethyl substituierter, über ein Ringstickstoffatom gebundener Rest ausgewählt aus Pyrrolyl, 2,5-Dihydropyrrolyl, Pyrrolinyl, Imidazolyl, Imidazolidinyl, Pyrazolinyl, Pyrazolidinyl, Triazolyl, wie 1,2,3-, 1,2,4- oder 1,3,4-Triazolyl, Tetrazolyl, wie 1- oder 2-Tetrazolyl, Tetrahydro-oxazolyl, Tetrahydro-isoxazolyl, Tetrahydro-thiazolyl, Tetrahydro-isothiazolyl, Indolyl, Isoindolyl, Benzimidazolyl, Piperidinyl, Piperazin-1-yl, Morpholino, Thiomorpholino, S,S-Dioxothiomorpholino, 1,2-Dihydro- oder 1,2,3,4-Tetrahydrochinolyl und 1,2-Di- oder 1,2,3,4-Tetrahydroisochinolyl ist, der endständig an Niederalkyl gebunden ist; Niederalkanoyl, Halogenniederalkanoyl, Phenylniederalkanoyl, Benzoyl, Carbamoyl, N-Mono- oder N,N-Diniederalkylcarbamoyl, N-Mono- oder N,N-Bis(hydroxyniederalkyl)carbamoyl, Thiocarbamoyl, N-Mono- oder N,N-Diniederalkylthiocarbamoyl, N-Mono- oder N,N-Bis(hydroxyniederalkyl)thiocarbamoyl, Niederalkoxycarbonyl, Phenylniederalkoxycarbonyl, Niederalkylsulfonyl, Benzolsulfonyl oder im Benzolrest durch Niederalkyl, Niederalkoxy, Hydroxy, Halogen und/oder Trifluormethyl substituiertes Benzolsulfonyl bedeuten; oder worin A₁ und A₂ zusammen Niederalkylen bilden, welches unsubstituiert oder substituiert ist durch bis zu 3 Substituenten ausgewählt aus Niederalkyl, Amino, Aminoniederalkyl, Mono- oder Diniederalkylamino, Mono- oder Diniederalkylaminoniederalkyl, worin der endständige Niederalkylrest ein- oder zweifach wie oben in Diniederalkylaminoniederalkyl A₁ oder A₂ substituiert oder unsubstituiert ist, C₃-C₈-Cycloalkylamino, C₃-C₈-Cycloalkylaminoniederalkyl, Phenylniederalkylamino, Phenylniederalkylaminoniederalkyl, Phenylamino, Phenylaminoniederalkyl, Niederalkanoylamino, Phenylniederalkanoylamino, Phenylcarbonylamino, Niederakanoylamino niederalkyl, Phenylniederalkanoylaminoniederalkyl, Phenylcarbonylaminoniederalkyl, Hydroxy, Hydroxyniederalkyl, Niederalkoxy oder Niederalkoxyniederalkyl, worin der endständige Niederalkylrest ein- oder zweifach wie oben in Diniederalkylaminoniederalkyl A₁ oder A₂ substituiert oder unsubstituiert ist, Phenylniederalkoxy, Phenylniederalkoxyniederalkyl, Niederalkanoyloxy, Niederalkanoyloxyniederalkyl, Mereapto, Mercaptoniederalkyl, Niederalkylthio oder Niederalkylthioniederalkyl, worin der endständige Niederalkylrest ein- oder zweifach wie oben in Diniederalkylaminoniederalkyl A₁ oder A₂ substituiert oder unsubstituiert ist, Phenylniederalkylthio, Phenylniederalkylthioniederalkyl, Niederalkanoylthio, Niederalkanoylthioniederalkyl, Carboxy, Carboxyniederalkyl, Niederalkoxycarbonyl, Niederalkoxycarbonylniederalkyl, Phenylniederalkoxycarbonylniederalkyl, Cyano, Cyanoniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, N-Hydroxycarbamoyl, N-Hydroxycarbamoylniederalkyl, N-Phenylcarbamoyl, N-Phenylcarbamoylniederalkyl, Thiocarbamoyl, Thiocarbamoylniederalkyl, N-Niederalkylthiocarbamoyl, N-Niederalkylthiocarbamoylniederalkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl substituiertes Ureido oder Ureidoniederalkyl, Thioureido, Thioureidoniederalkyl, an einem oder beiden Stickstoffatomen durch Niederalkyl substituiertes Thioureido oder Thioureidoniederalkyl, Hydrazino, Hydrazinoniederalkyl, an einem oder beiden Stickstoffatomen durch Niederalkyl substituiertes Hydrazino oder Hydrazinoniederalkyl, Amidino, Amidinoniederalkyl, an einem oder beiden Stickstoffatomen durch Niederalkyl substituiertes Amidino oder Amidinoniederalkyl, Guanidino, Guanidinoniederalkyl, an einem, zwei oder allen drei Stickstoffatomen durch Niederalkyl substituiertes Guanidino oder Guanidinoniederalkyl, Oxo, Oxoniederalkyl, Thioxo, Thioxoniederalkyl, Imino, Iminoniederalkyl, Niederalkylimino, Niederalkyliminoniederalkyl, Niederalkanoylimino, Niederalknoyliminoniederalkyl, Hydroxyimino, Hydroxyiminoniederalkyl, Niederalkoxyimino, Niederalkoxyiminoniederalkyl, Hydrazono, Hydrazononiederalkyl, N-Mono- oder N,N-Diniederalkylhydrazono, N-Mono- oder N,N-Diniederalkylhydrazononiederalkyl, N-Niederalkanoylhydrazono, Niederalkoxycarbonylhydrazono, N-Niederaaanoylhydrazononiederalkyl, Niederalkoxycarbonylhydzazononiederalkyl, Niederalkylthioimino und Niederalkylthioiminoniederalkyl; Ar₁ und Ar₂ unabhängig voneinander unsubstituiertes oder durch einen Rest ausgewählt aus Niederalkyl, Hydroxy, Niederalkoxy, Halogen, Carboxy, Niederalkoxycarbonyl und Cyano o-, m- oder p-substituiertes Phenyl, Pentafluorphenyl, über ein Ringstickstoffatom gebundenes Heteroaryl ausgewählt aus Imidazolyl, Triazolyl, Pyridyl, Pyrimidinyl und Triazinyl, welche unsubstituiert oder durch Niederalkyl, Hydroxy, Niederalkoxy, Halogen, Cyano und/oder Trifluormethyl substituiert sind, oder C₃-C₈-Cycloalkyl, welches unsubstituiert oder durch Niederalkoxy oder Hydroxy substituiert ist, bedeuten; X für Sauerstoff oder Schwefel steht; und worin Q einen bivalenten Rest der Formel 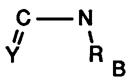 bedeutet, welcher über sein Y tragendes Kohlenstoffatom an das Q bindende Kohlenstoffatom des Benzolringes und über sein R_{B} tragendes Stickstoffatom an das R_{A} tragende Stickstoffatom in Formel I gebunden ist, worin Y unabhängig von X für Sauerstoff oder Schwefel steht und R_{A} und R_{B} unabhängig voneinander Wasserstoff, unsubstituiertes oder substituiertes Niederalkyl, wie oben für A₁ und A₂ definiert; Niederalkanoyl, Halogenniederakanoyl, Phenylniederalkanoyl, Benzoyl; Carbamoyl, N-Mono- oder N,N-Diniederalkylcarbamoyl, N-Mono- oder N,N-Bis(hydroxyniederalkyl)carbamoyl, Thiocarbamoyl, N-Mono- oder N,N-Diniederalkylthiocarbamoyl, N-Mono- oder N,N-Bis(hydroxyniederalkyl)thiocarbamoyl, Niederalkoxycarbonyl oder Phenylniederalkoxycarbonyl bedeuten; oder worin Q einen bivalenten Rest der Formel 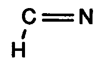 bedeutet, welcher über sein Kohlenstoffatom an das Q bindende Kohlenstoffatom des Benzolringes und über sein Stickstoffatom an das R_{A} tragende Stickstoffatom in Formel I gebunden ist, wobei R_{A} unsubstituiertes oder substituiertes Niederalkyl, vorzugsweise wie oben definiert, bedeutet; Salze davon, sofern salzbildende Gruppen vorliegen, und Tautomere davon, sofern tautomerisierbare Gruppen vorliegen.

4. Verbindungen der Formel I gemäss Anspruch 3, worin Q einen bivalenten Rest der Formel 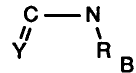 bedeutet, welcher über sein Y tragendes Kohlenstoffatom an das Q bindende Kohlenstoffatom des Benwlringes und über sein R_{B} tragendes Stickstoffatom an das R_{A} tragende Stickstoffatom in Formel I gebunden ist, worin Y unabhängig von X für Sauerstoff oder Schwefel steht und R_{A} und R_{B} unabhängig voneinander Wasserstoff, unsubstituiertes oder substituiertes Niederalkyl, wie oben für A₁ und A₂ definiert; Niederalkanoyl, Halogenniederalkanoyl, Phenylniederakanoyl, Benzoyl; Carbamoyl, N-Mono- oder N,N-Diniederalkylcarbamoyl, N-Mono- oder N,N-Bis(hydroxyniederalkyl)carbamoyl, Thiocarbamoyl, N-Mono- oder N,N-Diniederalkylthiocarbamoyl, N-Mono- oder N,N-Bis(hydroxyniederalkyl)thiocarbamoyl, Niederalkoxycarbonyl oder Phenylniederalkoxycarbonyl bedeuten, und worin die übrigen Reste die genannten Bedeutungen haben, Salze davon, sofern salzbildende Gruppen vorliegen, und Tautomere davon, sofern tautomerisierbare Gruppen vorliegen.

5. Verbindungen der Formel I gemäss Anspruch 3, worin Q einen bivalenten Rest der Formel 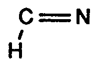 bedeutet, welcher über sein Kohlenstoffatom an das Q bindende Kohlenstoffatom des Benzolringes und über sein Stickstoffatom an das R_{A} tragende Stickstoffatom in Formel I gebunden ist, und worin die übrigen Reste die genannten Bedeutungen haben, Salze davon, sofern salzbildende Gruppen vorliegen, und TAutomere davon, sofern tautomersierbare Gruppen vorliegen.

6. Verbindungen der Formel I gemäss Anspruch 1, worin A₁ und A₂ unabhängig voneinander Wasserstoff, Niederalkyl; substituiertes Niederalkyl, welches durch bis zu 2 Reste ausgewählt aus Amino, Mono- oder Diniederalkylamino, Hydroxy, Niederalkoxy, Carboxy, Niederalkoxycarbonyl, Phenylniederalkoxycarbonyl, Cyano, Carbamoyl, N-Niederalkylcarbamoyl, N,N-Diniederalkylcarbamoyl, Ureido, an einem oder beiden Stickstoffatomen durch Niederalkyl substituiertes Ureido, Thioureido oder an einem oder beiden Stickstoffatomen durch Niederalkyl substituiertes Thioureido substituiert ist; Niederalkenyl oder Niederalkinyl bedeuten; oder worin A₁ und A₂ zusammen Niederalkylen bilden, welches unsubstituiert oder substituiert ist durch Niederalkyl, Amino, Aminoniederalkyl, Mono- oder Diniederalkylamino, Hydroxy, Hydroxyniederalkyl, Niederalkoxy oder Niederalkoxyniederalkyl, Carboxy, Carboxyniederalkyl, Niederalkoxycarbonyl, Niederalkoxycarbonylniederalkyl, Cyano, Cyanoniederalkyl, Carbamoyl, Carbamoylniederalkyl, N-Niederalkylcarbamoyl, N,N-Diniederalkylcarbamoyl, N-Niederalkylcarbamoylniederalkyl, N,N-Diniederalkylcarbamoylniederalkyl, Thiocarbamoyl, Thiocarbamoylniederalkyl, N-Niederalkylthiocarbamoyl, N-Niederalkylthiocarbamoylniederalkyl, N,N-Diniederalkylthiocarbamoyl, N,N-Diniederalkylthiocarbamoylniederalkyl, Ureido, Ureidoniederalkyl, an einem oder beiden Stickstoffatomen durch Niederalkyl substituiertes Ureido oder Ureidoniederalkyl, Thioureido, Thioureidoniederalkyl oder an einem oder beiden Stickstoffatomen durch Niederalkyl substituiertes Thioureido oder Thioureidoniederalkyl; Ar₁ und Ar₂ unabhängig voneinander unsubstituiertes oder durch einen Rest ausgewählt aus Niederalkyl, Hydroxy, Niederalkoxy, Halogen, Carboxy, Niederalkoxycarbonyl und Cyano o-, m- oder p-substituiertes Phenyl, Pentafluorphenyl oder C₃-C₈-Cycloalkyl bedeuten, vorzugsweise beide den gleichen Rest; X für Sauerstoff oder Schwefel steht; und worin Q einen bivalenten Rest der Formel 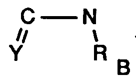 bedeutet, welcher über sein Y tragendes Kohlenstoffatom an das Q bindende Kohlenstoffatom des Benzolringes und über sein R_{B} tragendes Stickstoffatom an das R_{A} tragende Stickstoffatom in Formel I gebunden ist, worin Y unabhängig von X für Sauerstoff oder Schwefel steht, wobei R_{A} und R_{B} unabhängig voneinander Wasserstoff, unsubstituiertes oder durch einen Rest ausgewählt aus Amino, Mono- oder Diniederalkylamino, Hydroxy und Niederalkoxy substituiertes Niederalkyl; Niederalkanoyl, Carbamoyl, N-Mono- oder N,N-Diniederalkylcarbamoyl, N-Mono- oder N,N-Bis(hydroxyniederalkyl)carbamoyl, Thiocarbamoyl, N-Mono- oder N,N-Diniederalkylthiocarbamoyl, N-Mono- oder N,N-Bis(hydroxyniederalkyl)thiocarbamoyl, Niederalkoxycarbonyl oder Phenylniederalkoxycarbonyl bedeuten; oder worin Q einen bivalenten Rest der Formel 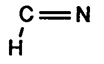 bedeutet, welcher über sein Kohlenstoffatom an das Q bindende Kohlenstoffatom des Benzolringes und über sein Stickstoffatom an das R_{A} tragende Stickstoffatom in Formel I gebunden ist, wobei R_{A} unsubstituiertes oder substituiertes Niederalkyl, wie oben definiert, bedeutet; Salze davon, sofern salzbildende Gruppen vorliegen, und Tautomere davon, sofern tautomerisierbare Gruppen vorliegen.

7. Verbindungen der Formel I gemäss Anspruch 1 mit der Formel IA, 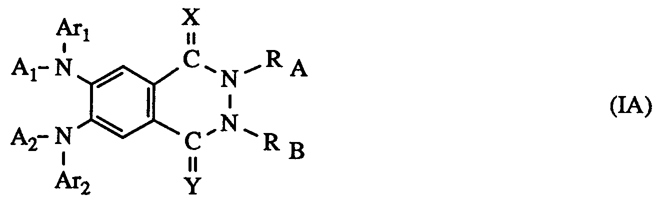 worin A₁ und A₂ unabhängig voneinander Wasserstoff oder Niederalkyl bedeuten, oder worin A₁ und A₂ zusammen Niederalkylen bedeuten; Ar₁ und Ar₂ unabhängig voneinander unsubstituiertes oder durch Halogen oder Niederalkyl in o-, m- oder p-Stellung substituiertes Phenyl bedeuten; X und Y unabhängig voneinander Sauerstoff oder Schwefel bedeuten; und R_{A} und R_{B} unabhängig voneinander Wasserstoff, Niederalkyl, Hydroxyniederalkyl, Carbamoyl oder Thiocarbamoyl bedeuten; und Tautomere davon, sofern tautomerisierbare Gruppen vorliegen.

8. Verbindungen der Formel I gemäss Anspruch 1 mit der Formel IB, 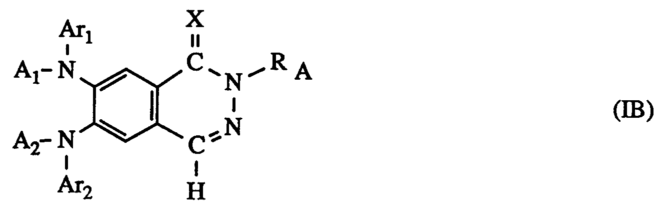 worin A₁ und A₂ unabhängig voneinander Wasserstoff oder Niederalkyl bedeuten, oder worin A₁ und A₂ zusammen Niederalkylen bedeuten; Ar₁ und Ar₂ unabhängig voneinander unsubstituiertes oder durch Halogen oder Niederalkyl in o-, m- oder p-Stellung substituiertes Phenyl oder Cyclohexyl, welches unsubstituiert oder durch Niederalkyl, Hydroxy oder Halogen substituiert ist, bedeuten; X Sauerstoff oder Schwefel bedeutet; und R_{A} Wasserstoff, Niederalkyl, Hydroxyniederalkyl, wie 2-Hydroxyethyl, Carbamoyl oder Thiocarbamoyl bedeutet; und Tautomere davon, sofern tautomerisierbare Gruppen vorliegen.

9. Verbindungen der Formel IA gemäss Anspruch 7, worin A₁ und A₂ Wasserstoff bedeuten, Ar₁ und Ar₂ unabhängig voneinander einen Rest ausgewählt aus Phenyl, 4-Fluorphenyl oder 4-Methylphenyl bedeuten, X und Y unabhängig voneinander Sauerstoff oder Schwefel bedeuten und R_{A} und R_{B} unabhängig voneinander Wasserstoff, Methyl, 2-Hydroxyethyl oder Carbamoyl bedeuten; und Tautomere davon, sofern tautomerisierbare Gruppen vorliegen.

10. Verbindungen der Formel IB gemäss Anspruch 8, worin A₁ und A₂ Wasserstoff bedeuten, Ar₁ und Ar₂ unabhängig voneinander einen Rest ausgewählt aus Phenyl und Cyclohexyl bedeuten, X Sauerstoff oder Schwefel bedeutet und R_{A} Wasserstoff bedeutet.

11. Verbindungen gemäss Anspruch 1, worin A₁ und A₂ Wasserstoff bedeuten; oder worin einer der beiden Reste A₁ und A₂ Wasserstoff bedeutet, der andere unter die Definition unsubstituiertes oder durch einen der jeweils genannten Substituenten substituiertes Niederalkyl fällt; oder worin A₁ und A₂ zusammen einen Rest bedeuten, der unter die Definitionen von unsubstituiertem oder durch einen der jeweils genannten Substituenten substituiertem Niederalkylen fällt; während die übrigen Reste die genannten Bedeutungen haben, pharmazeutisch verwendbare Salze davon, sofern salzbildende Gruppen vorliegen, und Tautomere davon, sofern tautomerisierbare Gruppen vorliegen.

12. Eine Verbindung der Formel I gemäss Anspruch 1 mit der Bezeichnung 6,7-Dianilino-2H-3H-phthalazin-1,4-dion.

13. Eine Verbindung der Formel I gemäss Anspruch 1 mit der Bezeichnung 6,7-Bis(4-fluoranilino)-2H,3H-phthalazin-1,4-dion.

14. Eine Verbindung der Formel I gemäss Anspruch 1 mit der Bezeichnung 6,7-Bis(4-methylanilino)-2H,3H-phthalazin-1,4-dion.

15. Eine Verbindung der Formel I gemäss Anspruch 1 mit der Bezeichnung 6,7-Dianilino-2,3-dimethyl-2H,3H-phthalazin-1,4-dion.

16. Eine Verbindung der Formel I gemäss Anspruch 1 mit der Bezeichnung 6,7-Dianilino-4-hydroxy-2-methyl-2H-phthalazin-1-on.

17. Eine Verbindung der Formel I gemäss Anspruch 1 mit der Bezeichnung 2-Aminothiocarbonyl-6,7-dianilino-4-hydroxy-2H-phthalazin-1-on.

18. Eine Verbindung der Formel I gemäss Anspruch 1 mit der Bezeichnung 6,7-Dianilino-4-hydroxy-2-(2-hydroxyethyl)-2H-phthalazin-1-on.

19. Eine Verbindung der Formel I gemäss Anspruch 1 mit der Bezeichnung 6,7-Dianilino-2H-phthalazin-1-on.

20. Eine Verbindung der Formel I gemäss Anspruch 1 mit der Bezeichnung 6-Anilino-7-cyclohexylamino-2H-phthalazin-1-on.

21. Eine Verbindung der Formel I gemäss Anspruch 1 mit der Bezeichnung 6,7-Dicyclohexylamino-2H-phthalazin-1-on.

22. Pharmazeutische Präparate enthaltend Verbindungen der Formel I oder ein pharmazeutisch verwendbares Salz einer solchen Verbindung mit mindestens einer salzbildenden Gruppe gemäss einem der Ansprüche 1 bis 21 zusammen mit pharmazeutisch verwendbarem Trägermaterial.

23. Eine Verbindung der Formel I oder ein pharmazeutisch verwendbares Salz einer solchen Verbindung mit mindestens einer salzbildenden Gruppe gemäss einem der Ansprüche 1 bis 21 zur Anwendung in einem Verfahren zur therapeutischen Behandlung des tierischen oder menschlichen Körpers.

24. Verwendung einer Verbindung der Formel I oder eines pharmazeutisch verwendbaren Salzes einer solchen Verbindung mit mindestens einer salzbildenden Gruppe gemäss einem der Ansprüche 1 bis 21 zur Herstellung pharmazeutischer Präparate für die Behandlung von Krankheiten, die auf eine Hemmung von Protein-Tyrosinkinasen oder Serin/Threonin-Kinasen ansprechen.

25. Verfahren zur Herstellung von Verbindungen der Formel I gemäss Anspruch 1, dadurch gekennzeichnet, dass man
a) zur Herstellung von Verbindungen der Formel I, worin Q einen bivalenten Rest der Formel 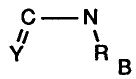 bedeutet, welcher über sein Y tragendes Kohlenstoffatom an das Q bindende Kohlenstoffatom des Benzolringes und über sein R_{B} tragendes Stickstoffatom an das R_{A} tragende Stickstoffatom in Formel I gebunden ist, worin Y unabhängig von X für Sauerstoff oder Schwefel steht, R_{A} und R_{B} unabhängig voneinander Wasserstoff, unsubstituiertes oder substituiertes Niederalkyl oder Acyl bedeuten und die übrigen Reste die genannten Bedeutungen haben, eine Dicarbonsäure der Formel II, 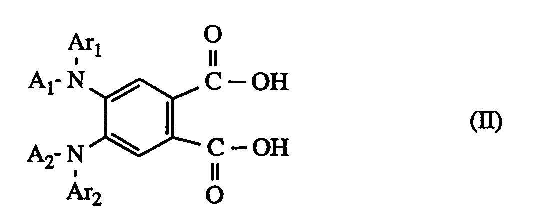 worin A₁, A₂, Ar₁ und Ar₂ die genannten Bedeutungen haben, oder ein reaktionsfähiges Derivat davon, worin die Reste die für Verbindungen der Formel I genannten Bedeutungen haben, mit einer Hydrazinverbindung der Formel III,
R_{A}-NH-NH-R_{B} (III)
worin R_{A} und R_{B} die für Verbindungen der Formel I genannten Bedeutungen haben, umsetzt, wobei die Verbindungen der Formeln II und/oder m bei Vorliegen salzbildender Gruppen auch in Form von Salzen eingesetzt werden können und in den Verbindungen der Formeln II und/oder III funktionelle Gruppen, die nicht an der Reaktion teilnehmen sollen, erforderlichenfalls in geschützter Form vorliegen, und vorhandene Schutzgruppen abspaltet, oder
b) zur Herstellung von Verbindungen der Formel I, worin Q einen bivalenten Rest der Formel 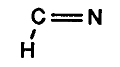 bedeutet, welcher über sein Kohlenstoffatom an das Q bindende Kohlenstoffatom des Benzolringes und über sein Stickstoffatom an das R_{A} tragende Stickstoffatom in Formel I gebunden ist, wobei R_{A} unsubstituiertes oder substituiertes Niederalkyl bedeutet, ein Formylbenzoesäurederivat der Formel XIII, 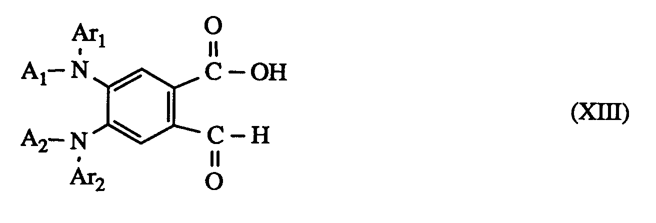 worin die Reste die zuletzt genannten Bedeutungen haben, oder ein reaktionsfähiges Derivat davon, mit einer Verbindung der Formel XIV,
R_{A}-NH-NH₂ (XIV)
worin R_{A} die zuletzt genannten Bedeutungen hat, umsetzt, wobei funktionelle Gruppen, die nicht an der Reaktion teilnehmen sollen, erforderlichenfalls geschützt sind, und vorhandene Schutzgruppen abspaltet,
und gewünschtenfalls als zusätzliche Verfahrensmassnahme eine erhältliche Verbindung der Formel I in eine andere Verbindung der Formel I umwandelt, und/oder ein erhältliches Salz in die freie Verbindung oder in ein anderes Salz umwandelt, und/oder eine erhältliche freie Verbindung der Formel I in ein Salz umwandelt und/oder ein erhältliches Gemisch von Isomeren von Verbindungen der Formel I in die Isomeren auftrennt.
